# EUROPEAN PATENT APPLICATION

(11) **EP 4 008 330 A1**
(43) Date of publication of application: **08.06.2022**
(21) Application number: 20212332.9
(22) Date of filing: 07.12.2020
(51) Int. Cl.: A61K 31/517, A61K 31/519, A61K 31/635, A61P 3/10, A61P 17/00, A61P 19/02, A61P 19/04, A61P 37/00, A61P 43/00, A61K 31/4402

(54) **COMBINED INHIBITION OF AMINO ACID TRANSPORTERS FOR INHIBITING HUMAN PLASMACYTOID DENDRITIC CELL ACTIVITY DURING AUTOIMMUNITY**

(71) Applicant: Max-Planck-Gesellschaft zur Förderung der Wissenschaften e.V., 80539 München (DE)
(72) Inventor: The designation of the inventor has not yet been filed
(74) Representative: Arth, Hans-Lothar

(57) **Abstract**

The present invention relates to the treatment of autoimmune diseases through inhibition of human plasmacytoid dendritic cell activity. The treatment is based on a combination of one inhibitor of antiporter system x_{c}⁻, and at least one inhibitor selected from the group consisting of a JAK2 inhibitor, a SLC7A5:SLC3A2 inhibitor, an inhibitor targeting IL-3 receptor alpha, IL-3 receptor beta, an inhibitor targeting GM-CSF, an inhibitor targeting GM-CSF receptor alpha, and an inhibitor targeting STAT5. The present invention also provided pharmaceutical compositions comprising said combination.

## Description

### Specification

The present invention relates to the treatment of autoimmune diseases through inhibition of human plasmacytoid dendritic cell activity. The treatment is based on a combination of one inhibitor of antiporter system x_{c}⁻, and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5. The present invention also provided pharmaceutical compositions comprising said combination.

### Background of the invention

Plasmacytoid dendritic cells (pDC) reside in blood and lymphoid organs, but during inflammation enter tissues where they serve as a major source of type I interferons (IFN). They play roles in both protective immune responses and disease related immune responses to viral infections, and are implicated in inflammatory and autoimmune diseases like systemic lupus erythematosus, SLE.

SLE is a life-threatening chronic autoimmune disease that can affect kidneys, skin, brain and other tissues.

Although SLE varies patient to patient, one of the clinical hallmarks is the presence of autoantibodies and immune complexes, which contain host-derived nucleic acids, in the circulation. These immune complexes stimulate type I interferon-alpha (IFNα) and interleukin 6 (IL-6) production from plasmacytoid dendritic cells (pDC) via toll-like receptor 7 (TLR7) and TLR9. IFNα and IL-6 both, in turn, support auto-reactive B cells and plasmablast expansion, subsequently driving the differentiation and accumulation of auto-antibody producing plasma cells. In the last decade, B cell depleting antibodies such as anti-CD20, e.g. Rituximab, Ocrelizumab, Ofatumumab, and drugs that inhibit B cell activating factors, e.g. Belimumab, BLyS inhibition; Atacicept, BLyS/APRIL inhibition, have been introduced as new therapies for SLE patients. B cell depletion or inhibition of B cell maturation has proven useful in cases of severe disease and occasionally in patients who do not respond to global immune suppression with steroids or non-steroidal anti-inflammatory drugs: the current "standard of care". Although B cell depleting therapies offer a more directed treatment with fewer side effects than the traditional standard of care options, patients still experience serious adverse events. The main concern is an elevated risk of severe infection, such as upper respiratory tract and urinary tract infections, as well as influenza, in addition to patients experiencing headaches and joint pain. Moreover, current B-cell targeting strategies fail to target plasma cells and do not reduce circulating levels of autoantibodies and immune complexes. Thus, there remains a desperate need for new SLE-specific treatment options.

An alternative treatment option on the horizon relates to direct targeting of pDCs. The primary mechanism of activation of pDC is through ligation of endosomal TLR7 or TLR9 by their nucleic acid agonists. Physiologically, access of nucleic acid ligands to these TLRs is promoted by the uptake of nucleic acid-containing immune complexes. Downstream of TLRs, type I IFN are induced through an MyD88-Interferon regulatory factor 7 (IRF7)-dependent pathway, but further signaling through MYD88-dependent NF_{K}-B activation leads to the additional production of TNF-α.

pDC express high levels of interleukin 3 receptor a (IL-3Ra) compared to other leukocytes. IL-3, along with granulocyte-macrophage colony-stimulating factor (GM-CSF) and IL-5, is a member of the common β chain cytokine family. Binding of these cytokines to their respective cytokine-specific α subunits results in dimerization with common β chain which initiates signaling through Janus kinase 2 (JAK2), which in turn phosphorylates Signal transducer and activator of transcription 5 (STAT5), causing translocation of STAT5 to the nucleus and initiation of transcription. A correlation between elevated levels of IL-3 and SLE incidents has been noted, and there is a link between an IL-3-stimulated gene signature and the type I Interferon-stimulated gene signature in SLE patients. Monoclonal antibodies (mAb) directed against IL-3Ra were recently shown to diminish responses by human pDC to TLR7/9 stimulation and to selectively deplete these cells from peripheral blood mononuclear cells (PBMC). Likewise, in a mouse model of SLE, MRL/lpr, administration of anti-IL-3 mAb ameliorated disease symptoms associated with kidney damage. However, the nature of the effect of IL-3 on pDC has remained unclear.

**It is the objective of the present invention to provide combinations and/or pharmaceutical compositions targeting activation of plasmacytoid dendritic cells and that can be used especially for the treatment of autoimmune diseases.**

**The objective of the present invention is solved by the teaching of the independent claims. Further advantageous features, aspects and details of the invention are evident from the dependent claims, the description, the figures, and the examples of the present application.**

### Brief description of the invention

The present invention relates to a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and
   an inhibitor targeting STAT5,
wherein the autoimmune disease is mediated by plasmacytoid dendritic cells.

More in particular, the present invention relates to a combination for use in the treatment of an autoimmune disease, wherein the combination consists of:
at least one inhibitor of antiporter system x_{c}⁻ and
at least one inhibitor selected from the group consisting of:
   a JAK2 inhibitor, and
   a SLC7A5:SLC3A2 inhibitor,
wherein the autoimmune disease is mediated by plasmacytoid dendritic cells.

A preferred embodiment of the invention is directed to a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and
   an inhibitor targeting STAT5,
wherein the autoimmune disease is mediated by plasmacytoid dendritic cells; and wherein at least one inhibitor of the antiporter system x_{c}⁻ is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-I-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthylethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib.

A more preferred embodiment of the invention is directed to a combination for use in the treatment of an autoimmune disease, wherein the combination consists of:
at least one inhibitor of antiporter system x_{c}⁻ and
at least one inhibitor selected from the group consisting of:
   a JAK2 inhibitor, and
   a SLC7A5:SLC3A2 inhibitor,
wherein the autoimmune disease is mediated by plasmacytoid dendritic cells; and wherein at least one inhibitor of the antiporter system x_{c}⁻ is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-I-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthylethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib.

A preferred embodiment of the invention is directed to a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and
   an inhibitor targeting STAT5,
wherein the autoimmune disease is mediated by plasmacytoid dendritic cells; and wherein at least one inhibitor of the antiporter system x_{c}⁻ is sulfasalazine.

A more preferred embodiment of the invention is directed to a combination for use in the treatment of an autoimmune disease, wherein the combination consists of:
at least one inhibitor of antiporter system x_{c}⁻ and
at least one inhibitor selected from the group consisting of:
   a JAK2 inhibitor, and
   a SLC7A5:SLC3A2 inhibitor,
wherein the autoimmune disease is mediated by plasmacytoid dendritic cells; and wherein at least one inhibitor of the antiporter system x_{c}⁻ is sulfasalazine.

A more preferred embodiment of the invention is directed to a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and
   an inhibitor targeting STAT5,
   wherein the autoimmune disease is mediated by plasmacytoid dendritic cells; and wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
   wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine; KHK2898 antibody, IGN523 antibody; or
   wherein the inhibitor targeting IL-3 receptor alpha is selected from the group comprising CSL362 antibody, flotetuzumab, IL3LDM, DT388IL3, and SGN-CD123A; or
   wherein the inhibitor targeting IL-3 receptor beta is CSL311 antibody, or wherein the inhibitor targeting GM-CSF is otilimab, namilumab, lenzilumab, plonmarlimab, or gimsilumab; or
   wherein the inhibitor targeting GM-CSF receptor alpha is mavrilimumab; or wherein the inhibitor targeting STAT5 is JPX1188, JPX0802, JPX1185, compound 17f, compound AC-4-130, CAS 285986-31-4, BP-1-075, BP-1-107, BP-1-108, SF-1-087, SF-1-088, IQDMA, or CAS 2062-78-4.

A more preferred embodiment of the invention is directed to a combination for use in the treatment of an autoimmune disease, wherein the combination consists of:
at least one inhibitor of antiporter system x_{c}⁻ and
at least one inhibitor selected from the group consisting of:
   a JAK2 inhibitor, and
   a SLC7A5:SLC3A2 inhibitor,
wherein the autoimmune disease is mediated by plasmacytoid dendritic cells; and wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine; KHK2898 antibody, IGN523 antibody.

A further preferred embodiment of the invention is directed to a combination for use in the treatment of an autoimmune disease, wherein the combination consists of:
at least one inhibitor of antiporter system x_{c}⁻ and
at least one a JAK2 inhibitor,
wherein the autoimmune disease is mediated by plasmacytoid dendritic cells; and wherein the at least one JAK2 inhibitor is baricitinib, or delgocitinib.

Another embodiment of the invention provides a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and
   an inhibitor targeting STAT5,
wherein the autoimmune disease is mediated by plasmacytoid dendritic cells; and wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-lgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

A further embodiment of the invention provides a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and
   an inhibitor targeting STAT5,
wherein the autoimmune disease is mediated by plasmacytoid dendritic cells; and wherein the autoimmune disease is systemic lupus erythematosus.

A preferred embodiment of the invention provides a pharmaceutical composition containing at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor beta, an inhibitor targeting IL-3 receptor alpha, an inhibitor targeting GM-CSF, an inhibitor targeting GM-CSF receptor alpha, and an inhibitor targeting STAT5, together with at least one pharmaceutically acceptable vehicle, excipient and/or diluent.

A further preferred embodiment of the invention provides a pharmaceutical composition containing at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor beta, an inhibitor targeting IL-3 receptor alpha, an inhibitor targeting GM-CSF, an inhibitor targeting GM-CSF receptor alpha, and an inhibitor targeting STAT5, together with at least one pharmaceutically acceptable vehicle, excipient and/or diluent, and
wherein the at least one inhibitor of the antiporter system x_{c}⁻ is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methyiamino-L-alanine, L-serine-O-sulphate, L-α-aminopimelate, L-homocysteate, S-sulpho-I-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthyl-ethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib.

In another aspect, the present invention provides a pharmaceutical composition containing at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent, and wherein the at least one inhibitor of the antiporter system x_{c}⁻ is sulfasalazine.

In a particular aspect, the present invention provides a pharmaceutical composition containing at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent, and
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine; KHK2898 antibody, IGN523 antibody; or
wherein the inhibitor targeting IL-3 receptor alpha is selected from the group comprising CSL362 antibody, flotetuzumab, IL3LDM, DT388IL3, and SGN-CD123A,; or
wherein the inhibitor targeting IL-3 receptor beta is CSL311 antibody, or wherein the inhibitor targeting GM-CSF is otilimab, namilumab, lenzilumab, plonmarlimab, or gimsilumab; or
wherein the inhibitor targeting GM-CSF receptor alpha is mavrilimumab; or wherein the inhibitor targeting STAT5 is JPX1188, JPX0802, JPX1185, compound 17f, compound AC-4-130, CAS 285986-31-4, BP-1-075, BP-1-107, BP-1-108, SF-1-087, SF-1-088, IQDMA, or CAS 2062-78-4.

In a preferred aspect, the present invention provides a pharmaceutical composition containing at least one inhibitor of antiporter system x_{c}⁻ and at least one JAK2 inhibitor, together with at least one pharmaceutically acceptable vehicle, excipient and/or diluent, wherein the at least one JAK2 inhibitor is barcitinib.

In a preferred aspect, the present invention provides a pharmaceutical composition containing at least one inhibitor of antiporter system x_{c}⁻ and at least one JAK2 inhibitor, together with at least one pharmaceutically acceptable vehicle, excipient and/or diluent, wherein the at least one JAK2 inhibitor is delgocitinib.

In a further preferred aspect, the present invention provides a pharmaceutical composition containing at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent, **for use** in the treatment of an autoimmune disease, wherein the autoimmune disease is mediated by plasmacytoid dendritic cells.

In a more preferred aspect, the present invention provides a pharmaceutical composition containing at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent, for use in the treatment of an autoimmune disease, wherein the autoimmune disease is mediated by plasmacytoid dendritic cells, and wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-lgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

In a still more preferred aspect, the present invention provides a pharmaceutical composition containing at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent, for use in the treatment of an autoimmune disease, wherein the autoimmune disease is mediated by plasmacytoid dendritic cells, and wherein the autoimmune disease is systemic lupus erythematosus.

### Description of the invention

Plasmacytoid dendritic cells (pDC) is a unique cell population that produces large amounts of type I interferon upon recognition of nucleic acids placing them at the crossroad of both innate and adaptive immunity. Their ability to produce interferon makes them central to anti-viral responses. They are also responsive to circulating autoantibodies bound to nuclear antigens and in that scenario the release of interferons initiate self-directed immune responses. There are now a growing number of autoimmune disorders where unabated activation of pDC is suspected to be pathogenic (Panda et al., Curr Opin Immunol 2017, 44: 20 - 25).

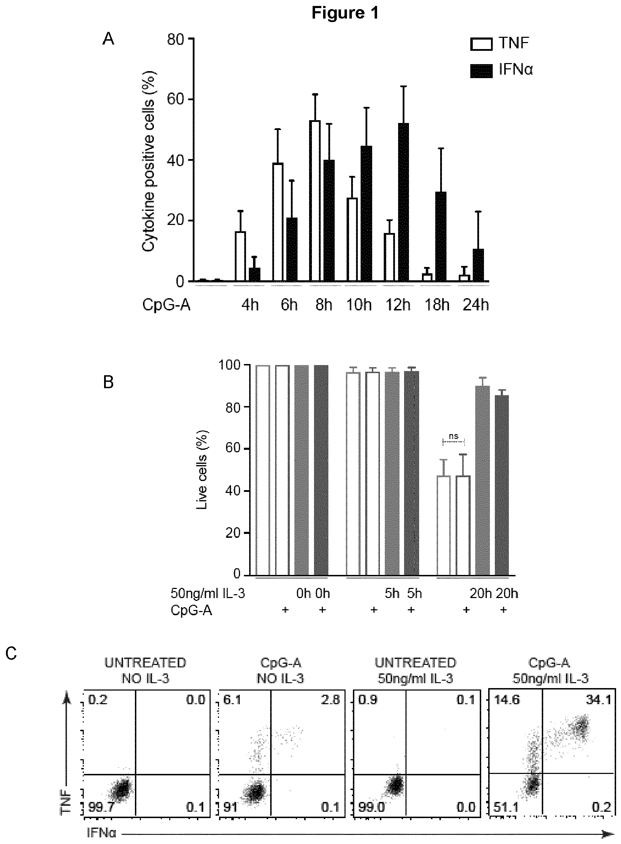

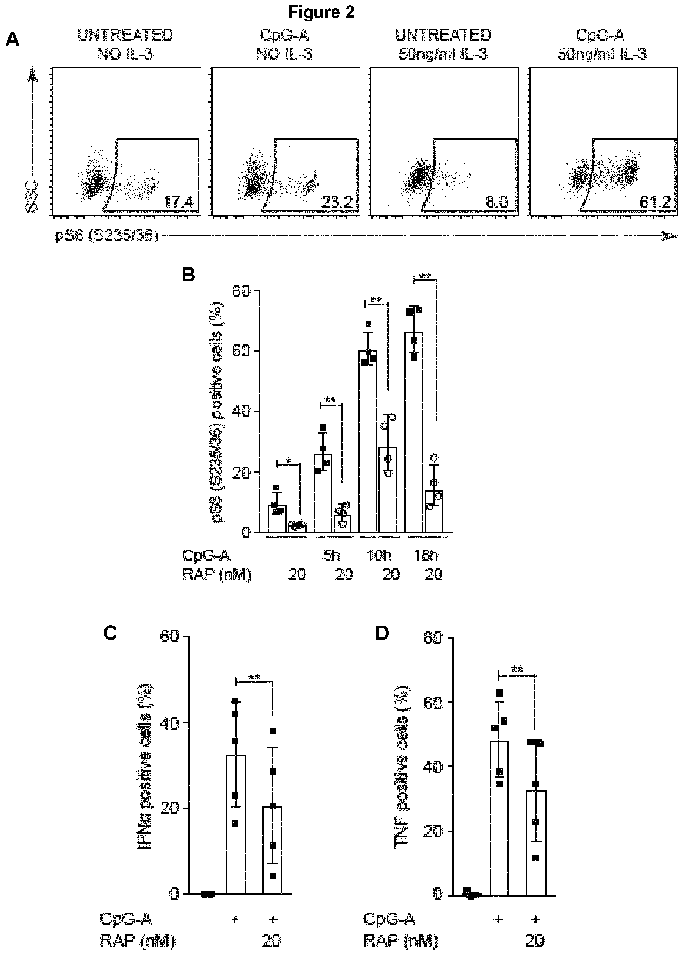

The inventors have here found that IL-3 stimulated cytokine production by pDC in response to TLR9 stimulation **(****Figures 1A - 1C****).**
Moreover, IL-3 played a critical role in priming pDC to allow mTORC1 activation upon CpG-A stimulation **(****Figures 2A, 2B****),** which mediated production of IFNα and TNFα **(****Figures 2C, 2D****).**
Results also showed that mTORC1 facilitates increased anabolic activity **(****Figure 3B** - **3G****)** linked to type I IFN and TNFα production **(****Figure 4C****),** and the expression of the *SLC7A11* subunit **(****Figure 12A****,** **12C****)** of system x_{c}⁻, the cystine/glutamate antiporter, which comprises *SLC7A11* together with *SLC3A2.* The system x_{c}⁻ exchanges intracellular glutamate for environmental cystine, an amino acid that is essential for glutathione synthesis.

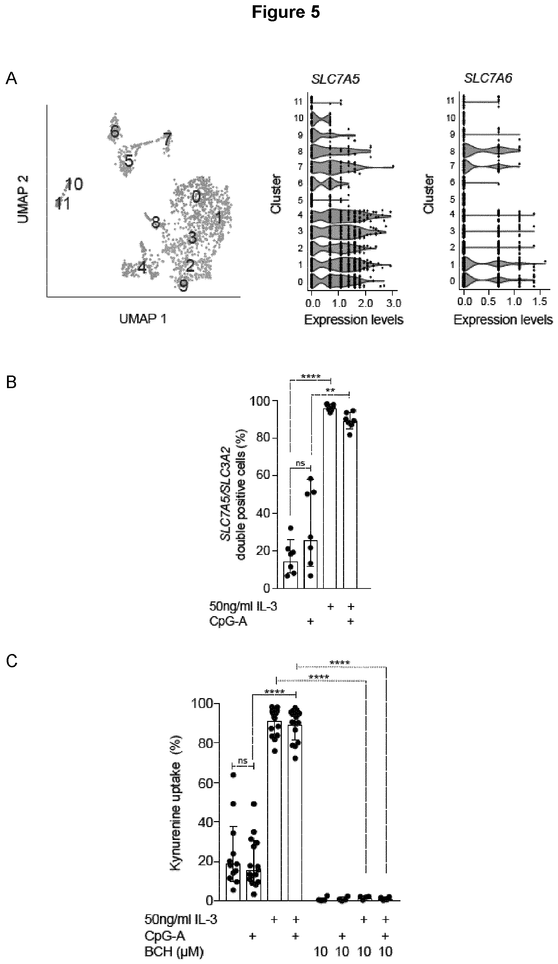

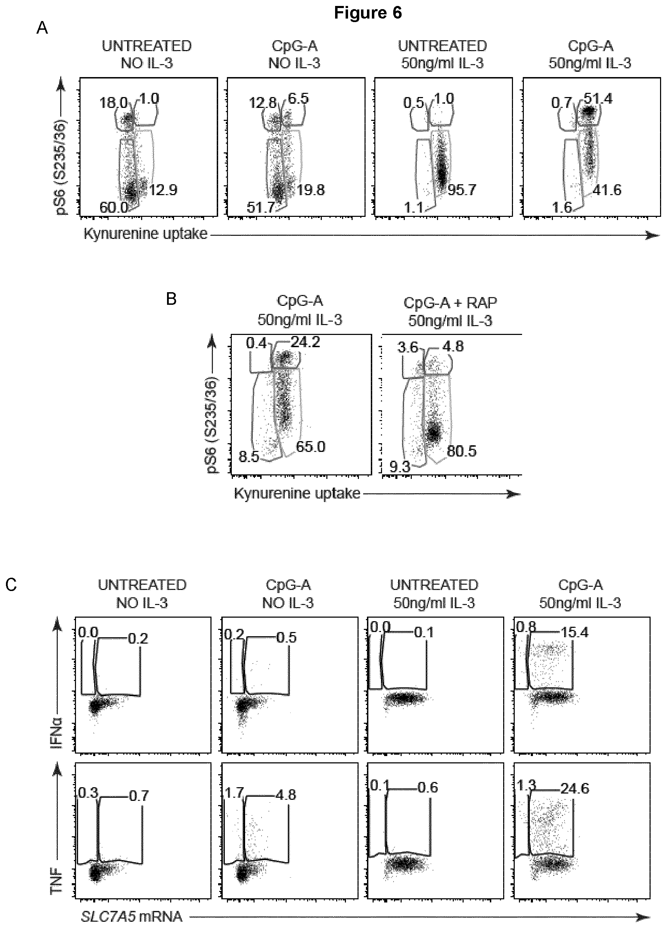

Thereafter, it was found that IL-3 induces JAK2-dependent expression of the *SLC7A5* and *SLC3A2* **(****Figure 5B, 5C****)** components of the System L amino acid (System LAA) transporter, activity of which is required for activation of mTORC1 (mammalian target of rapamycin complex 1) in response to TLR agonists **(****Figure 6A****).**

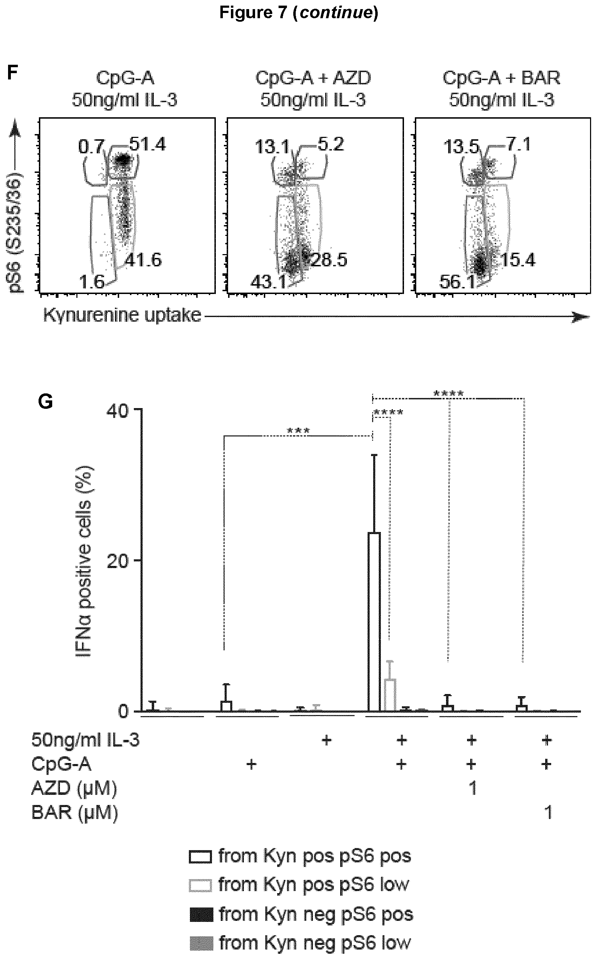

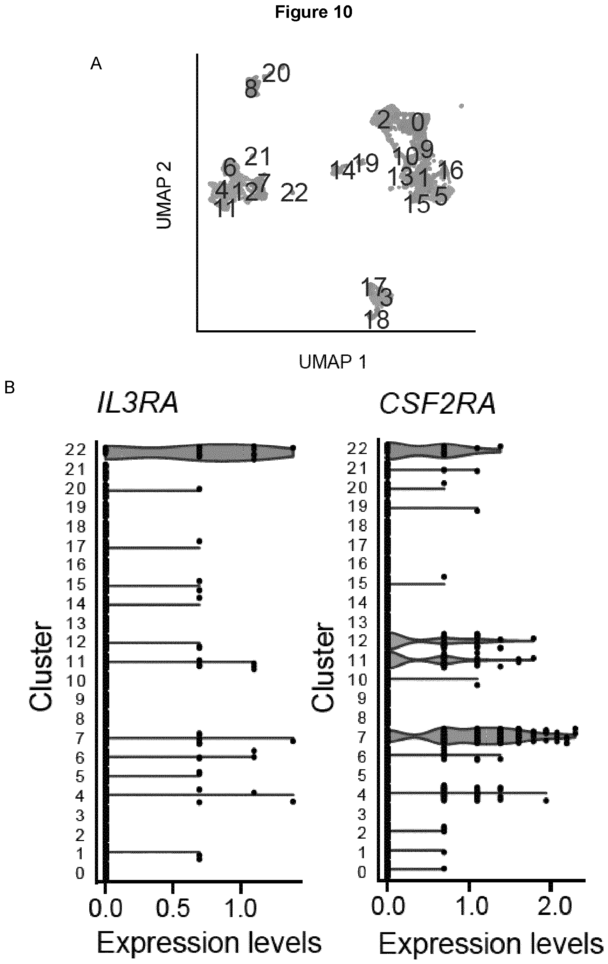

The expression of *SLC7A5* and *SLC3A2* of system LAA transporter were necessary for the establishment of maximal mTORC1 activity **(****Figures 6A** - **6B****)** and cytokine production **(****Figures 6C** - **6D****)** in response to the TLR9 agonist, CpG-A.
This pathway was only active in the type I IFN- and TNFa-producing subset of TLR9-stimulated pDC **(****Figure 6A** **and** **7G****)** and this population of cells was transcriptionally similar to pDC at the site of nephritic disease in SLE patients **(****Figures 10A - 10C** **-10E).**

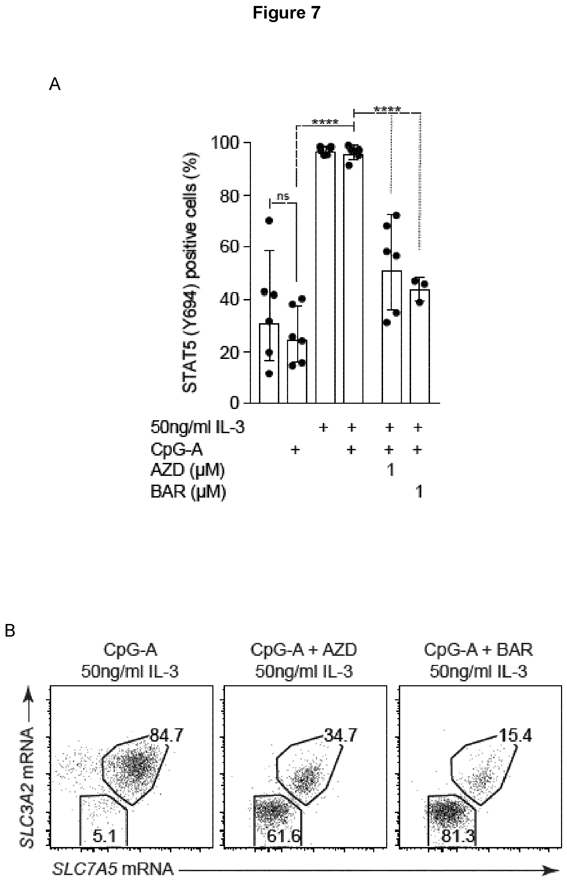

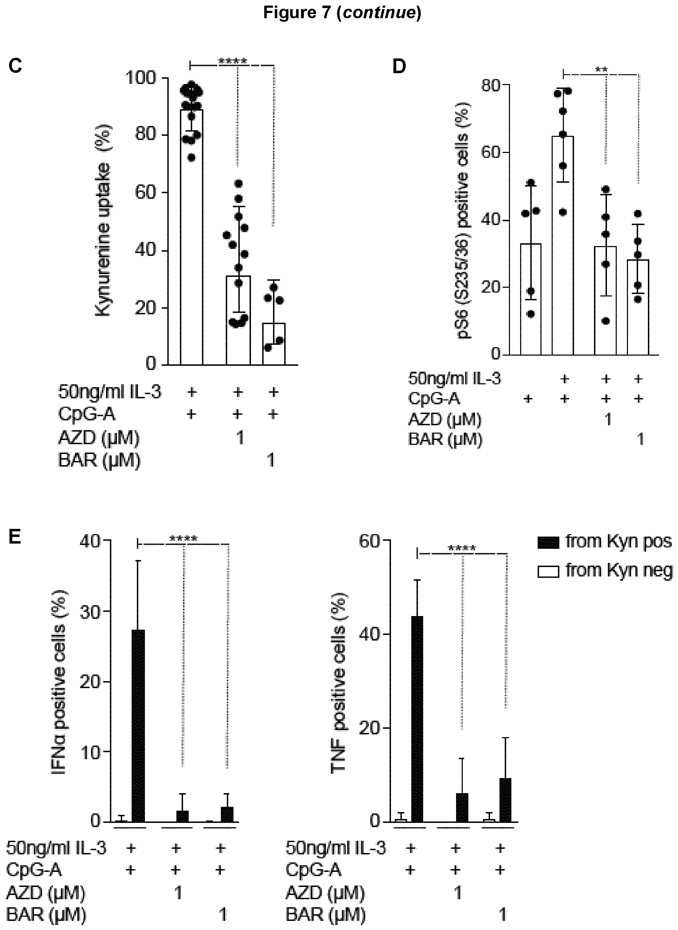

Importantly, the inventors found that IL-3, but not CpG-A, was able to induce phosphorylation of STAT5 (Y694) **(****Figure 7A****).**
As expected, AZD and BAR inhibited both IL-3-induced STAT5 (Y694) phosphorylation **(****Figure 7A****),** and IL-3 induced expression of *SLC7A5:SLC3A2* and activity **(****Figure 7B****,** **7C****)** in TLR9-activated pDC. JAK2 and JAK2/1 inhibitors (AZD, BAR respectively) caused a decrease in IFNα and TNFα production **(****Figure 7E****)** linked to reductions in *SLC7A5:SLC3A2* expression **(****Figure 7B****),** and mTORC1 activity **(****Figure 7D****)** in TLR9-activated pDC. Finally, only pDC with both high rates of kynurenine uptake and high mTORC1 activity (S6 phosphorylation, **Figure 7F****)** made IFNα **(****Figure 7G****),** and that cytokine production was lost following JAK2 inhibition **(****Figure 7G****),** arguing that the subset of activated cells that may play a pathological role during disease can be inhibited in this fashion.

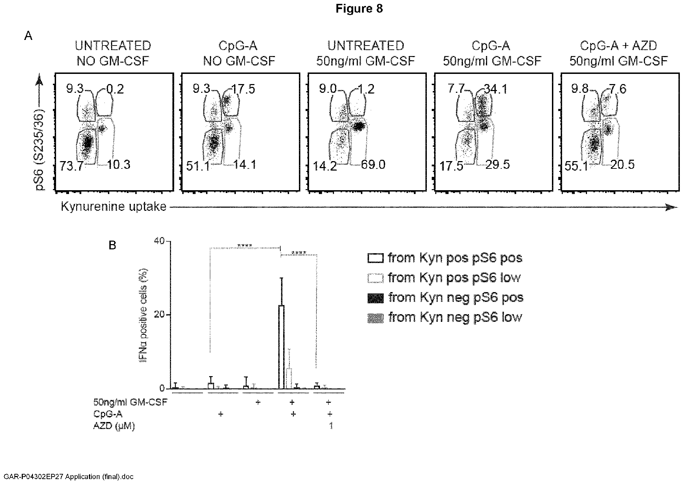

Like IL-3, GM-CSF supported pDC survival, induced a population of cells with high rates of kynurenine uptake and mTORC1 activity **(****Figure 8A****)** and these cells were IFNα producers in response to CpG-A **(****Figure 8B****).** Moreover, all of these parameters were inhibited by JAK2 inhibitor AZD **(****Figures 8A, 8B****).**

Taken together, these data reveal the emergence of a subpopulation of pDC capable of making IFNα and TNFα in response to CpG-A. These cells express functional System LAA transporters (SLC7A5:SLC3A2) and are able to activate mTORC1 upon exposure to CpG-A (TLR9 stimulation). These processes are dependent on priming by **IL-3 or GM-CSF** through the **JAK2-STAT5** pathway downstream of the common β subunit of the IL-3 and GM-CSF receptors.

These data highlight that inhibition of IL-3 signalling, GM-CSF signalling, JAK2, STAT5, SLC7A5:SLC3A2 activities is a therapeutic option in conditions where pDC activation is implicated in disease.

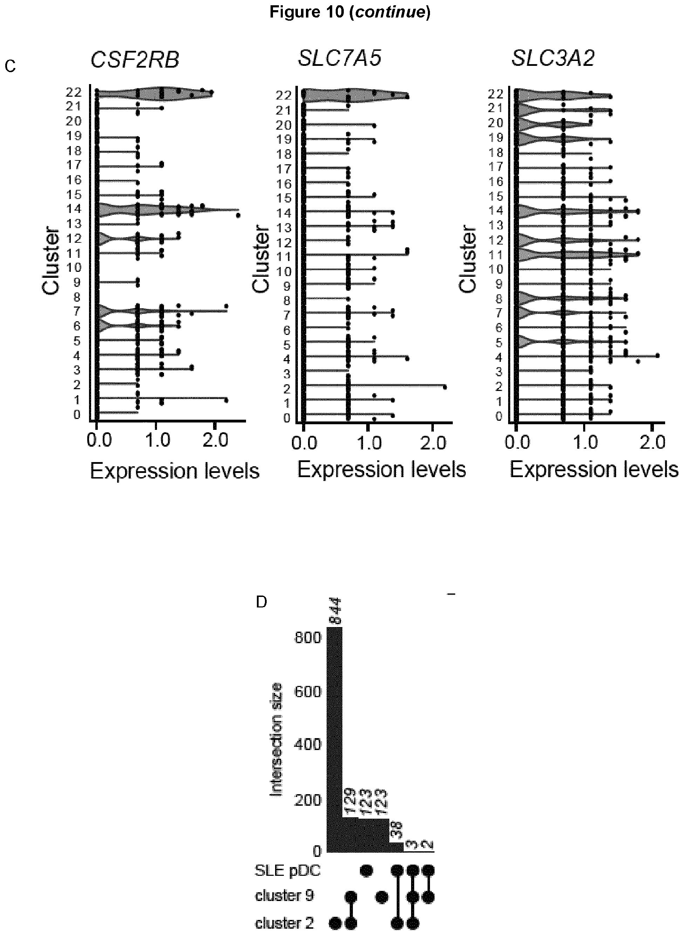

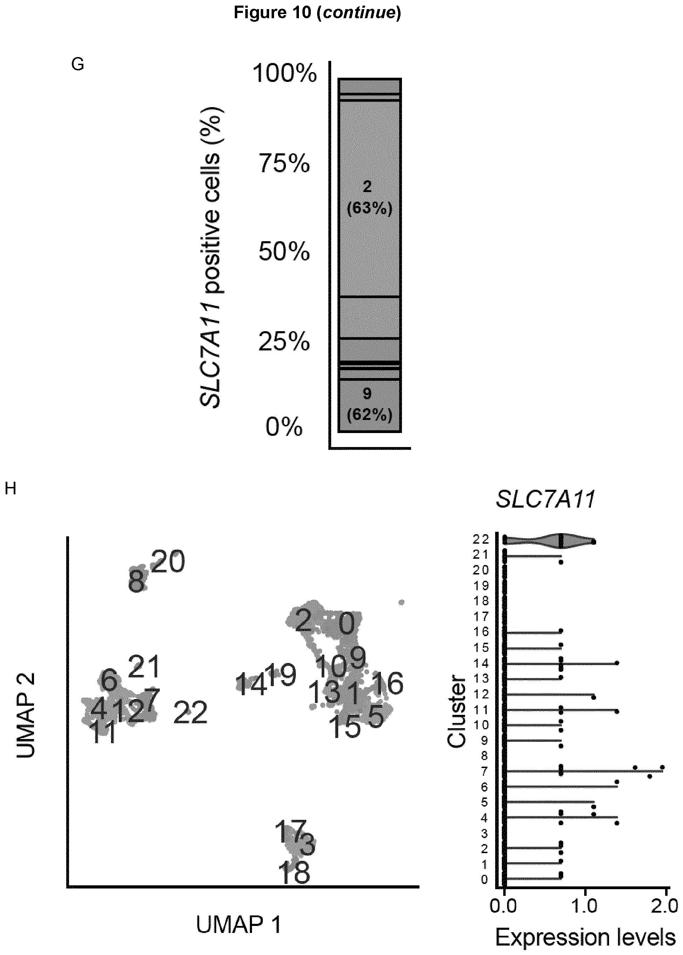

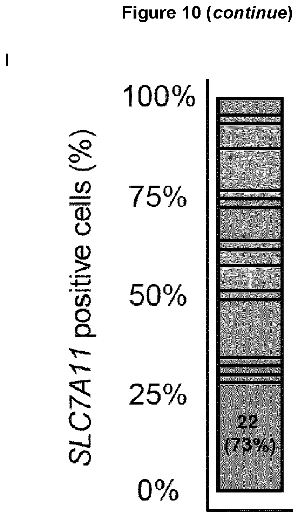

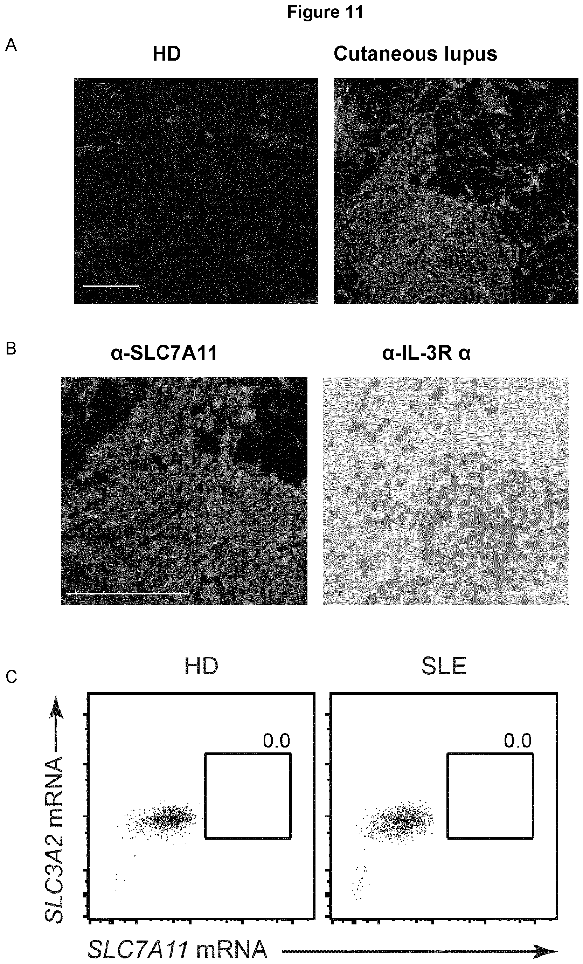

*SLC7A11* expression, a subunit of the glutamine cystine transporter x_{c}⁻, was shared between in vitro-activated pDC **(****Figures 10D** - **10G****)** and pDC at sites of SLE-associated pathology **(****Figures 10H****,** **10I** **11A, 11B****),** but not by pDC in the bloodstream **(****Figure 11C****).**

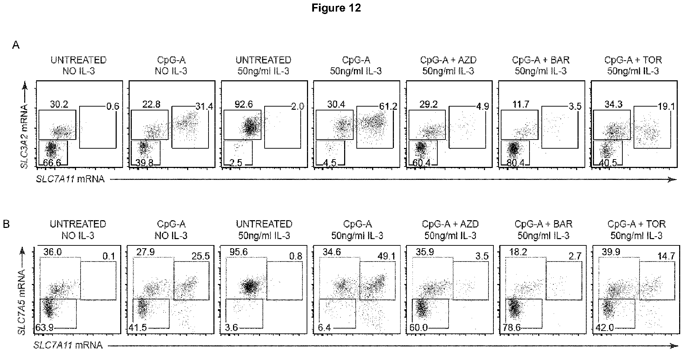

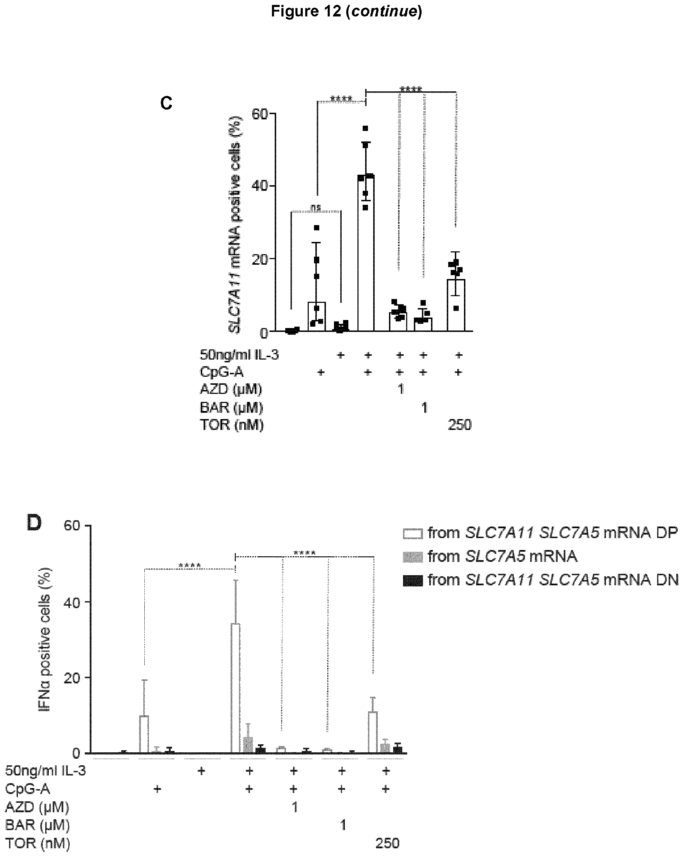

IL-3 depletion and JAK2 inhibitors prevented expression of *SLC7A11* in response to TLR9 ligand CpG-A **(****Figure 12A****,** **12** **B, 12C),** suggesting that this is due to their ability to effectively inhibit the upstream steps that are critical for mTORC1 activation controlling SLC7A11 expression **(****Figures 12A, 12B****,** **12C****).**

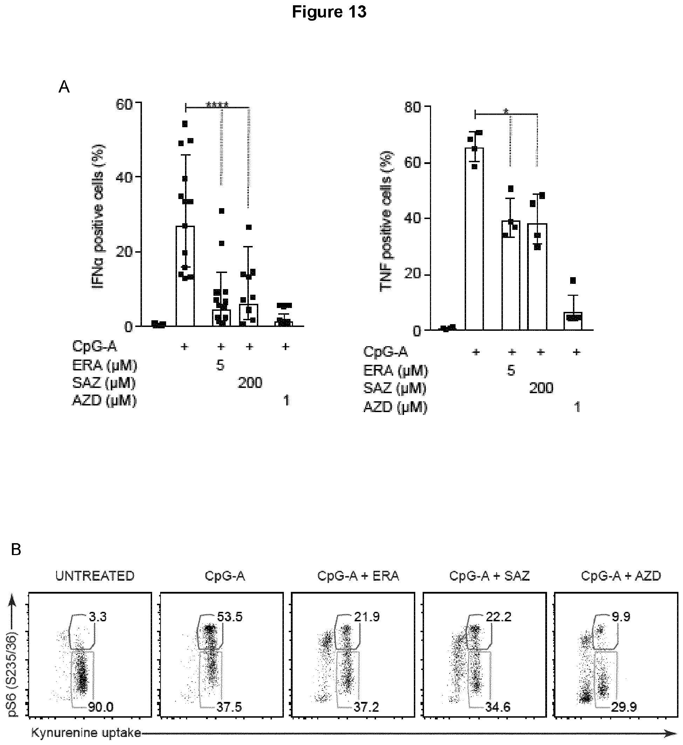

Interestingly, small molecule inhibitors of x_{c}⁻ erastin and sulfasalazine were able to diminish cytokine production in pDC **(****Figure 13A** - **13C****).**

The data above demonstrate that a two-step process is required for pDC activation, in which IL-3/GM-CSF/JAK2/STAT5 lead to the sequential expression of amino acid transporter SLC5A5/SLC3A2 and system x_{c}⁻ that are critical for pDC to make inflammatory cytokines.

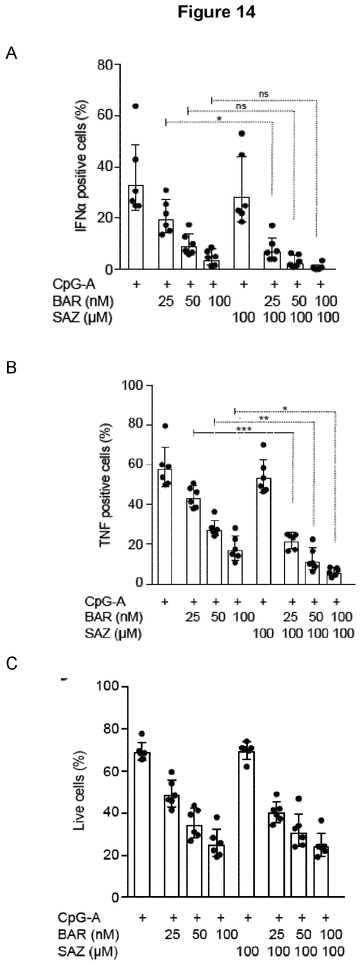

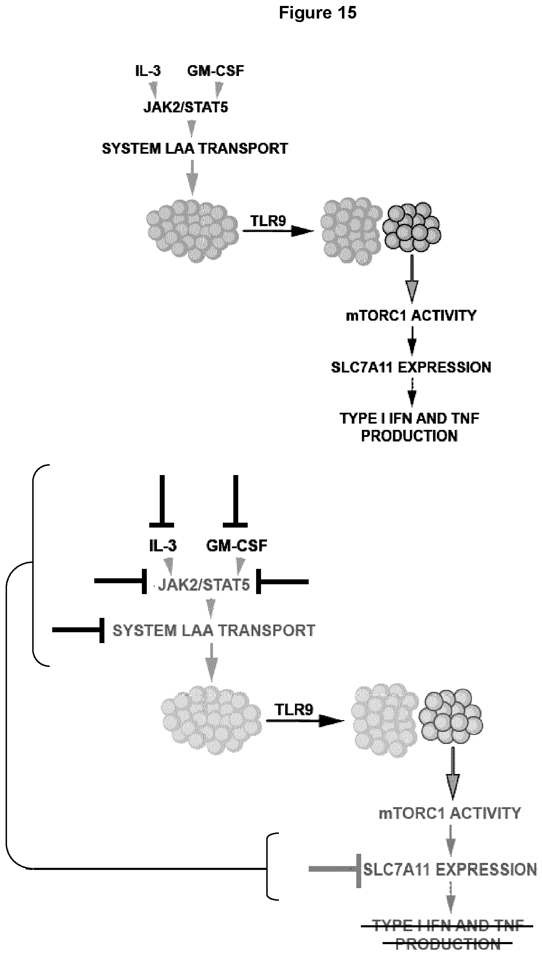

Of importance, the combination of SLC7A11 inhibitors and JAK2 inhibitors, which target SLC7A5:SLC3A2 transporter, effectively and synergistically switched off cytokine production by activated pDC (Figures 14A - 14B). This represents an important advantage, as the inhibitors in the combination are effective at lower doses, which would be suboptimal or not effective when taken alone.
The data presented here highlight that simultaneous targeting of the two-step process, IL-3/GM-CSF/JAK2/STAT5/SLC7A5:SLC3A2 on one side, and system x_{c}⁻ on the other side, could offer a new and pDC-selective therapy for autoimmunity (Figure 15).

A valuable advantage of the present invention is that it can simultaneously target a set of cytokines, i.e. IFNalpha and TNFalpha, whereas most prior art therapies are only directed to one of them, mostly IFNalpha, for example by using IFNalpha blocking antibodies.

Moreover, the combination therapy described herein targets two independent pathways, IL-3/GM-CSF/JAK2/STAT5/SLC7A5:SLC3A2, and system x_{c}⁻ on the other side, each critical for full pDC activation, in a single multi-hit approach. Targeting each pathway independently is shown to have significant inhibitory effects on cytokine production by activated pDCs, and targeting both pathways together results in synergistic inhibitory effects on cytokine production. This offers the possibility of a scaled step-up or step-down approach for therapy. In aggressive onset disease, both drugs could be administered initially to minimize organ damage, and then the treatment could be tapered by the removal of one of the drugs. The use of intensive therapy at the outset of disease could increase the rate of remission. Alternatively, in mild onset disease, treatment could be started with one drug before moving to combination therapy if required. The combination of two drugs targeting distinct pathways for synergistic inhibition of pDC activation may circumvent possible effects of the expression of multiple-drug resistance genes, a process that has been linked to the development of resistance to current therapies in diseases such as SLE.

Thus, the present invention is directed to a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and
   an inhibitor targeting STATS,
wherein the autoimmune disease is mediated by plasmacytoid dendritic cells.

More particularly, the present invention is directed to a combination for use in the treatment of an autoimmune disease, wherein the combination consists of:
at least one inhibitor of antiporter system x_{c}⁻ and
at least one inhibitor selected from the group consisting of:
   a JAK2 inhibitor, and
   a SLC7A5:SLC3A2 inhibitor,
wherein the autoimmune disease is mediated by plasmacytoid dendritic cells.

In one aspect, the present invention is directed to a combination for use in the treatment of an autoimmune disease, wherein the combination consists of:
at least one inhibitor of antiporter system x_{c}⁻ and
at least one a JAK2 inhibitor,
wherein the autoimmune disease is mediated by plasmacytoid dendritic cells.

In another aspect, the present invention is directed to a combination for use in the treatment of an autoimmune disease, wherein the combination consists of:
at least one inhibitor of antiporter system x_{c}⁻ and
at least one SLC7A5:SLC3A2 inhibitor,
wherein the autoimmune disease is mediated by plasmacytoid dendritic cells.

In still another aspect, the present invention is directed to a combination for use in the treatment of an autoimmune disease, wherein the combination consists of:
at least one inhibitor of antiporter system x_{c}⁻ and
at least one inhibitor targeting IL-3 receptor,
wherein the autoimmune disease is mediated by plasmacytoid dendritic cells.

In a particular aspect, the present invention is directed to a combination for use in the treatment of an autoimmune disease, wherein the combination consists of:
at least one inhibitor of antiporter system x_{c}⁻ and
at least one inhibitor targeting GM-CSF,
wherein the autoimmune disease is mediated by plasmacytoid dendritic cells.

In a further particular aspect, the present invention is directed to a combination for use in the treatment of an autoimmune disease, wherein the combination consists of:
at least one inhibitor of antiporter system x_{c}⁻ and
at least one inhibitor targeting GM-CSF receptor alpha,
wherein the autoimmune disease is mediated by plasmacytoid dendritic cells.

In a further more particular aspect, the present invention is directed to a combination for use in the treatment of an autoimmune disease, wherein the combination consists of:
at least one inhibitor of antiporter system x_{c}⁻ and
at least one inhibitor targeting STAT-5,
wherein the autoimmune disease is mediated by plasmacytoid dendritic cells.

### Definitions

**"Plasmacytoid dendritic cells"** pDCs have a unique role in linking innate and adaptive immunity. They have a lymphoid shape and a plasma cell morphology with an extensive endoplasmic reticulum, multiple mitochondria, and a small Golgi apparatus. pDCs have poor antigen presentation ability, in particular for exogenous antigens, but can acquire antigen-presenting cell function following activation with the expression of co-stimulatory molecules that can instruct T cells toward specific functional subsets. Once activated by TLRs, the expression of co-stimulatory molecules is induced in all pDCs, which confers T cell priming properties to these cells. Although pDCs can be activated through different cell surface receptors and cytosolic nucleic acid sensors, the sensing of nucleic acids through TLR7 and TLR9 seems to be the dominant mode of activation of these cells with respect to IFN production. Signaling through these two TLRs leads to the rapid and massive production of all type I and type III IFNs, which triggers the induction of IFN-stimulated genes (ISGs), many of them with antiviral properties. Because of these properties, it is postulated that the key function of pDCs is to act as antiviral cells. The activation of pDCs and the production of IFN-I can be important to the antiviral response and can promote tissue repair. However, the chronic or long-term persistent activation of these cells, which can be seen in autoimmunity and persistent viral infections, can lead or contribute to impaired immunity and disease progression, as discussed below.

As used herein, the term **"in combination"** refers to the use of more than one therapeutic agent (e.g., one inhibitor selected from a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, an inhibitor targeting STAT5, and a system x_{c}⁻ inhibitor). The use of the term **"in combination"** does not restrict the order in which said therapeutic agents are administered to a subject with a disease or disorder, e.g., an autoimmune disease.

As used herein, **"antagonist"** or **"inhibitor"** refers to a compound or combination of compounds that can reduce, minimize, suppress, block, or eliminate expression or function of a target molecule.

**"Inhibitors"** or **"inhibitor compounds"** contemplated by the present disclosure can take any of a variety of forms including natural compounds, chemical small molecule compounds or biological compounds. Exemplary compounds include a nucleic acid (e.g., an aptamer), a polypeptide, a peptide, a small molecule, an antibody, a monoclonal antibody, a polyclonal antibody, a chimeric antibody, an humanized antibody, an antigen binding fragment of an antibody, an antibody-drug conjugate, a fusion protein, multispecific antibodies, bispecific antibodies.

As used herein, the term ***"in vitro"*** or ***"ex vivo"*** refers to an artificial environment and to processes or reactions that occur within an artificial environment, for example, but not limited to, test tubes and cell cultures. The term "in vivo" refers to a natural environment (e.g., an animal or a cell) and to processes or reactions that occur within a natural environment.

**"Immunoglobulin"** or **"antibody"** is used broadly to refer to both antibody molecules and a variety of antibody-derived molecules and includes any member of a group of glycoproteins occurring in higher mammals that are major components of the immune system. The term **"antibody"** is used in the broadest sense and specifically covers monoclonal antibodies, antibody compositions with polyepitopic specificity, bispecific antibodies, diabodies, and single-chain molecules, as well as antibody fragments (e.g., Fab, F(ab')2, and Fv), so long as they exhibit the desired biological activity. An immunoglobulin molecule includes antigen binding domains, which each include the light chains and the end-terminal portion of the heavy chain, and the Fc region, which is necessary for a variety of functions, such as complement fixation. There are five classes of immunoglobulins wherein the primary structure of the heavy chain, in the Fc region, determines the immunoglobulin class. Specifically, the alpha, delta, epsilon, gamma, and mu chains correspond to IgA, IgD, IgE, IgG and IgM, respectively. As used herein "immunoglobulin" or "antibody" includes all subclasses of alpha, delta, epsilon, gamma, and mu and also refers to any natural (e.g., IgA and IgM) or synthetic multimers of the four-chain immunoglobulin structure. Antibodies non-covalently, specifically, and reversibly bind an antigen.

The term **"monoclonal antibody"** as used herein refers to an antibody obtained from a population of substantially homogeneous antibodies, i.e., the individual antibodies comprising the population are identical except for possible naturally occurring mutations that can be present in minor amounts. For example, monoclonal antibodies may be produced by a single clone of antibody-producing cells. Unlike polyclonal antibodies, monoclonal antibodies are monospecific (e.g., specific for a single epitope of a single antigen). The modifier "monoclonal" indicates the character of the antibody as being obtained from a substantially homogeneous population of antibodies, and is not to be construed as requiring production of the antibody by any particular method.

As used herein **"chimerized"** refers to an immunoglobulin, wherein the heavy and light chain variable regions are not of human origin and wherein the constant regions of the heavy and light chains are of human origin.

**"Humanized"** refers to an immunoglobulin such as an antibody, wherein the amino acids directly involved in antigen binding, the complementarity determining regions (CDR), of the heavy and light chains are not of human origin, while the rest of the immunoglobulin molecule, the framework regions of the variable heavy and light chains and the constant regions of the heavy and light chains, are of human origin.

**"Fully human"** refers to an immunoglobulin, such as an antibody, where the whole molecule is of human origin or consists of an amino acid sequence identical to a human form of the antibody.

### Inhibitors of system x_{c}⁻"

Thus, an **"inhibitor of system** x_{c}⁻" refers to a compound or combination of compounds that can reduce, minimize, suppress, block, or eliminate expression or function of **system** x_{c}⁻.

SLC7A11 (AB026891, NM_014331) together with SLC3A2 form a heterodimer constituting the cystine/glutamate transporter system x_{c}⁻, which provides in many cell types cystine for the synthesis of glutathione. It is Na⁺ independent and electroneutral and obeys an obligatory exchange mode, exchanging extracellular anionic cystine (pH dependence) for glutamate with a stoichiometry of 1:1.

**System** x_{c}⁻ **inhibitors** for use in the present method include small molecule inhibitors such as, but not limited to,
sulfasalazine (SAS),
erastin and erastin analogs,
azo-linked amino-naphthyl-sulfonate analogs (AANS) of sulfasalazine,
ethyl 2-hydroxy-5-[(E)-2-4{4-[(pyridine-2-yl)sulfamoyl]phenyl}diazen-1-yl]-benzoate,
2-Hydroxy-5-[(E)-2-{4-[(pyridine-2-yl)sulfamoyl]phenyl}diazen-1-yl]benzamide,
4-[(E)-2-(2-Hydroxynaphthalen-1-yl)diazen-1-yl]benzene-1-sulfonic acid, 4-nitro-N-phenylbenzene-1-sulfonamide,
2-Hydroxy-5-(3-phenylpropanamido)benzoic acid,
HG106,
glutamate α-aminoadipic acid,
α-aminosuberic acid,
cystathionine,
ibotenate,
quisqualate,
L-β-N-oxalyl-L-α,β-diaminopropionate (L-β-ODAP),
L-alanosine, β-N-methylamino-L-alanine (BMAA),
L-serine-O-sulphate, L-α-aminopimelate,
L-homocysteate,
S-sulpho-I-cysteine,
S-4-carboxyphenylglycine,
S-4-carboxy,3-hydroxy-phenylglycine,
S-3-carboxy,3-hydroxy-phenylglycine,
R,S-sulphothienylglycine,
[(R,S)-4-[4'-carboxyphenyl]-phenylglycine,
5-benzyl-4-bis-TFM-HMICA,
5-naphthyl-4-bis-TFM-HMICA,
5-4-TFM-benzyl-4-bis-TFM-HMICA,
R,S-4-Br-homoibotenate, S-2-naphthyl-ethyl-ACPA (NACPA),
bis-trifluoromethyl-phenyl-isoxazole-4-hydrazone (TFMIH),
5-naphthylethyl isoxazole-4-(2,4-dinitrophenol)hydrazone-dinitrophenol (NEIH),
sorafenib.

**"Erastin"** and **"erastin analogs"** are compounds which have the ability to induce ferroptosis through blocking the function of system x_{c}⁻.

Examples for erastin analogs are preferably piperazine erastin (PE) and carbonyl erastin analogs (Larraufie et al., Bioorg Med Chem Lett. 2015; 25(21): 4787-4792), such as: ketone erastin (KE), α-fluoro ketone erastin (FKE), trifluoroketone erastin (TFKE), morpholine ketone erastin (MKE), N-methylpiperazine ketone erastin (MPKE), N-allylpiperazine ketone erastin (APKE), N-p-methoxybenzylpiperazine ketone erastin (PMB-PKE), aldehyde erastin (AE), imidazole ketone erastin (IKE), piperazine ketone erastin (PKE).

Piperazine erastin has a piperazine moiety on the meta position of the aniline ring of erastin, whereas carbonyl erastin analogs have reactive carbonyl moieties in the meta position of the aniline-derived moiety of erastin.

The **"azo amino-naphthylene sulfonic acid (AANS) analogs of sulfasalazine"** have been described by Nesher et al, (Neurochemical research, 2020, 45, 1375) as potent inhibitor of system xc, and are named AANS #2 - 4, which are bis-AANS analogs, and AANS #5 - 7, which are symmetrical bis-azo analogues having dual AANS substituents positioned at the distal ends of the molecule. The structure of AANS #2-7 and SAS are reported in the following Table 1, together with the respective IC50, as reported by Nesher et al. 2020.

**Table 1**

| **Compound** | **Structure** | **IC₅₀(**_{µ}**M), mean±SE M (n)** |
|---|---|---|
| Sulfasalazine | 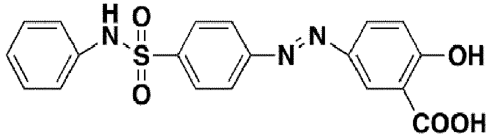 | 10.4 ± 1.3 (7) |
| **AANS#2** | 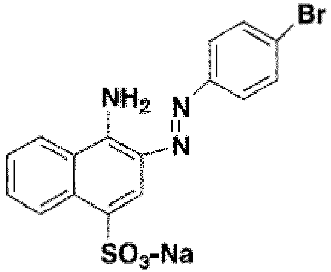 | 25 ± 4 |
| **AANS#3** | 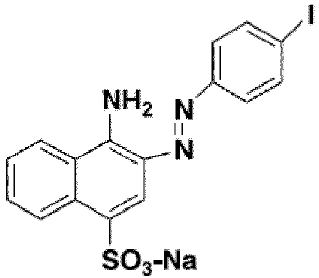 | 14 ± 2 |
| **AANS#4** | 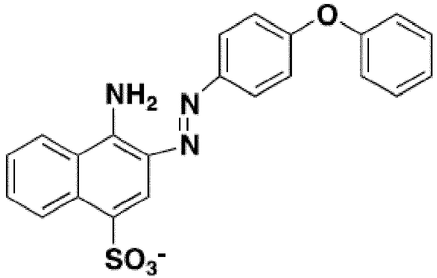 | 14 ± 2 |
| **AANS#5** | 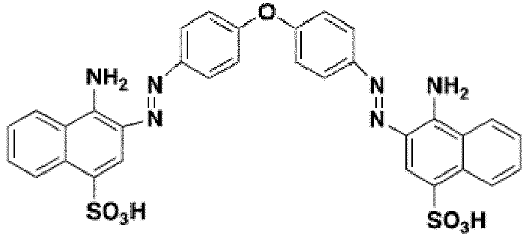 | 11 ± 1.3 (5) |
| **AANS#6** | 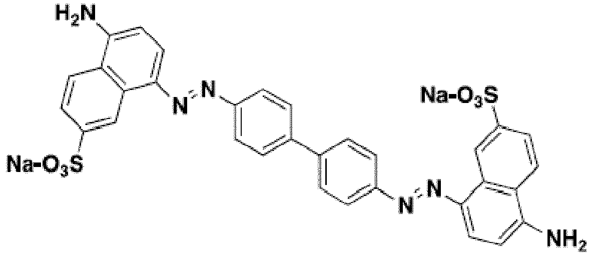 | 4.3 ± 0.8 (5) |
| **AANS#7** | 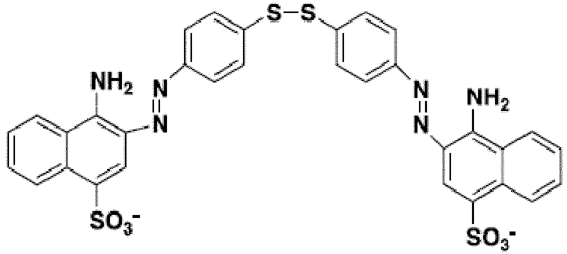 | 4.3 ± 1.5 (3) |

The compounds ethyl 2-hydroxy-5-[(E)-2-4{4-[(pyridine-2-yl)sulfamoyl]phenyl}diazen-1-yl]-benzoate **(4),** 2-Hydroxy-5-[(E)-2-{4-[(pyridine-2-yl)sulfamoyl]phenyl}diazen-1-yl]benza-mide **(5),** 4-[(E)-2-(2-Hydroxynaphthalen-1-yl)diazen-1-yl]benzene-1-sulfonic acid **(15),** 4-nitro-N-phenylbenzene-1-sulfonamide(**19**), 2-Hydroxy-5-(3-phenylpropanamido)benzoic acid **(12),** are sulfasalazine analogs described by Patel at al, Drug Dev Res, 2019, 80, 758-777. The structure and IC₅₀ of these sulfasalazine analogs are reported in the following **Table 2.**

| | |
|---|---|
| **Compound** | 2-hydroxy-5-[(E)-2-4{4-[(pyridine-2-yl)sulfamoyl]phenyl}diazen-1-yl]-benzoate |
| **Structure** | 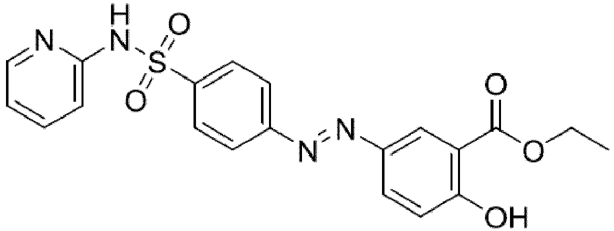 |
| **Compound** | 2-Hydroxy-5-[(E)-2-{4-[(pyridine-2-yl)sulfamoyl]phenyl}diazen-1-yl]benza-mide |
| **Structure** | 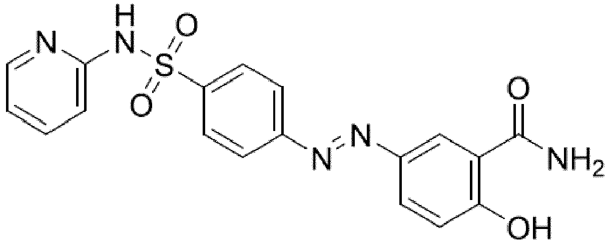 |
| **Compound** | 2-Hydroxy-5-(3-phenylpropanamido)benzoic acid |
| **Structure** | 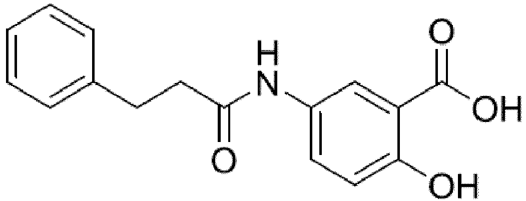 |
| **Compound** | 4-[(E)-2-(2-Hydroxynaphthalen-1-yl)diazen-1-yl]benzene-1-sulfonic acid |
| **Structure** | 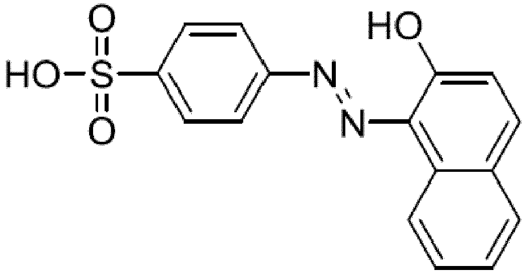 |
| **Compound** | 4-nitro-N-phenylbenzene-1-sulfonamide |
| **Structure** | 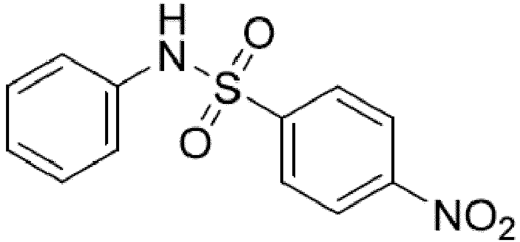 |

Thus, a preferred embodiment of the invention is a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and
   an inhibitor targeting STAT5,
wherein the autoimmune disease is mediated by plasmacytoid dendritic cells, and wherein at least one inhibitor of the antiporter system x_{c}⁻ is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methyiamino-L-alanine, L-serine-O-sulphate, L-α-aminopimelate, L-homocysteate, S-sulpho-I-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthylethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib.

A more preferred embodiment of the invention is a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and
   an inhibitor targeting STAT5,
wherein the autoimmune disease is mediated by plasmacytoid dendritic cells, and wherein the at least one inhibitor of the antiporter system x_{c}⁻ is sulfasalazine.

### Second inhibitor component of the combination

The second component of the combination according to the present invention is at least one inhibitor selected from the group consisting of:
a JAK2 inhibitor,
a SLC7A5:SLC3A2 inhibitor,
an inhibitor targeting IL-3 receptor alpha,
an inhibitor targeting IL-3 receptor beta,
an inhibitor targeting GM-CSF,
an inhibitor targeting GM-CSF receptor alpha, and
an inhibitor targeting STAT5.

A **"JAK2 inhibitor,"** as used herein, includes any compound that disrupts JAK2 activity or production and or the JAK2/STAT signaling pathway. Examples of JAK2 inhibitors include, but are not limited to, baricitinib, delgocitinib, fedratinib, gandotinib, momelotinib, oclacitinib, pacritinib, peficitinib, ruxolitinib, tofacitinib, AZD1480.

The heterodimer **"SLC7A5:SLC3A2"** constitutes the system LAA (large amino acids) transporter. The SLC7 family is divided into two sub-groups, the cationic amino acid transporters (the CAT family, SLC7A1-4) and the glycoprotein-associated amino acid transporters (the gpaAT family, SLC7A5-11), also called light chains or catalytic chains of the heterodimeric amino acid transporters (HAT). The CAT transporters subfamily members have 14 putative transmembrane (TM) segments and are glycosylated, whereas the gpaAT transporters have two TM segments less, are not glycosylated, and need to associate with a glycoprotein of the SLC3 family, heavy chains 4F2hc (CD98, SLC3A2) or rBAT (related to b0,+AT-type amino acid transporter, where b0,+AT is the basic and neutral amino acid transporter) for surface expression.
SLC7A5 (also named LAT1, L-type amino acid transporter-1, NM_003486) was identified on the basis of its capability to transport large neutral aminoacids into *Xenopus oocytes* when expressed with SLC3A2 (NM_001012662.3). As expected for system L, this HAT protein also transports 2-(-)-endoamino-bicycloheptane-2-carboxylic acid (BCH) and its function is not Na⁺ sensitive. Its uptake selectivity range is relatively broad and the apparent affinity for the uptake of branched and aromatic amino acids is quite high (micromolar range). SLC7A5:SLC3A2 is an obligatory exchanger that does not mediate any measurable amino acid efflux in the absence of extracellular amino acids, i.e. no facilitated diffusion. Predominant substrates of SLC7A5:SLC3A2 are large neutral I-amino acids, T3, T4, L-DOPA, BCH.

**"SLC7A5:SLC3A2 inhibitors"** for use in the present method include small molecule inhibitors such as, but not limited to,
**JPH203** (KYT-0353), a tyrosine derivative;
**triiodothyronine (T3) analogs:** 3,3',5-triiodothyronine (L-T3), 3',5',3-triodothyronine (r L-T3), 3-iodo-L-tyrosine; 3,5-diiodo-L-tyrosine, SKN102-105;
**tetraiodothyronine** (L-T4), which is a thyroxine (T4) analog;
**1,2,3-dithiazole derivatives,** described below;
**1,2,4-dithiazine derivatives,** described below;
**phenylalanine** ;
**phenylalanine derivates,** described below;
**tryptophan analogs:** tryptophan prodrug of valproic acid, L-tryptophan prodrug of benzoic acid;
2-aminobicyclo-(2,2,1)-heptane-2-carboxylic acid, **BCH;**
(S)- 2-Amino-3-(3-((2,4-dicyano-3-(4-(2-(methylamino)-2-oxoethoxy)phenyl) benzo[4,5]imidazo[1,2-a]pyridin-1-yl)- carbamoyl)phenyl)propanoic acid 1 **(KMH-233);**
**2-amino-3-(3-benzylphenyl)propanoic acid,** a phenylalanine analog
**2-amino-3-(3-benzyl-4-hydroxyphenyl)propanoic acid,** a tyrosine analog **HA-Phe, HA-Leu, HA-lle, HA-Met:** hydroxamic acids (HA) conjugated to SLC7A5 substrates Phe, Tyr, Leu, Ile, and Met, for example by replacing carboxylic acid in phenylalanine with HA;
**baclofen,** β-(4-chlorophenyl)-γ-aminobutyric acid (β-(4-chlorophenyl)-GABA), is a derivative of the neurotransmitter γ-aminobutyric acid (GABA);
**gabapentin,** an analogue of the neurotransmitter GABA;
**acivicin,** a glutamine analog, (2S)-Amino[(5S)-3-chloro-4,5-dihydro-1,2-oxazol-5-yl]ethanoic acid;
**fenclonine,** also known as para-chlorophenylalanine (PCPA);
**L-DOPA,** also known as levodopa and L-3,4-dihydroxyphenylalanine, is an amino acid that is made and used as part of the normal biology of humans;
**KHK2898,** a fully human anti CD98 (SLC3A2) monoclonal antibody, **IGN523,** a human anti CD98 (SLC3A2) monoclonal antibody

**JPH203** (KYT-0353), is a tyrosine derivative and a known selective L-type amino acid transporter 1 inhibitor. JPH203 is the abbreviation for (S)-2-Amino-3-(4-((5-amino-2-phenylbenzo[d]oxazol-7-yl)methoxy)-3,5-dichlorophenyl)propanoic acid.

**1,2,3-dithiazole derivatives** are compounds which have 1,2,3-dithiazoles, primary aromatic or heteroaromatic amines or alcohols that are reacted with 4,5-dichloro-1,2,3-dithiazolium chloride (Appel's salt) I, followed by treatment with a base (2 equiv) to give the corresponding [(4-chloro-5H-1,2,3-dithiazol-5-ylidene)amino]arenes or 2-(4-chloro-5H-1,2,3-dithiazol-5-ylidene)arene-1(2H)-ones.
Examples for **1,2,3-dithiazole derivatives** are compound 5, 10, 11, 12, 16, 17, 19 described by Napolitano et al, (Biochemical Pharmacology, 2017, 143: 39-52),shown in **Table 3** below.

**1,2,4-dithiazine derivatives** are compounds synthetized by reaction of the appropriate (Z)-N-(4-chloro-5H-1,2,3-dithiazol-5-ylidene)-1H-pyrazol-5-amine III with diethylamine followed by treatment with concentrated H₂SO₄.

Example for 1,2,4-dithiazine derivatives is compound 59, described by Napolitano et al, (Biochemical Pharmacology, 2017, 143: 39-52),shown in **Table 3** below.

**Table 3**

| **Compound** | **Structure** | **% Inhibition at 100 µM** |
|---|---|---|
| **5** | 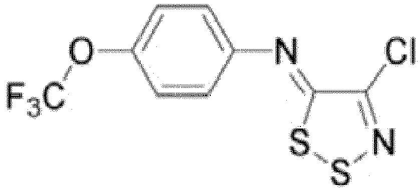 | > 90 % |
| **10** | 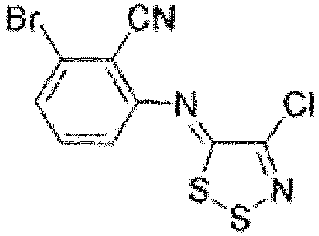 | > 90 % |
| **11** | 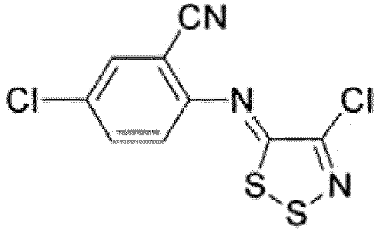 | > 90 % |
| **12** | 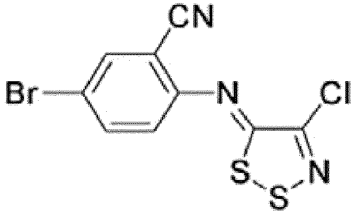 | > 90 % |
| **16** | 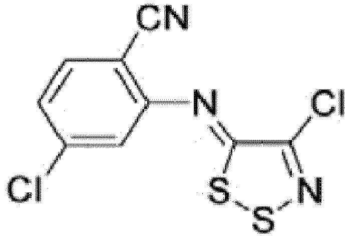 | .> 90 % |
| **17** | 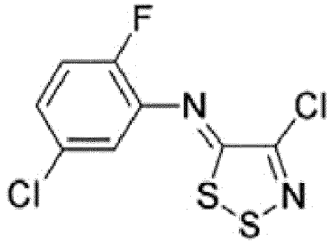 | .> 90 % |
| **19** | 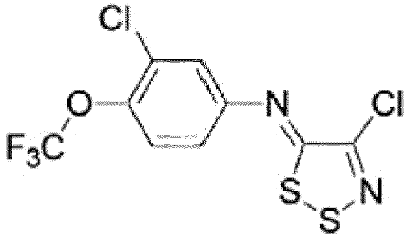 | > 90 % |
| **59** | 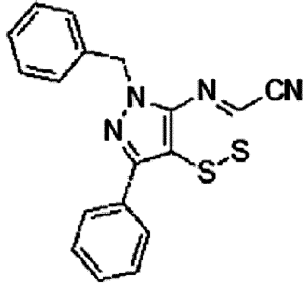 | > 90 % |

Examples for **phenylalanine derivatives** are 3-([1,10-biphenyl]-3-yl)-2-aminopropanoic acid, and compounds 28, 36, 42 (reported below, Singh et al, Int J Mol Sci, 2019);

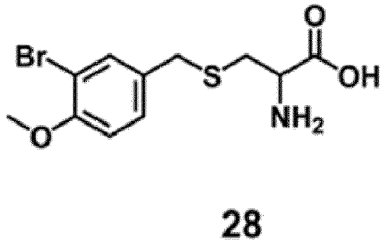

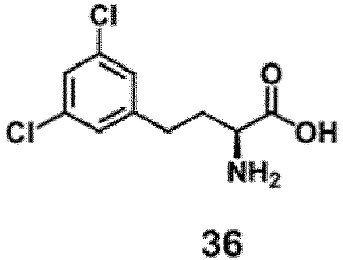

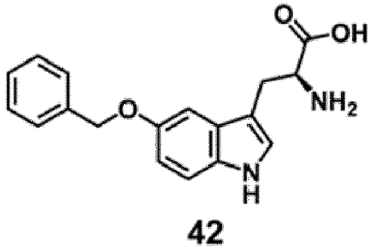

**"Nitrogen mustard derivatives of phenylalanine"** comprise **p-N-di-(2-chloroethyl)aminophenylalanine, melphalan** ((2S)-2-amino-3-{4-[bis(2-chloroethyl)amino]phenyl}propanoic acid), **o-Sarcolysin, m-Sarcolysin,** (sarcolysin is an isomeric form of melphalan), **DL-2-NAM-7** ((2-amino-7-bis[(2-chloroethyl)amino]-1,2,3,4-tetrahydro-2-naphthoic acid).

**IL-3 receptor** is comprised of a ligand specific alpha subunit (IL3RA or IL3R-alpha) and a signal transducing beta subunit (IL3RB, or IL3R-beta) shared by the receptors for interleukin 3 (IL-3), colony stimulating factor 2 (CSF2/GM-CSF), and interleukin 5 (IL5). IL-3R-alpha is a type I transmembrane protein with a deduced molecular weight of about 41 kDa containing an extracellular domain involved in IL-3 binding, a transmembrane domain and a short cytoplasmic tail of about 50 amino acids.
An **"inhibitor targeting IL-3 receptor alpha"** is preferably an inhibitor able to interact with IL-3 receptor alpha chain and blocking its binding with the ligand IL-3. An **"inhibitor targeting IL-3 receptor beta"** is also preferably an inhibitor able to interact with IL-3 receptor beta chain causing blocking signal transduction upon binding of IL-3 to the receptor and the biological activities of IL-3.
An **"inhibitor targeting IL-3 receptor alpha"** as used in present invention is selected from the group comprising: CSL362 antibody, flotetuzumab, IL3LDM, DT388IL3, SGN-CD123A. An **"inhibitor targeting IL-3 receptor beta"** as used in present invention is preferably CSL311 antibody.

**"Granulocyte macrophage-colony stimulating factor"** or **"GM-CSF"** refers to a family of glycoprotein growth factors that control the production, differentiation, and function of granulocytes and monocytes-macrophages. **GM-CSF** was originally discovered as a protein with the capacity to generate both granulocyte and macrophage colonies from precursor cells in mouse bone marrow, and was accordingly named. Subsequent studies have demonstrated a role of GM-CSF in potentiating the function of mature macrophages and granulocytes, suggesting a role for GM-CSF in inflammatory responses. Moreover, GM-CSF has other functions arising from its ability to affect the properties of more mature myeloid cells such as granulocytes, macrophages and eosinophils. The functions of GM-CSF are mediated by binding to CD116, the granulocyte-macrophage colony stimulating factor receptor, also known as colony stimulating factor 2 receptor alpha (GM-CSFRa, GM-CSF receptor alpha) that binds GM-CSF with low affinity. The beta subunit, called CD131, which is also shared with the IL-3 and IL-5 receptors, has no detectable binding activity for GM-CSF by itself but is necessary for high affinity binding when in association with the alpha subunit and plays a fundamental role in signal transduction.

An **"inhibitor targeting GM-CSF"** is a compound, more preferably an antibody, that can antagonize the activity of GM-CSF.
An **"inhibitor targeting GM-CSF"** as used in present invention is selected from otilimab (MOR103/GSK3196165), namilumab (MT203), lenzilumab (KB003), plonmarlimab (TJ003234 or TJM2), and gimsilumab.
An **"inhibitor targeting GM-CSF receptor alpha"** is a compound, more preferably an antibody or blocking antibody, that can neutralise the biological activity of GM-CSF receptor alpha by binding to GM-CSF receptor alpha or by inhibiting binding of GM-CSF to GM-CSF receptor alpha.
An **"inhibitor targeting GM-CSF receptor alpha"** as used in present invention is mavrilimumab.

In normal cells, **STAT5** protein activation is tightly regulated by cytokines (IL-3, IL-2, IL-5, IL-7, GM-CSF, erythropoietin, thrombopoietin and prolactin) and growth factors. Binding of these extracellular ligands to the target receptor induces activation of receptor-associated JAK kinase, which induces STAT5 phosphorylation. Phosphorylated STAT5 monomers form homo- or hetero-STAT5-STATX dimers due to the interaction between phosphorylated tyrosine and SH2. Activated STAT5 dimers migrate to the nucleus where they bind to STAT5 DNA response elements and Induces transcription of specific genes involved in controlled cell growth and division, cell proliferation, programmed cell death, or apoptosis, cell specialization, or differentiation and inflammation.

An **"inhibitor targeting STAT5"** is a compound that neutralizes biological activity of STAT5. As used herein, an **"inhibitor targeting STAT5"** is a monovalent small molecule that degrades STAT5. Preferably, an **"inhibitor targeting STAT5"** is selected from the group comprising:
- **JPX1188, JPX0802, JPX1185** (Janpix, https://www.janpixbio.com)
- **compound 17f** (Juen et al., J. Med. Chem. 2017, 60, 14, 6119-6136) 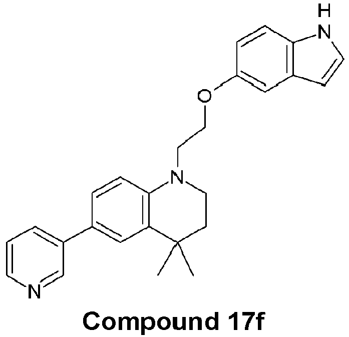 - **compound AC-4-130** (Wingelhofer et al., Leukemia 2018, 32, 1135 - 1146) 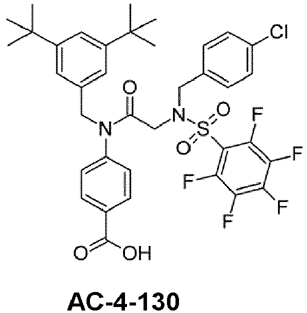 - N'-((4-Oxo-4H-chromen-3-yl)methylene)nicotinohydrazide, also referred to as CAS 285986-31-4**,** (Muller et al., Chembiochem 2008, 9(5):723 - 727).
- **BP-1-075, BP-1-107, BP-1-108, SF-1-087, SF-1-088** (Page et al., J. Med. Chem. 2012, 55, 3, 1047 - 1055), of general Formula: 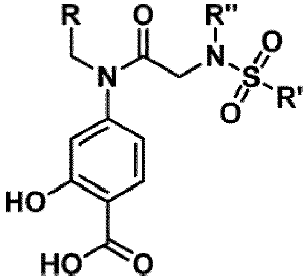 wherein:

| Compound | R | R' | R" |
|---|---|---|---|
| BP-1-075 | 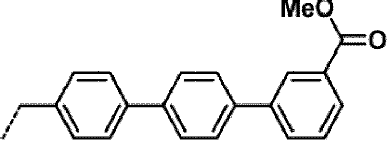 | 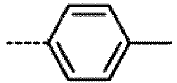 | - CH₃ |
| BP-1-107 | 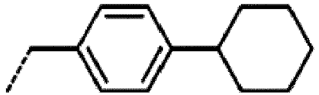 | 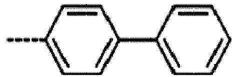 | - CH₃ |
| BP-1-108 | | 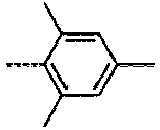 | - CH₃ |
| SF-1-087 | | 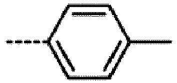 | - Boc |
| SF-1-088 | 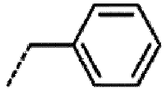 | | - Boc |

- N1-(11H-indolo[3,2-c]quinolin-6-yl)-N2,N2-dimethylethane-1,2-diamine, also referred to as **IQDMA** (Chien, et al., Chemico-Biological Interactions 2008, 176, 1, 40-47), of Formula: 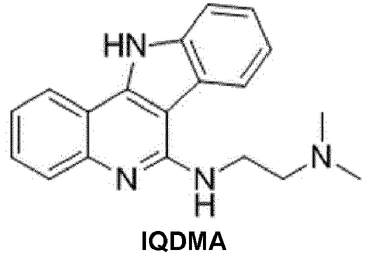 - pimozide, also referred to as CAS 2062-78-4**,** 1-(1-(4,4-Bis(4-fluorophenyl)butyl) -4-piperidinyl)-1,3-dihydro-2H-benzimidazol-2-one, (Nelson et al., Blood 2011, 117 (12): 3421-3429).

Thus, an **"inhibitor targeting STAT5"** is JPX1188, JPX0802, JPX1185, compound 17f, compound AC-4-130, CAS 285986-31-4, BP-1-075, BP-1-107, BP-1-108, SF-1-087, SF-1-088, IQDMA, or CAS 2062-78-4.

A preferred embodiment of the invention is directed to a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and
   an inhibitor targeting STAT5,
   wherein the autoimmune disease is mediated by plasmacytoid dendritic cells; and wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
   wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine; KHK2898 antibody, IGN523 antibody; or
   wherein the inhibitor targeting IL-3 receptor alpha is selected from the group comprising CSL362 antibody, flotetuzumab, IL3LDM, DT388IL3, and SGN-CD123A,; or
   wherein the inhibitor targeting IL-3 receptor beta is CSL311 antibody, or wherein the inhibitor targeting GM-CSF is otilimab, namilumab, lenzilumab, plonmarlimab, or gimsilumab; or
   wherein the inhibitor targeting GM-CSF receptor alpha is mavrilimumab; or wherein the inhibitor targeting STAT5 is JPX1188, JPX0802, JPX1185, compound 17f, compound AC-4-130, CAS 285986-31-4, BP-1-075, BP-1-107, BP-1-108, SF-1-087, SF-1-088, IQDMA, or CAS 2062-78-4.

A more preferred embodiment of the invention is directed to a combination for use in the treatment of an autoimmune disease, wherein the combination consists of:
at least one inhibitor of antiporter system x_{c}⁻ and
at least one inhibitor JAK2 inhibitor,
wherein the autoimmune disease is mediated by plasmacytoid dendritic cells; and wherein the at least one JAK2 inhibitor is barcitinib.

A still more preferred embodiment of the invention is directed to a combination for use in the treatment of an autoimmune disease, wherein the combination consists of:
at least one inhibitor of antiporter system x_{c}⁻ and
at least one inhibitor JAK2 inhibitor,
wherein the autoimmune disease is mediated by plasmacytoid dendritic cells; and wherein the at least one JAK2 inhibitor is delgocitinib.

In another aspect, the present invention is directed to a combination for use in the treatment of an autoimmune disease, wherein the combination consists of:
at least one inhibitor of antiporter system x_{c}⁻ and
at least one SLC7A5:SLC3A2 inhibitor,
wherein the autoimmune disease is mediated by plasmacytoid dendritic cells; and wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine; KHK2898 antibody, IGN523 antibody.

In still another aspect, the present invention is directed to a combination for use in the treatment of an autoimmune disease, wherein the combination consists of:
at least one inhibitor of antiporter system x_{c}⁻ and
at least one inhibitor targeting IL-3 receptor alpha or at least one inhibitor targeting IL-3 receptor beta
wherein the autoimmune disease is mediated by plasmacytoid dendritic cells; and wherein the inhibitor targeting IL-3 receptor alpha is selected from the group comprising CSL362 antibody, flotetuzumab, IL3LDM, DT388IL3, and SGN-CD123A;
wherein the inhibitor targeting IL-3 receptor beta is CSL311 antibody.

In a particular aspect, the present invention is directed to a combination for use in the treatment of an autoimmune disease, wherein the combination consists of:
at least one inhibitor of antiporter system x_{c}⁻ and
at least one inhibitor targeting GM-CSF,
wherein the autoimmune disease is mediated by plasmacytoid dendritic cells; wherein the inhibitor targeting GM-CSF is otilimab, namilumab, lenzilumab, plonmarlimab, or gimsilumab.

In a further particular aspect, the present invention is directed to a combination for use in the treatment of an autoimmune disease, wherein the combination consists of:
at least one inhibitor of antiporter system x_{c}⁻ and
at least one inhibitor targeting GM-CSF receptor alpha,
wherein the autoimmune disease is mediated by plasmacytoid dendritic cells; and wherein the inhibitor targeting GM-CSF receptor alpha is mavrilimumab.

In a further more particular aspect, the present invention is directed to a combination for use in the treatment of an autoimmune disease, wherein the combination consists of:
at least one inhibitor of antiporter system x_{c}⁻ and
at least one inhibitor targeting STAT-5,
wherein the autoimmune disease is mediated by plasmacytoid dendritic cells; and wherein the inhibitor targeting STAT5 is JPX1188, JPX0802, JPX1185, compound 17f, compound AC-4-130, CAS 285986-31-4, BP-1-075, BP-1-107, BP-1-108, SF-1-087, SF-1-088, IQDMA, or CAS 2062-78-4.

A further more preferred embodiment of the invention is directed to a combination for use in the treatment of an autoimmune disease, wherein the combination consists of:
at least one inhibitor of antiporter system x_{c}⁻ and
at least one inhibitor selected from the group consisting of:
   a JAK2 inhibitor, and
   a SLC7A5:SLC3A2 inhibitor,
wherein the autoimmune disease is mediated by plasmacytoid dendritic cells, and
wherein at least one inhibitor of the antiporter system x_{c}⁻ is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-l-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthyl-ethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib.

A further more preferred embodiment of the invention is directed to a combination for use in the treatment of an autoimmune disease, wherein the combination consists of:
at least one inhibitor of antiporter system x_{c}⁻ and
at least one inhibitor selected from the group consisting of:
   a JAK2 inhibitor, and
   a SLC7A5:SLC3A2 inhibitor,
wherein the autoimmune disease is mediated by plasmacytoid dendritic cells, and
wherein at least one inhibitor of the antiporter system x_{c}⁻ is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-l-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthyl-ethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib, and
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids- phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine; KHK2898 antibody, IGN523 antibody.

A still more preferred embodiment of the invention is directed to a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and
   an inhibitor targeting STAT5,
wherein the autoimmune disease is mediated by plasmacytoid dendritic cells, and
wherein at least one inhibitor of the antiporter system x_{c}⁻ is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-l-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthyl-ethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib, and
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine; KHK2898 antibody, IGN523 antibody; or
wherein the inhibitor targeting IL-3 receptor alpha is selected from the group comprising CSL362 antibody, flotetuzumab, IL3LDM, DT388IL3, SGN-CD123A; or
wherein the inhibitor targeting IL-3 receptor beta is CSL311 antibody, or wherein the inhibitor targeting GM-CSF is otilimab, namilumab, lenzilumab, plonmarlimab, or gimsilumab; or
wherein the inhibitor targeting GM-CSF receptor alpha is mavrilimumab; or wherein the inhibitor targeting STAT5 is JPX1188, JPX0802, JPX1185, compound 17f, compound AC-4-130, CAS 285986-31-4, BP-1-075, BP-1-107, BP-1-108, SF-1-087, SF-1-088, IQDMA, or CAS 2062-78-4.

A further preferred embodiment of the invention is a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and
   an inhibitor targeting STAT5,
wherein the autoimmune disease is mediated by plasmacytoid dendritic cells, and wherein the at least one inhibitor of the antiporter system x_{c}⁻ is sulfasalazine and
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine; KHK2898 antibody, IGN523 antibody; or
wherein the inhibitor targeting IL-3 receptor alpha is selected from the group comprising CSL362 antibody, flotetuzumab, IL3LDM, DT388IL3, and SGN-CD123A; or
wherein the inhibitor targeting IL-3 receptor beta is CSL311 antibody, or wherein the inhibitor targeting GM-CSF is otilimab, namilumab, lenzilumab, plonmarlimab, or gimsilumab; or
wherein the inhibitor targeting GM-CSF receptor alpha is mavrilimumab; or wherein the inhibitor targeting STAT5 is JPX1188, JPX0802, JPX1185, compound 17f, compound AC-4-130, CAS 285986-31-4, BP-1-075, BP-1-107, BP-1-108, SF-1-087, SF-1-088, IQDMA, or CAS 2062-78-4.

A still more preferred embodiment of the invention is directed to a combination for use in the treatment of an autoimmune disease, wherein the combination consists of:
at least one inhibitor of antiporter system x_{c}⁻ and
at least one JAK2 inhibitor,
wherein the autoimmune disease is mediated by plasmacytoid dendritic cells, and
wherein at least one inhibitor of the antiporter system x_{c}⁻ is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-l-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthyl-ethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib, and
wherein the at least one JAK2 inhibitor is barcitinib.

A still more preferred embodiment of the invention is directed to a combination for use in the treatment of an autoimmune disease, wherein the combination consists of:
at least one inhibitor of antiporter system x_{c}⁻ and
at least one JAK2 inhibitor,
wherein the autoimmune disease is mediated by plasmacytoid dendritic cells, and
wherein at least one inhibitor of the antiporter system x_{c}⁻ is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-l-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthyl-ethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib, and
wherein the at least one JAK2 inhibitor is delgocitinib.

A more preferred embodiment of the invention is a combination for use in the treatment of an autoimmune disease, wherein the combination consists of:
at least one inhibitor of antiporter system x_{c}⁻ and
at least one JAK2 inhibitor,
wherein the autoimmune disease is mediated by plasmacytoid dendritic cells, and wherein the at least one inhibitor of the antiporter system x_{c}⁻ is sulfasalazine, and
wherein the at least one JAK2 inhibitor is barcitinib.

A further more preferred embodiment of the invention is a combination for use in the treatment of an autoimmune disease, wherein the combination consists of:
at least one inhibitor of antiporter system x_{c}⁻ and
at least one JAK2 inhibitor,
wherein the autoimmune disease is mediated by plasmacytoid dendritic cells, and wherein the at least one inhibitor of the antiporter system x_{c}⁻ is sulfasalazine, and
wherein the at least one JAK2 inhibitor is delgocitinib.

An **"autoimmune diseases",** as pertains to the present invention, is a disease or disorder arising from and directed against an individual's own tissues or a co-segregate or manifestation thereof or resulting condition therefrom. This class of disorders is highly varied, both between and within different kinds of autoimmune diseases, which complicates diagnosis and effective treatment. The causes of autoimmune diseases are also poorly understood, which results in courses of treatment that focus primarily on the symptoms. An **"autoimmune disease mediated by plasmacytoid dendritic cells"** refers to an autoimmune disease characterized by a chronic activation of plasmacytoid dendritic cells releasing high amounts of type I interferon (IFN-I). Thus, an **"autoimmune disease mediated by plasmacytoid dendritic cells"** is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-lgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

**"Systemic Lupus Erythematosus"** (SLE) is a debilitating autoimmune disease that can damage multiple organs, induce chronic renal failure, and lead to severe morbidity and mortality. A characteristic feature of SLE is the presence of antinuclear autoantibodies that form immune complexes with cellular debris and cause end-organ damage. Current treatment regimens are limited to non-specific immune suppression and management of inflammatory symptoms.

The term **"lupus"** as used herein is an autoimmune disease or disorder that in general involves antibodies that attack connective tissue. The principal form of lupus is a systemic one, systemic lupus erythematosus (SLE); including cutaneous SLE and subacutecutaneous SLE, as well as other types of lupus including nephritis, extrarenal, cerebritis, pediatric, non-renal, discoid, and alopecia. In certain embodiments, the term **"systemic lupus erythematosus"** refers to a chronic autoimmune disease that can result in skin lesions, joint pain and swelling, kidney disease (lupus nephritis), fluid around the heart and/or lungs, inflammation of the heart, and various other systemic conditions. In certain embodiments, the term **"lupus nephritis"** refers to inflammation of the kidneys that occurs in patients with SLE. Lupus nephritis may include, for example, glomerulonephritis and/or interstitial nephritis, and can lead to hypertension, proteinuria, and kidney failure. Lupus nephritis classes include class I (minimal mesangial lupus nephritis), class II (mesangial proliferative lupus nephritis), class 111 (focal lupus nephritis), class IV (diffuse segmental (IV-S) or diffuse global (IV-G) lupus nephritis), class V (membranous lupus nephritis), and class VI (advanced sclerosing lupus nephritis). The term "lupus nephritis" encompasses all of the classes.

The term **"conditions induced by SLE"** or **"SLE-induced conditions"** includes, for example serositis, malar rash (rash over the cheeks and bridge of the nose), discoid rash (scaly, disk-shaped sores on the face, neck and chest), sensitivity to light, alopecia, Raynaud's syndrome (sensitivity to cold), sores or ulcers (on the tongue, in the mouth or nose), abdominal pain, including peritonitis and bowel infarction, lupus hepatitis, hemolytic anemia, low lymphocytic count, low platelet count, leukopenia (low white blood cells count), the presence of antinuclear bodies in the blood, skin lesions, central nervous system (CNS) effects including loss of memory, seizures, strokes and psychosis, CNS lupus, lung symptoms/effects including inflammation (pleuritis), chronic pneumonitis, chronic diffuse interstitial lung disease and scarring of the lungs, alternations in pulmonary vessel structure and function; hair loss, lupus nephritis, hematuria, proteinuria, fatigue, fever, nausea, dyspepsia, vomiting, diarrhoea, swollen glands, lack of appetite, weight loss, arthralgia and myalgia, arthritis, osteonecrosis, myopathy, atherosclerotic plaque, pericarditis, vasculitis, keratoconjunctivitis sicca, lupus retinopathy, pregnancy complications with increased rate of fetal death in utero, thyroid dysfunction,

Thus, an embodiment of the invention relates to a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and
   an inhibitor targeting STAT5,
wherein the autoimmune disease is mediated by plasmacytoid dendritic cells; and wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-lgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

A further embodiment of the invention relates to a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and
   an inhibitor targeting STAT5,
wherein at least one inhibitor of the antiporter system x_{c}⁻ is selected from the group comprising erastin, imidazole ketone erastin, erastin analogs, HG106, glutamate α-aminoadipic acid, α-aminosuberic acid, cystathionine, ibotenate, quisqualate, L-β-N-oxalyl-L-α,β-diaminopropionate (L-β-ODAP), L-alanosine, β-N-methylamino-L-alanine (BMAA), L-serine-O-sulphate, L-α-aminopimelate, L-homocysteate, S-sulpho-l-cysteine S-4-carboxy-phenylglycine, S-4-carboxy,3-hydroxy-phenylglycine, R,S-sulphothienylglycine, 5-benzyl-4-bis-TFM-HMICA, 5-naphthyl-4-bis-TFM-HMICA, 5-4-TFM-benzyl-4-bis-TFM-HMICA, (RS)-4-Br-homoibotenate, S-2-naphthyl-ethyl-ACPA (NACPA), bis-trifluoromethyl-phenyl-isoxazole-4-hydrazone (TFMIH), 5-naphthylethyl isoxazole-4-(2,4-dinitrophenol)hydrazone-dinitrophenol (NEIH), azo amino-naphthylene sulfonic acid (AANS) analogs, sulfasalazine, sulfasalazine derivatives, sorafenib;
wherein the autoimmune disease is mediated by plasmacytoid dendritic cells; and wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-lgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

A preferred embodiment of the invention relates to a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and an inhibitor targeting STAT5,
wherein the at least one inhibitor of the antiporter system x_{c}⁻ is sulfasalazine,
wherein the autoimmune disease is mediated by plasmacytoid dendritic cells; and
wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-lgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

A more preferred embodiment of the invention relates to a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and
   an inhibitor targeting STAT5,
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine; KHK2898 antibody, IGN523 antibody; or
wherein the inhibitor targeting IL-3 receptor alpha is selected from the group comprising CSL362 antibody, flotetuzumab, IL3LDM, DT388IL3, and SGN-CD123A; or
wherein the inhibitor targeting IL-3 receptor beta is CSL311 antibody, or wherein the inhibitor targeting GM-CSF is otilimab, namilumab, lenzilumab, plonmarlimab, or gimsilumab; or
wherein the inhibitor targeting GM-CSF receptor alpha is mavrilimumab; or
wherein the inhibitor targeting STAT5 is JPX1188, JPX0802, JPX1185, compound 17f, compound AC-4-130, CAS 285986-31-4, BP-1-075, BP-1-107, BP-1-108, SF-1-087, SF-1-088, IQDMA, or CAS 2062-78-4,
wherein the autoimmune disease is mediated by plasmacytoid dendritic cells; and
wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-lgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

A still more preferred embodiment of the invention relates to a combination for use in the treatment of an autoimmune disease, wherein the combination consists of:
at least one inhibitor of antiporter system x_{c}⁻ and
at least one JAK2 inhibitor,
wherein the at least one JAK2 inhibitor is barcitinib,
wherein the autoimmune disease is mediated by plasmacytoid dendritic cells; and
wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-lgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

A still more preferred embodiment of the invention relates to a combination for use in the treatment of an autoimmune disease, wherein the combination consists of:
at least one inhibitor of antiporter system x_{c}⁻ and
at least one JAK2 inhibitor,
wherein the at least one JAK2 inhibitor is delgocitinib,
wherein the autoimmune disease is mediated by plasmacytoid dendritic cells; and
wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-IgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

A further embodiment of the invention is directed to a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and
   an inhibitor targeting STAT5,
wherein at least one inhibitor of the antiporter system x_{c}⁻ is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-I-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthylethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib, and
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine; KHK2898 antibody, IGN523 antibody; or
wherein the inhibitor targeting IL-3 receptor alpha is selected from the group comprising CSL362 antibody, flotetuzumab, IL3LDM, DT388IL3, and SGN-CD123A; or
wherein the inhibitor targeting IL-3 receptor beta is CSL311 antibody, or wherein the inhibitor targeting GM-CSF is otilimab, namilumab, lenzilumab, plonmarlimab, or gimsilumab; or
wherein the inhibitor targeting GM-CSF receptor alpha is mavrilimumab; or wherein the inhibitor targeting STAT5 is JPX1188, JPX0802, JPX1185, compound 17f, compound AC-4-130, CAS 285986-31-4, BP-1-075, BP-1-107, BP-1-108, SF-1-087, SF-1-088, IQDMA, or CAS 2062-78-4; and wherein the autoimmune disease is mediated by plasmacytoid dendritic cells, and
wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-lgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

An embodiment of the invention is a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and
   an inhibitor targeting STAT5,
wherein the at least one inhibitor of the antiporter system x_{c}⁻ is sulfasalazine and
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine; KHK2898 antibody, IGN523 antibody; or
wherein the inhibitor targeting IL-3 receptor alpha is selected from the group comprising CSL362 antibody, flotetuzumab, IL3LDM, DT388IL3, and SGN-CD123A; or
wherein the inhibitor targeting IL-3 receptor beta is CSL311 antibody, or wherein the inhibitor targeting GM-CSF is otilimab, namilumab, lenzilumab, plonmarlimab, or gimsilumab; or
wherein the inhibitor targeting GM-CSF receptor alpha is mavrilimumab; or wherein the inhibitor targeting STAT5 is JPX1188, JPX0802, JPX1185, compound 17f, compound AC-4-130, CAS 285986-31-4, BP-1-075, BP-1-107, BP-1-108, SF-1-087, SF-1-088, IQDMA, or CAS 2062-78-4; and
wherein the autoimmune disease is mediated by plasmacytoid dendritic cells, and
wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-IgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

A preferred embodiment of the invention is directed to a combination for use in the treatment of an autoimmune disease, wherein the combination consists of:
at least one inhibitor of antiporter system x_{c}⁻ and
at least one JAK2 inhibitor,
wherein at least one inhibitor of the antiporter system x_{c}⁻ is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methyiamino-L-alanine, L-serine-O-sulphate, L-α-aminopimelate, L-homocysteate, S-sulpho-I-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthylethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib, and
wherein the at least one JAK2 inhibitor is barcitinib,
wherein the autoimmune disease is mediated by plasmacytoid dendritic cells, and
wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-IgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

A more preferred embodiment of the invention is directed to a combination for use in the treatment of an autoimmune disease, wherein the combination consists of:
at least one inhibitor of antiporter system x_{c}⁻ and
at least one JAK2 inhibitor,
wherein at least one inhibitor of the antiporter system x_{c}⁻ is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methyiamino-L-alanine, L-serine-O-sulphate, L-α-aminopimelate, L-homocysteate, S-sulpho-I-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthylethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib, and
wherein the at least one JAK2 inhibitor is delgocitinib,
wherein the autoimmune disease is mediated by plasmacytoid dendritic cells, and
wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-IgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

A more preferred embodiment of the invention is a combination for use in the treatment of an autoimmune disease, wherein the combination consists of:
at least one inhibitor of antiporter system x_{c}⁻ and
at least one JAK2 inhibitor,
wherein the at least one inhibitor of the antiporter system x_{c}⁻ is sulfasalazine and
wherein the at least one JAK2 inhibitor is barcitinib,
wherein the autoimmune disease is mediated by plasmacytoid dendritic cells, and
wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-IgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

A still more preferred embodiment of the invention is a combination for use in the treatment of an autoimmune disease, wherein the combination consists of:
at least one inhibitor of antiporter system x_{c}⁻ and
at least one JAK2 inhibitor,
wherein the at least one inhibitor of the antiporter system x_{c}⁻ is sulfasalazine and
wherein the at least one JAK2 inhibitor is delgocitinib,
wherein the autoimmune disease is mediated by plasmacytoid dendritic cells, and
wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-IgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

A more preferred embodiment of the invention relates to a combination for use in the treatment of an autoimmune disease, wherein the combination consists of:
at least one inhibitor of antiporter system x_{c}⁻ and
at least one inhibitor selected from the group consisting of:
   a JAK2 inhibitor, and
   a SLC7A5:SLC3A2 inhibitor,
wherein the autoimmune disease is mediated by plasmacytoid dendritic cells; and wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-IgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

A still more preferred embodiment of the invention relates to a combination for use in the treatment of an autoimmune disease, wherein the combination consists of:
at least one inhibitor of antiporter system x_{c}⁻ and
at least one inhibitor selected from the group consisting of:
   a JAK2 inhibitor, and
   a SLC7A5:SLC3A2 inhibitor,
wherein at least one inhibitor of the antiporter system x_{c}⁻ is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-l-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthyl-ethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib,
wherein the autoimmune disease is mediated by plasmacytoid dendritic cells; and wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-IgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

An embodiment of the invention relates to a combination for use in the treatment of an autoimmune disease, wherein the combination consists of:
at least one inhibitor of antiporter system x_{c}⁻ and
at least one inhibitor selected from the group consisting of:
   a JAK2 inhibitor, and
   a SLC7A5:SLC3A2 inhibitor,
wherein the at least one inhibitor of the antiporter system x_{c}⁻ is sulfasalazine,
wherein the autoimmune disease is mediated by plasmacytoid dendritic cells; and
wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-IgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

A preferred embodiment of the invention relates to a combination for use in the treatment of an autoimmune disease, wherein the combination consists of:
at least one inhibitor of antiporter system x_{c}⁻ and
at least one inhibitor selected from the group consisting of:
   a JAK2 inhibitor, and
   a SLC7A5:SLC3A2 inhibitor,
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids- phenylalanine, hydroxamic acids-leucine, hydroxamic acids-isoleucine, hydroxamic acids-methionine; β-(4-chlorophenyl)-y-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine; KHK2898 antibody, IGN523 antibody;
wherein the autoimmune disease is mediated by plasmacytoid dendritic cells; and
wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-IgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

Thus, an embodiment of the invention relates to a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and
   an inhibitor targeting STAT5,
wherein the autoimmune disease is mediated by plasmacytoid dendritic cells; and
wherein the autoimmune disease is systemic lupus erythematosus.

A further embodiment of the invention relates to a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and
   an inhibitor targeting STAT5,
wherein at least one inhibitor of the antiporter system x_{c}⁻ is selected from the group comprising erastin, imidazole ketone erastin, erastin analogs, HG106, glutamate α-aminoadipic acid, α-aminosuberic acid, cystathionine, ibotenate, quisqualate, L-β-N-oxalyl-L-α,β-diaminopropionate (L-β-ODAP), L-alanosine, β-N-methylamino-L-alanine (BMAA), L-serine-O-sulphate, L-α-aminopimelate, L-homocysteate, S-sulpho-l-cysteine S-4-carboxy-phenylglycine, S-4-carboxy,3-hydroxy-phenylglycine, R,S-sulphothienylglycine, 5-benzyl-4-bis-TFM-HMICA, 5-naphthyl-4-bis-TFM-HMICA, 5-4-TFM-benzyl-4-bis-TFM-HMICA, (RS)-4-Br-homoibotenate, S-2-naphthyl-ethyl-ACPA (NACPA), bis-trifluoromethyl-phenyl-isoxazole-4-hydrazone (TFMIH), 5-naphthylethyl isoxazole-4-(2,4-dinitrophenol)hydrazone-dinitrophenol (NEIH), azo amino-naphthylene sulfonic acid (AANS) analogs, sulfasalazine, sulfasalazine derivatives, sorafenib;
wherein the autoimmune disease is mediated by plasmacytoid dendritic cells; and
wherein the autoimmune disease is systemic lupus erythematosus.

A preferred embodiment of the invention relates to a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and
   an inhibitor targeting STAT5,
wherein the at least one inhibitor of the antiporter system x_{c}⁻ is sulfasalazine,
wherein the autoimmune disease is mediated by plasmacytoid dendritic cells; and
wherein the autoimmune disease is systemic lupus erythematosus.

A more preferred embodiment of the invention relates to a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and
   an inhibitor targeting STAT5,
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1 -yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine; KHK2898 antibody, IGN523 antibody; or
wherein the inhibitor targeting IL-3 receptor alpha is selected from the group comprising CSL362 antibody, flotetuzumab, IL3LDM, DT388IL3, and SGN-CD123A; or
wherein the inhibitor targeting IL-3 receptor beta is CSL311 antibody, or wherein the inhibitor targeting GM-CSF is otilimab, namilumab, lenzilumab, plonmarlimab, or gimsilumab; or
wherein the inhibitor targeting GM-CSF receptor alpha is mavrilimumab; or
wherein the inhibitor targeting STAT5 is JPX1188, JPX0802, JPX1185, compound 17f, compound AC-4-130, CAS 285986-31-4, BP-1-075, BP-1-107, BP-1-108, SF-1-087, SF-1-088, IQDMA, or CAS 2062-78-4,
wherein the autoimmune disease is mediated by plasmacytoid dendritic cells; and
wherein the autoimmune disease is systemic lupus erythematosus.

A still more preferred embodiment of the invention relates to a combination for use in the treatment of an autoimmune disease, wherein the combination consists of:
at least one inhibitor of antiporter system x_{c}⁻ and
at least one JAK2 inhibitor,
wherein the at least one JAK2 inhibitor is barcitinib,
wherein the autoimmune disease is mediated by plasmacytoid dendritic cells; and
wherein the autoimmune disease is systemic lupus erythematosus.

A still more preferred embodiment of the invention relates to a combination for use in the treatment of an autoimmune disease, wherein the combination consists of:
at least one inhibitor of antiporter system x_{c}⁻ and
at least one JAK2 inhibitor,
wherein the at least one JAK2 inhibitor is delgocitinib,
wherein the autoimmune disease is mediated by plasmacytoid dendritic cells; and
wherein the autoimmune disease is systemic lupus erythematosus.

A further embodiment of the invention is directed to a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and
   an inhibitor targeting STAT5,
wherein at least one inhibitor of the antiporter system x_{c}⁻ is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenylldiazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-I-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthylethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib, and
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine; KHK2898 antibody, IGN523 antibody; or
wherein the inhibitor targeting IL-3 receptor alpha is selected from the group comprising CSL362 antibody, flotetuzumab, IL3LDM, DT388IL3, and SGN-CD123A; or
wherein the inhibitor targeting IL-3 receptor beta is CSL311 antibody, or wherein the inhibitor targeting GM-CSF is otilimab, namilumab, lenzilumab, plonmarlimab, or gimsilumab; or
wherein the inhibitor targeting GM-CSF receptor alpha is mavrilimumab; or wherein the inhibitor targeting STAT5 is JPX1188, JPX0802, JPX1185, compound 17f, compound AC-4-130, CAS 285986-31-4, BP-1-075, BP-1-107, BP-1-108, SF-1-087, SF-1-088, IQDMA, or CAS 2062-78-4; and wherein the autoimmune disease is mediated by plasmacytoid dendritic cells, and
wherein the autoimmune disease is systemic lupus erythematosus.

An embodiment of the invention is a combination for use in the treatment of an autoimmune disease, wherein the combination consists of:
at least one inhibitor of antiporter system x_{c}⁻ and
at least one inhibitor selected from the group consisting of:
   a JAK2 inhibitor,
   a SLC7A5:SLC3A2 inhibitor,
   an inhibitor targeting IL-3 receptor alpha,
   an inhibitor targeting IL-3 receptor beta,
   an inhibitor targeting GM-CSF,
   an inhibitor targeting GM-CSF receptor alpha, and
   an inhibitor targeting STAT5,
wherein the at least one inhibitor of the antiporter system x_{c}⁻ is sulfasalazine and
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine; KHK2898 antibody, IGN523 antibody; or
wherein the inhibitor targeting IL-3 receptor alpha is selected from the group comprising CSL362 antibody, flotetuzumab, IL3LDM, DT388IL3, and SGN-CD123A; or
wherein the inhibitor targeting IL-3 receptor beta is CSL311 antibody, or wherein the inhibitor targeting GM-CSF is otilimab, namilumab, lenzilumab, plonmarlimab, or gimsilumab; or
wherein the inhibitor targeting GM-CSF receptor alpha is mavrilimumab; or wherein the inhibitor targeting STAT5 is JPX1188, JPX0802, JPX1185, compound 17f, compound AC-4-130, CAS 285986-31-4, BP-1-075, BP-1-107, BP-1-108, SF-1-087, SF-1-088, IQDMA, or CAS 2062-78-4; and wherein the autoimmune disease is mediated by plasmacytoid dendritic cells, and
wherein the autoimmune disease is systemic lupus erythematosus.

A preferred embodiment of the invention is directed to a combination for use in the treatment of an autoimmune disease, wherein the combination consists of:
at least one inhibitor of antiporter system x_{c}⁻ and
at least one JAK2 inhibitor,
wherein at least one inhibitor of the antiporter system x_{c}⁻ is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-I-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthylethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib, and
wherein the at least one JAK2 inhibitor is barcitinib,
wherein the autoimmune disease is mediated by plasmacytoid dendritic cells, and
wherein the autoimmune disease is systemic lupus erythematosus.

A more preferred embodiment of the invention is directed to a combination for use in the treatment of an autoimmune disease, wherein the combination consists of:
at least one inhibitor of antiporter system x_{c}⁻ and
at least one JAK2 inhibitor,
wherein at least one inhibitor of the antiporter system x_{c}⁻ is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenylldiazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-I-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthylethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib, and
wherein the at least one JAK2 inhibitor is delgocitinib,
wherein the autoimmune disease is mediated by plasmacytoid dendritic cells, and
wherein the autoimmune disease is systemic lupus erythematosus.

A more preferred embodiment of the invention is a combination for use in the treatment of an autoimmune disease, wherein the combination consists of:
at least one inhibitor of antiporter system x_{c}⁻ and
at least one JAK2 inhibitor,
wherein the at least one inhibitor of the antiporter system x_{c}⁻ is sulfasalazine and
wherein the at least one JAK2 inhibitor is barcitinib,
wherein the autoimmune disease is mediated by plasmacytoid dendritic cells, and
wherein the autoimmune disease is systemic lupus erythematosus.

A still more preferred embodiment of the invention is a combination for use in the treatment of an autoimmune disease, wherein the combination consists of:
at least one inhibitor of antiporter system x_{c}⁻ and
at least one JAK2 inhibitor,
wherein the at least one inhibitor of the antiporter system x_{c}⁻ is sulfasalazine and
wherein the at least one JAK2 inhibitor is delgocitinib,
wherein the autoimmune disease is mediated by plasmacytoid dendritic cells, and
wherein the autoimmune disease is systemic lupus erythematosus.

A more preferred embodiment of the invention relates to a combination for use in the treatment of an autoimmune disease, wherein the combination consists of:
at least one inhibitor of antiporter system x_{c}⁻ and
at least one inhibitor selected from the group consisting of:
   a JAK2 inhibitor, and
   a SLC7A5:SLC3A2 inhibitor,
wherein the autoimmune disease is mediated by plasmacytoid dendritic cells; and wherein the autoimmune disease is systemic lupus erythematosus.

A still more preferred embodiment of the invention relates to a combination for use in the treatment of an autoimmune disease, wherein the combination consists of:
at least one inhibitor of antiporter system x_{c}⁻ and
at least one inhibitor selected from the group consisting of:
   a JAK2 inhibitor, and
   a SLC7A5:SLC3A2 inhibitor,
wherein at least one inhibitor of the antiporter system x_{c}⁻ is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-l-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthyl-ethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib,
wherein the autoimmune disease is mediated by plasmacytoid dendritic cells; and
wherein the autoimmune disease is systemic lupus erythematosus.

An embodiment of the invention relates to a combination for use in the treatment of an autoimmune disease, wherein the combination consists of:
at least one inhibitor of antiporter system x_{c}⁻ and
at least one inhibitor selected from the group consisting of:
   a JAK2 inhibitor, and
   a SLC7A5:SLC3A2 inhibitor,
wherein the at least one inhibitor of the antiporter system x_{c}⁻ is sulfasalazine,
wherein the autoimmune disease is mediated by plasmacytoid dendritic cells; and
wherein the autoimmune disease is systemic lupus erythematosus.

A preferred embodiment of the invention relates to a combination for use in the treatment of an autoimmune disease, wherein the combination consists of:
at least one inhibitor of antiporter system x_{c}⁻ and
at least one inhibitor selected from the group consisting of:
   a JAK2 inhibitor, and
   a SLC7A5:SLC3A2 inhibitor,
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids- phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine; KHK2898 antibody, IGN523 antibody;
wherein the autoimmune disease is mediated by plasmacytoid dendritic cells; and
wherein the autoimmune disease is systemic lupus erythematosus.

### PHARMACEUTICAL COMPOSITIONS

The disclosure also provides pharmaceutical compositions comprising one or more of the disclosed inhibitors in association with a pharmaceutically acceptable carrier.

Preferably, the present invention relates to a pharmaceutical composition consisting of at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent.

More preferably, the present invention relates to a pharmaceutical composition consisting of at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent, and
wherein the at least one inhibitor of the antiporter system x_{c}⁻ is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-l-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthylethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib.

Still more preferably, the present invention relates to a pharmaceutical composition consisting of at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent, and
wherein the at least one inhibitor of the antiporter system x_{c}⁻ is sulfasalazine.

An embodiment of the present invention is a pharmaceutical composition consisting of at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent, and
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine, KHK2898 antibody, IGN523 antibody; or
wherein the inhibitor targeting IL-3 receptor alpha is selected from the group comprising CSL362 antibody, flotetuzumab, IL3LDM, DT388IL3, and SGN-CD123A; or
wherein the inhibitor targeting IL-3 receptor beta is CSL311 antibody, or wherein the inhibitor targeting GM-CSF is otilimab, namilumab, lenzilumab, plonmarlimab, or gimsilumab; or
wherein the inhibitor targeting GM-CSF receptor alpha is mavrilimumab; or wherein the inhibitor targeting STAT5 is JPX1188, JPX0802, JPX1185, compound 17f, compound AC-4-130, CAS 285986-31-4, BP-1-075, BP-1-107, BP-1-108, SF-1-087, SF-1-088, IQDMA, or CAS 2062-78-4.

A particular embodiment of the present invention is a pharmaceutical composition consisting of at least one inhibitor of antiporter system x_{c}⁻ and at least one JAK2 inhibitor, together with at least one pharmaceutically acceptable vehicle, excipient and/or diluent, and wherein the at least one JAK2 inhibitor is barcitinib.

A particular embodiment of the present invention is a pharmaceutical composition consisting of at least one inhibitor of antiporter system x_{c}⁻ and at least one JAK2 inhibitor, together with at least one pharmaceutically acceptable vehicle, excipient and/or diluent, and wherein the at least one JAK2 inhibitor is delgocitinib.

A more particular embodiment of the present invention is a pharmaceutical composition consisting of at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent, and
wherein the at least one inhibitor of the **antiporter system x_{c}⁻** is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenylldiazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-I-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthyl-ethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib, and
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine, KHK2898 antibody, IGN523 antibody; or
wherein the inhibitor targeting IL-3 receptor alpha is selected from the group comprising CSL362 antibody, flotetuzumab, IL3LDM, DT388IL3, and SGN-CD123A; or
wherein the inhibitor targeting IL-3 receptor beta is CSL311 antibody, or wherein the inhibitor targeting GM-CSF is otilimab, namilumab, lenzilumab, plonmarlimab, or gimsilumab; or
wherein the inhibitor targeting GM-CSF receptor alpha is mavrilimumab; or wherein the inhibitor targeting STAT5 is JPX1188, JPX0802, JPX1185, compound 17f, compound AC-4-130, CAS 285986-31-4, BP-1-075, BP-1-107, BP-1-108, SF-1-087, SF-1-088, IQDMA, or CAS 2062-78-4.

Another more preferred embodiment of the present invention is a pharmaceutical composition consisting of at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent;
wherein the at least one inhibitor of the antiporter system x_{c}⁻ is sulfasalazine, and
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid;
2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine, KHK2898 antibody, IGN523 antibody; or
wherein the inhibitor targeting IL-3 receptor alpha is selected from the group comprising CSL362 antibody, flotetuzumab, IL3LDM, DT388IL3, SGN-CD123A; or
wherein the inhibitor targeting IL-3 receptor beta is CSL311 antibody, or wherein the inhibitor targeting GM-CSF is otilimab, namilumab, lenzilumab, plonmarlimab, or gimsilumab; or
wherein the inhibitor targeting GM-CSF receptor alpha is mavrilimumab; or wherein the inhibitor targeting STAT5 is JPX1188, JPX0802, JPX1185, compound 17f, compound AC-4-130, CAS 285986-31-4, BP-1-075, BP-1-107, BP-1-108, SF-1-087, SF-1-088, IQDMA, or CAS 2062-78-4.

A further embodiment of the present invention is a a pharmaceutical composition consisting of at least one inhibitor of antiporter system x_{c}⁻ and at least one JAK2 inhibitor, together with at least one pharmaceutically acceptable vehicle, excipient and/or diluent, and
wherein the at least one inhibitor of the **antiporter system x_{c}⁻** is selected from the group comprising sulfasalazine, erastin, erastin analogs, , azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-l-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthyl-ethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib, and
wherein the at least one JAK2 inhibitor is barcitinib.

Another still more preferred embodiment of the present invention is a pharmaceutical composition consisting of at least one inhibitor of antiporter system x_{c}⁻ and at least one JAK2 inhibitor, together with at least one pharmaceutically acceptable vehicle, excipient and/or diluent,
wherein the at least one inhibitor of the antiporter system x_{c}⁻ is sulfasalazine,
and wherein the at least one JAK2 inhibitor is barcitinib.

A further embodiment of the present invention is a a pharmaceutical composition consisting of at least one inhibitor of antiporter system x_{c}⁻ and at least one JAK2 inhibitor, together with at least one pharmaceutically acceptable vehicle, excipient and/or diluent, and
wherein the at least one inhibitor of the **antiporter system x_{c}⁻** is selected from the group comprising sulfasalazine, erastin, erastin analogs, , azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-I-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthyl-ethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib, and
wherein the at least one JAK2 inhibitor is delgocitinib.

Another still more preferred embodiment of the present invention is a pharmaceutical composition consisting of at least one inhibitor of antiporter system x_{c}⁻ and at least one JAK2 inhibitor, together with at least one pharmaceutically acceptable vehicle, excipient and/or diluent,
wherein the at least one inhibitor of the antiporter system x_{c}⁻ is sulfasalazine,
and wherein the at least one JAK2 inhibitor is delgocitinib.

A more particular embodiment of the present invention is a pharmaceutical composition consisting of at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent, **for use** in the treatment of an autoimmune disease, wherein the autoimmune disease is mediated by plasmacytoid dendritic cells.

A still more particular embodiment of the present invention is a pharmaceutical composition consisting of at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent, **for use** in the treatment of an autoimmune disease, wherein the autoimmune disease is mediated by plasmacytoid dendritic cells, and wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-lgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

A further more particular embodiment of the present invention is a pharmaceutical composition consisting of at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent, **for use** in the treatment of an autoimmune disease, wherein the autoimmune disease is mediated by plasmacytoid dendritic cells, and wherein the autoimmune disease is systemic lupus erythematosus.

Another embodiment of the present invention is a pharmaceutical composition consisting of at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent, and wherein the at least one inhibitor of the **antiporter system x_{c}⁻** is selected from the group comprising sulfasalazine, erastin, erastin analogs, , azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-l-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthyl-ethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib, **for use** in the treatment of an autoimmune disease, wherein the autoimmune disease is mediated by plasmacytoid dendritic cells.

Another preferred embodiment of the present invention is a pharmaceutical composition consisting of at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent, and
wherein the at least one inhibitor of the **antiporter system x_{c}⁻** is selected from the group comprising sulfasalazine, erastin, erastin analogs, , azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-l-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthyl-ethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib;
**for use in the treatment of an autoimmune disease,** wherein the autoimmune disease is mediated by plasmacytoid dendritic cells, and wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-lgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

Another more preferred embodiment of the present invention is a pharmaceutical composition consisting of at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent, and
wherein the at least one inhibitor of the **antiporter system x_{c}⁻** is selected from the group comprising sulfasalazine, erastin, erastin analogs, , azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-l-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthyl-ethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib,
**for use in the treatment of an autoimmune disease,** wherein the autoimmune disease is mediated by plasmacytoid dendritic cells, and wherein the autoimmune disease is **systemic lupus erythematosus.**

A particular embodiment of the present invention is a pharmaceutical composition consisting of at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent,
wherein the at least one inhibitor of the antiporter system x_{c}⁻ is sulfasalazine,
**for use** in the treatment of an autoimmune disease, wherein the autoimmune disease is mediated by plasmacytoid dendritic cells.

A more particular embodiment of the present invention is a pharmaceutical composition consisting of at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent,
wherein the at least one inhibitor of the antiporter system x_{c}⁻ is sulfasalazine,
**for use** in the treatment of an autoimmune disease, wherein the autoimmune disease is mediated by plasmacytoid dendritic cells, and wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-lgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

A still more particular embodiment of the present invention is a pharmaceutical composition consisting of at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent,
wherein the at least one inhibitor of the antiporter system x_{c}⁻ is sulfasalazine,
**for use** in the treatment of an autoimmune disease, wherein the autoimmune disease is mediated by plasmacytoid dendritic cells, and wherein the autoimmune disease is systemic lupus erythematosus.

A further more particular embodiment of the present invention is a pharmaceutical composition consisting of at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent; and
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine, KHK2898 antibody, IGN523 antibody; or
wherein the inhibitor targeting IL-3 receptor alpha is selected from the group comprising CSL362 antibody, flotetuzumab, IL3LDM, DT388IL3, and SGN-CD123A; or
wherein the inhibitor targeting IL-3 receptor beta is CSL311 antibody, or wherein the inhibitor targeting GM-CSF is otilimab, namilumab, lenzilumab, plonmarlimab, or gimsilumab; or
wherein the inhibitor targeting GM-CSF receptor alpha is mavrilimumab; or wherein the inhibitor targeting STAT5 is JPX1188, JPX0802, JPX1185, compound 17f, compound AC-4-130, CAS 285986-31-4, BP-1-075, BP-1-107, BP-1-108, SF-1-087, SF-1-088, IQDMA, or CAS 2062-78-4;
**for use** in the treatment of an autoimmune disease, wherein the autoimmune disease is mediated by plasmacytoid dendritic cells.

A still more particular embodiment of the present invention is a pharmaceutical composition consisting of at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent; and
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine, KHK2898 antibody, IGN523 antibody; or
wherein the inhibitor targeting IL-3 receptor alpha is selected from the group comprising CSL362 antibody, flotetuzumab, IL3LDM, DT388IL3, and SGN-CD123A; or
wherein the inhibitor targeting IL-3 receptor beta is CSL311 antibody, or wherein the inhibitor targeting GM-CSF is otilimab, namilumab, lenzilumab, plonmarlimab, or gimsilumab; or
wherein the inhibitor targeting GM-CSF receptor alpha is mavrilimumab; or wherein the inhibitor targeting STAT5 is JPX1188, JPX0802, JPX1185, compound 17f, compound AC-4-130, CAS 285986-31-4, BP-1-075, BP-1-107, BP-1-108, SF-1-087, SF-1-088, IQDMA, or CAS 2062-78-4;
**for use** in the treatment of an autoimmune disease, wherein the autoimmune disease is mediated by plasmacytoid dendritic cells, and wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-lgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

Another still more particular embodiment of the present invention is a pharmaceutical composition consisting of at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent, and
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine, KHK2898 antibody, IGN523 antibody; or
wherein the inhibitor targeting IL-3 receptor alpha is selected from the group comprising CSL362 antibody, flotetuzumab, IL3LDM, DT388IL3, and SGN-CD123A; or
wherein the inhibitor targeting IL-3 receptor beta is CSL311 antibody, or wherein the inhibitor targeting GM-CSF is otilimab, namilumab, lenzilumab, plonmarlimab, or gimsilumab; or
wherein the inhibitor targeting GM-CSF receptor alpha is mavrilimumab; or wherein the inhibitor targeting STAT5 is JPX1188, JPX0802, JPX1185, compound 17f, compound AC-4-130, CAS 285986-31-4, BP-1-075, BP-1-107, BP-1-108, SF-1-087, SF-1-088, IQDMA, or CAS 2062-78-4,
**for use** in the treatment of an autoimmune disease, wherein the autoimmune disease is mediated by plasmacytoid dendritic cells, and wherein the autoimmune disease is systemic lupus erythematosus.

A further more particular embodiment of the present invention is a pharmaceutical composition consisting of at least one inhibitor of antiporter system x_{c}⁻ and at least one JAK2 inhibitor, together with at least one pharmaceutically acceptable vehicle, excipient and/or diluent; and
wherein the at least one JAK2 inhibitor is delgocitinib,
**for use** in the treatment of an autoimmune disease, wherein the autoimmune disease is mediated by plasmacytoid dendritic cells.

A still more particular embodiment of the present invention is a pharmaceutical composition consisting of at least one inhibitor of antiporter system x_{c}⁻ and at least one JAK2 inhibitor, together with at least one pharmaceutically acceptable vehicle, excipient and/or diluent; and
wherein the at least one JAK2 inhibitor is delgocitinib,
**for use** in the treatment of an autoimmune disease, wherein the autoimmune disease is mediated by plasmacytoid dendritic cells, and wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-lgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

Another still more particular embodiment of the present invention is a pharmaceutical composition consisting of at least one inhibitor of antiporter system x_{c}⁻ and at least one JAK2 inhibitor, together with at least one pharmaceutically acceptable vehicle, excipient and/or diluent; and
wherein the at least one JAK2 inhibitor is delgocitinib,
**for use** in the treatment of an autoimmune disease, wherein the autoimmune disease is mediated by plasmacytoid dendritic cells, and wherein the autoimmune disease is systemic lupus erythematosus.

A further more particular embodiment of the present invention is a pharmaceutical composition consisting of at least one inhibitor of antiporter system x_{c}⁻ and at least one JAK2 inhibitor, together with at least one pharmaceutically acceptable vehicle, excipient and/or diluent; and
wherein the at least one JAK2 inhibitor is baricitinib,
**for use** in the treatment of an autoimmune disease, wherein the autoimmune disease is mediated by plasmacytoid dendritic cells.

A still more particular embodiment of the present invention is a pharmaceutical composition consisting of at least one inhibitor of antiporter system x_{c}⁻ and at least one JAK2 inhibitor, together with at least one pharmaceutically acceptable vehicle, excipient and/or diluent; and
wherein the at least one JAK2 inhibitor is baricitinib,
**for use** in the treatment of an autoimmune disease, wherein the autoimmune disease is mediated by plasmacytoid dendritic cells, and wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-lgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

Another still more particular embodiment of the present invention is a pharmaceutical composition consisting of at least one inhibitor of antiporter system x_{c}⁻ and at least one JAK2 inhibitor, together with at least one pharmaceutically acceptable vehicle, excipient and/or diluent; and
wherein the at least one JAK2 inhibitor is baricitinib,
**for use** in the treatment of an autoimmune disease, wherein the autoimmune disease is mediated by plasmacytoid dendritic cells, and wherein the autoimmune disease is systemic lupus erythematosus.

A more particular embodiment of the present invention is a pharmaceutical composition consisting of at least one inhibitor of antiporter system x_{c}⁻ and at least one JAK2 inhibitor, together with at least one pharmaceutically acceptable vehicle, excipient and/or diluent,
wherein the at least one inhibitor of the antiporter system x_{c}⁻ is sulfasalazine, and wherein the at least one JAK2 inhibitor is barcitinib,
**for use** in the treatment of an autoimmune disease, wherein the autoimmune disease is mediated by plasmacytoid dendritic cells.

A more particular embodiment of the present invention is a pharmaceutical composition consisting of at least one inhibitor of antiporter system x_{c}⁻ and at least one JAK2 inhibitor, together with at least one pharmaceutically acceptable vehicle, excipient and/or diluent,
wherein the at least one inhibitor of the antiporter system x_{c}⁻ is sulfasalazine, and wherein the at least one JAK2 inhibitor is delgocitinib,
**for use** in the treatment of an autoimmune disease, wherein the autoimmune disease is mediated by plasmacytoid dendritic cells.

A further particular embodiment of the present invention is a pharmaceutical composition consisting of at least one inhibitor of antiporter system x_{c}⁻ and at least one JAK2 inhibitor, together with at least one pharmaceutically acceptable vehicle, excipient and/or diluent,
wherein the at least one inhibitor of the antiporter system x_{c}⁻ is sulfasalazine, and wherein the at least one JAK2 inhibitor is barcitinib,
**for use** in the treatment of an autoimmune disease, wherein the autoimmune disease is mediated by plasmacytoid dendritic cells, and wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-lgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

A further more particular embodiment of the present invention is a pharmaceutical composition consisting of at least one inhibitor of antiporter system x_{c}⁻ and at least one JAK2 inhibitor, together with at least one pharmaceutically acceptable vehicle, excipient and/or diluent,
wherein the at least one inhibitor of the antiporter system x_{c}⁻ is sulfasalazine, and wherein the at least one JAK2 inhibitor is delgocitinib,
**for use** in the treatment of an autoimmune disease, wherein the autoimmune disease is mediated by plasmacytoid dendritic cells, and wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-IgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

A particular embodiment of the present invention is a pharmaceutical composition consisting of at least one inhibitor of antiporter system x_{c}⁻ and at least one JAK2 inhibitor, together with at least one pharmaceutically acceptable vehicle, excipient and/or diluent,
wherein the at least one inhibitor of the antiporter system x_{c}⁻ is sulfasalazine, and wherein the at least one JAK2 inhibitor is barcitinib,
**for use** in the treatment of an autoimmune disease, wherein the autoimmune disease is mediated by plasmacytoid dendritic cells, and
wherein the autoimmune disease is mediated by plasmacytoid dendritic cells, and wherein the autoimmune disease is systemic lupus erythematosus.

A more particular embodiment of the present invention is a pharmaceutical composition consisting of at least one inhibitor of antiporter system x_{c}⁻ and at least one JAK2 inhibitor, together with at least one pharmaceutically acceptable vehicle, excipient and/or diluent,
wherein the at least one inhibitor of the antiporter system x_{c}⁻ is sulfasalazine, and wherein the at least one JAK2 inhibitor is delgocitinib,
**for use** in the treatment of an autoimmune disease, wherein the autoimmune disease is mediated by plasmacytoid dendritic cells, and
wherein the autoimmune disease is mediated by plasmacytoid dendritic cells, and wherein the autoimmune disease is systemic lupus erythematosus.

Preferably, the present invention relates to a pharmaceutical composition consisting of at least one inhibitor of antiporter system x_{c}⁻ and at least one inhibitor selected from the group consisting of a JAK2 inhibitor, and a SLC7A5:SLC3A2 inhibitor, together with at least one pharmaceutically acceptable vehicle, excipient and/or diluent.

More preferably, the present invention relates to a pharmaceutical composition consisting of at least one inhibitor of antiporter system x_{c}⁻ and at least one inhibitor selected from the group consisting of a JAK2 inhibitor, and a SLC7A5:SLC3A2 inhibitor, together with at least one pharmaceutically acceptable vehicle, excipient and/or diluent, and
wherein the at least one inhibitor of the antiporter system x_{c}⁻ is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-l-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthylethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib.

Still more preferably, the present invention relates to a pharmaceutical composition consisting of at least one inhibitor of antiporter system x_{c}⁻ and at least one inhibitor selected from the group consisting of a JAK2 inhibitor, and a SLC7A5:SLC3A2 inhibitor, and
wherein the at least one inhibitor of the antiporter system x_{c}⁻ is sulfasalazine.

An embodiment of the present invention is a pharmaceutical composition consisting of at least one inhibitor of antiporter system x_{c}⁻ and at least one inhibitor selected from the group consisting of a JAK2 inhibitor, and a SLC7A5:SLC3A2 inhibitor, together with at least one pharmaceutically acceptable vehicle, excipient and/or diluent, and
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine, KHK2898 antibody, IGN523 antibody.

A more particular embodiment of the present invention is a pharmaceutical composition consisting of at least one inhibitor of antiporter system x_{c}⁻ and at least one inhibitor selected from the group consisting of a JAK2 inhibitor, and a SLC7A5:SLC3A2 inhibitor, together with at least one pharmaceutically acceptable vehicle, excipient and/or diluent, and
wherein the at least one inhibitor of the **antiporter system x_{c}⁻** is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-I-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthyl-ethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib, and
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine, KHK2898 antibody, IGN523 antibody.

Another more preferred embodiment of the present invention is a pharmaceutical composition consisting of at least one inhibitor of antiporter system x_{c}⁻ and at least one inhibitor selected from the group consisting of a JAK2 inhibitor, and a SLC7A5:SLC3A2 inhibitor, together with at least one pharmaceutically acceptable vehicle, excipient and/or diluent;
wherein the at least one inhibitor of the antiporter system x_{c}⁻ is sulfasalazine, and
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine, KHK2898 antibody, IGN523 antibody.

A more particular embodiment of the present invention is a pharmaceutical composition consisting of at least one inhibitor of antiporter system x_{c}⁻ and at least one inhibitor selected from the group consisting of a JAK2 inhibitor, and a SLC7A5:SLC3A2 inhibitor, together with at least one pharmaceutically acceptable vehicle, excipient and/or diluent, **for use** in the treatment of an autoimmune disease, wherein the autoimmune disease is mediated by plasmacytoid dendritic cells.

A still more particular embodiment of the present invention is a pharmaceutical composition consisting of at least one inhibitor of antiporter system x_{c}⁻ and at least one inhibitor selected from the group consisting of a JAK2 inhibitor, and a SLC7A5:SLC3A2 inhibitor, together with at least one pharmaceutically acceptable vehicle, excipient and/or diluent, **for use** in the treatment of an autoimmune disease, wherein the autoimmune disease is mediated by plasmacytoid dendritic cells, and wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-IgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

A further more particular embodiment of the present invention is a pharmaceutical composition consisting of at least one inhibitor of antiporter system x_{c}⁻ and at least one inhibitor selected from the group consisting of a JAK2 inhibitor, and a SLC7A5:SLC3A2 inhibitor, together with at least one pharmaceutically acceptable vehicle, excipient and/or diluent, **for use** in the treatment of an autoimmune disease, wherein the autoimmune disease is mediated by plasmacytoid dendritic cells, and wherein the autoimmune disease is systemic lupus erythematosus.

Another embodiment of the present invention is a pharmaceutical composition consisting of at least one inhibitor of antiporter system x_{c}⁻ and at least one inhibitor selected from the group consisting of a JAK2 inhibitor, and a SLC7A5:SLC3A2 inhibitor, together with at least one pharmaceutically acceptable vehicle, excipient and/or diluent, and
wherein the at least one inhibitor of the **antiporter system x_{c}⁻** is selected from the group comprising sulfasalazine, erastin, erastin analogs, , azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-l-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthyl-ethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib,
**for use** in the treatment of an autoimmune disease, wherein the autoimmune disease is mediated by plasmacytoid dendritic cells.

Another preferred embodiment of the present invention is a pharmaceutical composition consisting of at least one inhibitor of antiporter system x_{c}⁻ and at least one inhibitor selected from the group consisting of a JAK2 inhibitor, and a SLC7A5:SLC3A2 inhibitor, together with at least one pharmaceutically acceptable vehicle, excipient and/or diluent, and
wherein the at least one inhibitor of the **antiporter system x_{c}⁻** is selected from the group comprising sulfasalazine, erastin, erastin analogs, , azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-l-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthyl-ethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib;
**for use in the treatment of an autoimmune disease,** wherein the autoimmune disease is mediated by plasmacytoid dendritic cells, and wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-IgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

Another more preferred embodiment of the present invention is a pharmaceutical composition consisting of at least one inhibitor of antiporter system x_{c}⁻ and at least one inhibitor selected from the group consisting of a JAK2 inhibitor, and a SLC7A5:SLC3A2 inhibitor, together with at least one pharmaceutically acceptable vehicle, excipient and/or diluent, and
wherein the at least one inhibitor of the **antiporter system x_{c}⁻** is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-l-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthyl-ethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib,
**for use in the treatment of an autoimmune disease,** wherein the autoimmune disease is mediated by plasmacytoid dendritic cells, and wherein the autoimmune disease is **systemic lupus erythematosus.**

A particular embodiment of the present invention is a pharmaceutical composition consisting of at least one inhibitor of antiporter system x_{c}⁻ and at least one inhibitor selected from the group consisting of a JAK2 inhibitor, and a SLC7A5:SLC3A2 inhibitor, together with at least one pharmaceutically acceptable vehicle, excipient and/or diluent,
wherein the at least one inhibitor of the antiporter system x_{c}⁻ is sulfasalazine,
**for use** in the treatment of an autoimmune disease, wherein the autoimmune disease is mediated by plasmacytoid dendritic cells.

A more particular embodiment of the present invention is a pharmaceutical composition consisting of at least one inhibitor of antiporter system x_{c}⁻ and at least one inhibitor selected from the group consisting of a JAK2 inhibitor, and a SLC7A5:SLC3A2 inhibitor, together with at least one pharmaceutically acceptable vehicle, excipient and/or diluent,
wherein the at least one inhibitor of the antiporter system x_{c}⁻ is sulfasalazine,
**for use** in the treatment of an autoimmune disease, wherein the autoimmune disease is mediated by plasmacytoid dendritic cells, and wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-IgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

A still more particular embodiment of the present invention is a pharmaceutical composition consisting of at least one inhibitor of antiporter system x_{c}⁻ and at least one inhibitor selected from the group consisting of a JAK2 inhibitor, and a SLC7A5:SLC3A2 inhibitor, together with at least one pharmaceutically acceptable vehicle, excipient and/or diluent,
wherein the at least one inhibitor of the antiporter system x_{c}⁻ is sulfasalazine,
**for use** in the treatment of an autoimmune disease, wherein the autoimmune disease is mediated by plasmacytoid dendritic cells, and wherein the autoimmune disease is systemic lupus erythematosus.

A further more particular embodiment of the present invention is a pharmaceutical composition consisting of at least one inhibitor of antiporter system x_{c}⁻ and at least one inhibitor selected from the group consisting of a JAK2 inhibitor, and a SLC7A5:SLC3A2 inhibitor, together with at least one pharmaceutically acceptable vehicle, excipient and/or diluent; and
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine, KHK2898 antibody, IGN523 antibody;
**for use** in the treatment of an autoimmune disease, wherein the autoimmune disease is mediated by plasmacytoid dendritic cells.

A still more particular embodiment of the present invention is a pharmaceutical composition consisting of at least one inhibitor of antiporter system x_{c}⁻ and at least one inhibitor selected from the group consisting of a JAK2 inhibitor, and a SLC7A5:SLC3A2 inhibitor, together with at least one pharmaceutically acceptable vehicle, excipient and/or diluent; and
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine, KHK2898 antibody, IGN523 antibody;
**for use** in the treatment of an autoimmune disease, wherein the autoimmune disease is mediated by plasmacytoid dendritic cells, and wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-IgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

Another still more particular embodiment of the present invention is a pharmaceutical composition consisting of at least one inhibitor of antiporter system x_{c}⁻ and at least one inhibitor selected from the group consisting of a JAK2 inhibitor, and a SLC7A5:SLC3A2 inhibitor, together with at least one pharmaceutically acceptable vehicle, excipient and/or diluent, and
wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids-phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine, KHK2898 antibody, IGN523 antibody;
**for use** in the treatment of an autoimmune disease, wherein the autoimmune disease is mediated by plasmacytoid dendritic cells, and wherein the autoimmune disease is systemic lupus erythematosus.

"**Pharmaceutical composition**" refers to a preparation in a form that allows the biological activity of the active ingredient(s) to be effective, and which contain no additional components which are toxic to the subjects to which the formulation is administered.

As used herein, the terms "**pharmaceutically acceptable**", "**physiologically tolerable**" and grammatical variations thereof, are used interchangeably and represent that the materials are capable of administration to or upon a mammal without the production of undesirable physiological effects such as nausea, dizziness, gastric upset and the like.

"**Pharmaceutically acceptable carrier**" refers to an ingredient in a pharmaceutical composition, other than an active ingredient, which is nontoxic to the subject to whom it is administered. A pharmaceutically acceptable carrier includes, but is not limited to, a buffer, excipient, stabilizer, or preservative.

The pharmaceutical composition is designed to facilitate the administering of the inventive polypeptides comprising the single domain antibodies in an effective manner.

"**Pharmaceutically acceptable vehicle**" refers to an ingredient in a pharmaceutical formulation, other than an active ingredient, which is nontoxic to the subject to whom it is administered. A pharmaceutically acceptable vehicle includes, but is not limited to, a buffer, stabilizer, or preservative.

Examples of suitable vehicles or excipients include, without limitation, lactose, dextrose, sucrose, glucose, powdered sugar, sorbitol, mannitol, xylitol, starches, acacia gum, xanthan gum, guar gum, tara gum, mesquite gum, fenugreek gum, locust bean gum, ghatti gum, tragacanth gum, inositol, molasses, maltodextrin, extract of Irish moss, panwar gum, mucilage of isapol husks, Veegum, larch arabogalactan, calcium silicate, calcium phosphate, dicalcium phosphate, calcium sulfate, kaolin, sodium chloride, polyethylene glycol, alginates, gelatine, microcrystalline cellulose, polyvinylpyrrolidone, cellulose, water, saline, syrup, methylcellulose, ethylcellulose, hydroxypropylnethylcellulose, carboxymethylcellulose, polyacrylic acids such as Carbopols, such as Carbopol941, Carbopol980, Carbopol981,and gum bases such as Pharmagum^{™} (SPI Pharma Group; New Castle, Del.), and similar. Typically, the compositions of the present invention comprise from about 10% to about 90% by weight of the vehicle, the excipient or combinations thereof.

The pharmaceutical composition can be formulated into powders, granules, tablets, capsules, suspensions, emulsions, syrups, oral dosage form, external preparation, suppository or in the form of sterile injectable solutions, such as aerosolized in a usual manner, respectively. When formulated, it can be prepared using a **diluent** or **excipient** such as generally used fillers, extenders, binders, wetting agents, disintegrating agents, surface active agents.

In the pharmaceutical composition, the solid preparation for oral administration may be a tablet, pill, powder, granule, or capsule. The solid preparation may further comprise an excipient. Excipients may be, for example, starch, calcium carbonate, sucrose, lactose, or gelatine. In addition, the solid preparation may further comprise a lubricant, such as magnesium stearate, or talc. In the pharmaceutical composition, liquid preparations for oral administration may be best suspensions, solutions, emulsions, or syrups. The liquid formulation may comprise water, or liquid paraffin. The liquid formulation may, for excipients, for example, include wetting agents, sweeteners, aromatics or preservatives. For the purposes of parenteral administration, compositions containing the polypeptides of the invention are preferably dissolved in distilled water and the pH preferably adjusted to about 6 to 8.

Useful preparations of the compositions of the invention for parenteral administration also include sterile aqueous and non-aqueous solvents, suspensions and emulsions. Examples of useful non-aqueous solvents include propylene glycol, polyethylene glycol, vegetable oil, fish oil, and injectable organic esters.

For preparing solid compositions such as tablets, the principal active ingredient is mixed with a pharmaceutically acceptable carrier, e.g. conventional tableting ingredients such as corn starch, lactose, sucrose, sorbitol, talc, stearic acid, magnesium stearate, dicalcium phosphate or gums, and other pharmaceutical diluents, e.g. water, to for a solid preformulation composition containing a homogeneous mixture for a compound of the present invention, or a pharmaceutically acceptable salt thereof. When referring to these preformulation compositions as homogeneous, it is mean that the active ingredient is dispersed evenly throughout the composition so that the composition may be easily subdivided into equally effective unit dosage forms such as tablets, pills and capsules. This solid pre-formulation composition is then subdivided into unit dosage forms of the type described above containing from 0.1 to about 500 mg of the active ingredient of the present invention. Typical unit dosage forms contain from 1 to 100 mg, for example, 1, 2, 5, 0, 25, 5 or 00 mg, of the active ingredient. The tablets or pills can be coated o otherwise compounded to provide a dosage affording the advantage of prolonged action. For example, the tablet or pill can comprise an inner dosage and an outer dosage component, the latter being in the form of an envelope over the former. The two components can be separated by an enteric layer which, serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, acetyl alcohol and cellulose acetate.

The liquid forms in which the composition of the present invention may be incorporated for administration orally or by injection include aqueous solutions, suitably flavoured syrups, aqueous or oi suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium caboxymethylcellulose, luethylcellulose, polyvinylpyrrolidone or gelatin.

### USES of the COMBINATIONS

Also described herein is a **method for the treatment** of an autoimmune disease, comprising administering to a patient a combination consisting of a therapeutically effective amount of at least one inhibitor of antiporter system x_{c}⁻, and a therapeutically effective amount of at least one inhibitor selected from the group consisting of a JAK2 inhibitor, and a SLC7A5:SLC3A2 inhibitor, or a pharmaceutical composition comprising said combination, wherein the autoimmune disease is mediated by plasmacytoid dendritic cells.

In particular, it is described a **method for the treatment** of an autoimmune disease, comprising administering to a patient a combination consisting of a therapeutically effective amount of at least one inhibitor of antiporter system x_{c}⁻, and a therapeutically effective amount of at least one inhibitor selected from the group consisting of a JAK2 inhibitor, and a SLC7A5:SLC3A2 inhibitor, or a pharmaceutical composition comprising said combination, wherein the autoimmune disease is mediated by plasmacytoid dendritic cells, and wherein the at least one inhibitor of the antiporter system x_{c}⁻ is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-l-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthylethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib.

Also described herein is a **method for the treatment** of an autoimmune disease, comprising administering to a patient a combination consisting of a therapeutically effective amount of at least one inhibitor of antiporter system x_{c}⁻, and a therapeutically effective amount of at least one inhibitor selected from the group consisting of a JAK2 inhibitor, and a SLC7A5:SLC3A2 inhibitor, or a pharmaceutical composition comprising said combination, wherein the autoimmune disease is mediated by plasmacytoid dendritic cells, and wherein the at least one inhibitor of the antiporter system x_{c}⁻ is sulfasalazine.

Also described is a **method for the treatment** of an autoimmune disease, comprising administering to a patient a combination consisting of a therapeutically effective amount of at least one inhibitor of antiporter system x_{c}⁻, and a therapeutically effective amount of at least one inhibitor selected from the group consisting of a JAK2 inhibitor, and a SLC7A5:SLC3A2 inhibitor, or a pharmaceutical composition comprising said combination, wherein the autoimmune disease is mediated by plasmacytoid dendritic cells, and wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)-2-Amino-3-(3-((2,4-dicyano-3-(4-(2-(methylamino)-2-oxoethoxy)phenyl) benzo[4,5]imidazo[1,2-a]pyridin-1-yl)- carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids- phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine; KHK2898 antibody, IGN523 antibody.

Also described herein is a **method for the treatment** of an autoimmune disease, comprising administering to a patient a combination consisting of a therapeutically effective amount of at least one inhibitor of antiporter system x_{c}⁻, and a therapeutically effective amount of at least one JAK2 inhibitor, or a pharmaceutical composition comprising said combination, wherein the autoimmune disease is mediated by plasmacytoid dendritic cells, and wherein the at least one JAK2 inhibitor is barcitinib.

Also described herein is a **method for the treatment** of an autoimmune disease, comprising administering to a patient a combination consisting of a therapeutically effective amount of at least one inhibitor of antiporter system x_{c}⁻, and a therapeutically effective amount of at least one JAK2 inhibitor, or a pharmaceutical composition comprising said combination, wherein the autoimmune disease is mediated by plasmacytoid dendritic cells, and wherein the at least one JAK2 inhibitor is delgocitinib.

Also described herein is a **method for the treatment** of an autoimmune disease, comprising administering to a patient a combination consisting of a therapeutically effective amount of at least one inhibitor of antiporter system x_{c}⁻, and a therapeutically effective amount of at least one inhibitor selected from the group consisting of a JAK2 inhibitor, and a SLC7A5:SLC3A2 inhibitor, or a pharmaceutical composition comprising said combination, wherein the autoimmune disease is mediated by plasmacytoid dendritic cells, and wherein the autoimmune disease is selected from the group comprising lupus; cutaneous lupus; systemic lupus erythematosus (SLE); neonatal lupus; drug-induced lupus; SLE-induced conditions ; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-IgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

Also described herein is a **method for the treatment** of an autoimmune disease, comprising administering to a patient a combination consisting of a therapeutically effective amount of at least one inhibitor of antiporter system x_{c}⁻, and a therapeutically effective amount of at least one inhibitor selected from the group consisting of a JAK2 inhibitor, and a SLC7A5:SLC3A2 inhibitor, or a pharmaceutical composition comprising said combination, wherein the autoimmune disease is mediated by plasmacytoid dendritic cells, and wherein the autoimmune disease is systemic lupus erythematosus.

Also described herein is a **method for the treatment** of an autoimmune disease, comprising administering to a patient a combination consisting of a therapeutically effective amount of at least one inhibitor of antiporter system x_{c}⁻, and a therapeutically effective amount of at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, or a pharmaceutical composition comprising said combination, wherein the autoimmune disease is mediated by plasmacytoid dendritic cells.

In particular, it is described a **method for the treatment** of an autoimmune disease, comprising administering to a patient a combination consisting of a therapeutically effective amount of at least one inhibitor of antiporter system x_{c}⁻, and a therapeutically effective amount of at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, or a pharmaceutical composition comprising said combination, wherein the autoimmune disease is mediated by plasmacytoid dendritic cells, and wherein the at least one inhibitor of the antiporter system x_{c}⁻ is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methyiamino-L-alanine, L-serine-O-sulphate, L-α-aminopimelate, L-homocysteate, S-sulpho-I-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthyl-ethyl-ACPA, bis-trifluoromethyl-phenyl-isoxazole-4-hydrazone, 5-naphthylethyl isoxazole-4-(2,4-dinitrophenol)hydrazone-dinitrophenol, sorafenib.

Also described herein is a **method for the treatment** of an autoimmune disease, comprising administering to a patient a combination consisting of a therapeutically effective amount of at least one inhibitor of antiporter system x_{c}⁻, and a therapeutically effective amount of at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, or a pharmaceutical composition comprising said combination, wherein the autoimmune disease is mediated by plasmacytoid dendritic cells, and wherein the at least one inhibitor of the antiporter system x_{c}⁻ is sulfasalazine.

Also described is a **method for the treatment** of an autoimmune disease, comprising administering to a patient a combination consisting of a therapeutically effective amount of at least one inhibitor of antiporter system x_{c}⁻, and a therapeutically effective amount of at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, or a pharmaceutical composition comprising said combination, wherein the autoimmune disease is mediated by plasmacytoid dendritic cells, and wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)-2-Amino-3-(3-((2,4-dicyano-3-(4-(2-(methylamino)-2-oxoethoxy)phenyl)
benzo[4,5]imidazo[1,2-a]pyridin-1-yl)- carbamoyl)phenyl)propanoic acid 1; 2-amino-3-(3-benzylphenyl)propanoic acid; 2-amino-3-(3-benzyl-4-hydroxyphenyl)propanoic acid; hydroxamic acids- phenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine; KHK2898 antibody, IGN523 antibody; or
wherein the inhibitor targeting IL-3 receptor alpha is selected from the group comprising CSL362 antibody, flotetuzumab, IL3LDM, DT388IL3, and SGN-CD123A; or
wherein the inhibitor targeting IL-3 receptor beta is CSL311 antibody, or wherein the inhibitor targeting GM-CSF is otilimab, namilumab, lenzilumab, plonmarlimab, or gimsilumab; or
wherein the inhibitor targeting GM-CSF receptor alpha is mavrilimumab; or wherein the inhibitor targeting STAT5 is JPX1188, JPX0802, JPX1185, compound 17f, compound AC-4-130, CAS 285986-31-4, BP-1-075, BP-1-107, BP-1-108, SF-1-087, SF-1-088, IQDMA, or CAS 2062-78-4.

Also described herein is a **method for the treatment** of an autoimmune disease, comprising administering to a patient a combination consisting of a therapeutically effective amount of at least one inhibitor of antiporter system x_{c}⁻, and a therapeutically effective amount of at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, or a pharmaceutical composition comprising said combination, wherein the autoimmune disease is mediated by plasmacytoid dendritic cells, and wherein the autoimmune disease is selected from the group comprising lupus; cutaneous lupus; systemic lupus erythematosus (SLE); neonatal lupus; drug-induced lupus; SLE-induced conditions ; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-IgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

Also described herein is a **method for the treatment** of an autoimmune disease, comprising administering to a patient a combination consisting of a therapeutically effective amount of at least one inhibitor of antiporter system x_{c}⁻, and a therapeutically effective amount of at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, or a pharmaceutical composition comprising said combination, wherein the autoimmune disease is mediated by plasmacytoid dendritic cells, and wherein the autoimmune disease is systemic lupus erythematosus.

As used herein, the term **"administering"** refers to bringing a subject, tissue, organ or cells in contact with a therapeutically effective amount of a combination consisting of at least one inhibitor of antiporter system x_{c}⁻, and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, or of a pharmaceutical composition comprising said combination, as described in this disclosure. In certain embodiments, the present invention encompasses administering a therapeutically effective amount of a combination consisting of at least one inhibitor of antiporter system x_{c}⁻, and at least one inhibitor selected from the group consisting of a JAK2 inhibitor, and a SLC7A5:SLC3A2 inhibitor, or of a pharmaceutical composition comprising said combination, as described in this disclosure to a patient or subject.

"**Treatment**", **"treat", "treating", "therapy"** and/or **"therapy regimen"** refers to clinical intervention in an attempt to alter the natural course of a disease or disorder in the individual being treated, and can be performed either for prophylaxis or during the course of clinical pathology. Desired results of treatment can include, but are not limited to, preventing occurrence or recurrence of the disorder, alleviation of symptoms, diminishment of any direct or indirect pathological consequences of the disorder, slowing or stopping the progression or worsening of a disease, disorder, or condition and/or the remission of the disease, disorder or condition, decreasing the rate of progression, amelioration or palliation of a disease state, and remission or improved prognosis. For example, treatment can include administration of a therapeutically effective amount of at least one inhibitor of antiporter system x_{c}⁻, and a therapeutically effective amount of at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5,or a pharmaceutical composition comprising said combination, to a subject to delay development or slow progression of an autoimmune disease, wherein the autoimmune disease is mediated by plasmacytoid dendritic cells.

**"Concomitant administration," "concurrent administration,"** or "co**administration"** as used herein includes administration of the active agents (e.g., monoclonal antibodies, small molecules, biomolecules), in conjunction or combination, together, or before or after each other. The multiple agent(s) may be administered by the same or by different routes, simultaneously or sequentially, as long as they are given in a manner sufficient to allow all agents to achieve effective concentrations at the site of action. A person of ordinary skill in the art would have no difficulty determining the appropriate timing, sequence, and dosages of administration for particular drugs and compositions of the present invention.

The term **"effective amount"** or **"therapeutically effective amount"** refers to the amount of an agent (or a pharmaceutical formulation) that is sufficient to achieve a desired therapeutic or prophylactic result, e.g., to treat or prevent a disease or disorder in a subject. The therapeutically effective amount will vary depending upon the subject and disease condition being treated, the weight and age of the subject, the severity of the disease condition, the manner of administration and the like, which can readily be determined by one of ordinary skill in the art.

The specific **"effective amount"** will vary depending on the particular agent chosen, the dosing regimen to be followed, whether it is administered in combination with other compounds, timing of administration, and the physical delivery system in which it is carried. The specific **"effective amount"** will also vary with such factors as the particular condition being treated, the physical condition of the patient, the duration of the treatment, the nature of concurrent therapy (if any), and the specific formulations employed. The optimum effective amounts can be readily determined by one of ordinary skill in the art using routine experimentation.

As used herein, the term **"subject"** and **"patient"** are used interchangeably herein and refer to both human and nonhuman animals. The term "nonhuman animals" of the disclosure includes all vertebrates, e.g., mammals and non-mammals, such as nonhuman primates, sheep, dog, cat, horse, cow, chickens, amphibians, reptiles, and the like. Preferably, the subject is a human patient that is at for, or suffering from, an autoimmune disease.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the scope of the invention as described in the following claims.

### Description of the Figures

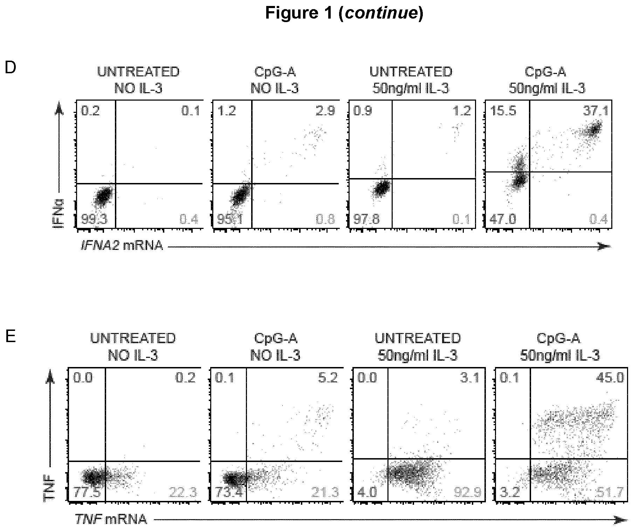

- Figure 1: shows IL-3 priming of activated pDC for cytokine production. Isolated pDC maintained +/- IL-3 were probed for: (**A**) IFNα and TNFα production with IL-3 and CpG-A (for 5h) stimulation [n=15 in 11 experiments (various time points)]; (**B**) live cells, where indicated cells were deprived from IL-3 for indicated time [*n=*4 in 2 experiments]; (**C**) IFNα and TNFα production [IFNα: n=8 in 5 experiments and TNFα: n=7 in 4 experiments]; (**D**) IFNA2 mRNA expression and IFNα production, n=3 in 3 experiments; (**E**) TNFα mRNA expression and TNFα production, n=3 in 3 experiments. Bar graphs are shown as mean +/- SD. Dots represent individual donors (n).
- Figure 2: shows IL-3 priming of activated pDC for mTORC1 activation. (**A**) Isolated pDCs maintained +/- IL-3 and indicated inhibitors o/n, then stimulated +/- CpG-A (for 5h) and probed for pS6 (S235/236) expression [*n*=7 in 4 experiments]; (**B**) Isolated pDC maintained in IL-3 were probed for pS6 (S235/236) expression, where indicated cells were treated with rapamicin (RAP) inhibitor for indicated time [n=5 in 4 experiments]; Isolated pDCs maintained +/- IL-3 and indicated inhibitors o/n, then stimulated +/- CpG-A (for 5h) and probed for (**C**) IFNα (*n*=4 in 2 experiments) and (**D**) TNFα production (n=7 in 4 experiments). Bar graphs are shown as mean +/- SD. Dots represent individual donors (*n*).

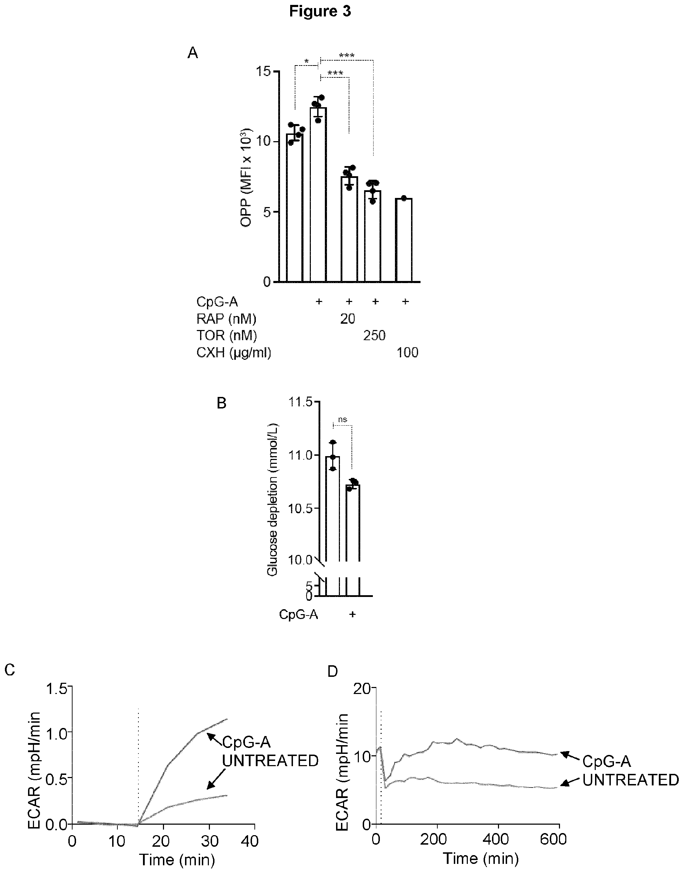

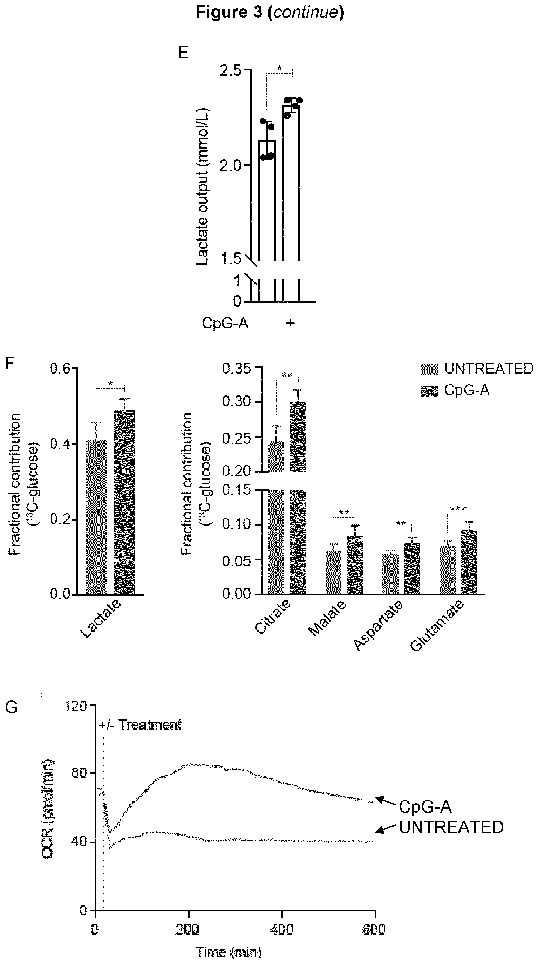

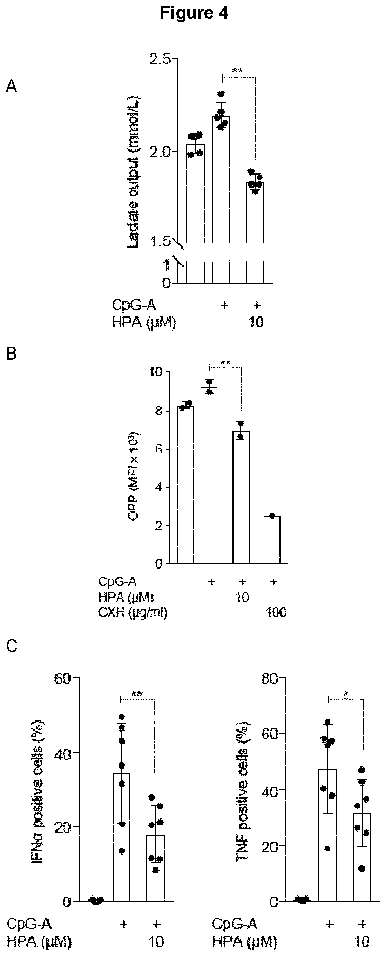

- Figure 3: shows that pDC activation is coupled to mTORC1 activity and changes in metabolism. Isolated pDC maintained in IL-3 were probed for: (**A**) protein synthesis measured by incorporation of puromycin analogue (OPP) in cells stimulated +/- CpG-A for 10 h and indicated inhibitors, *n*=4 in 1 experiment; (**B**) glucose (n=3 in 1 experiment) and (**E**) lactate (*n*=3 in 1 experiment) levels in medium from cells stimulated +/- CpG-A for 10 h, in (**E**) data shared with Figure 4A; (**C**) ECAR before and after addition of glucose (dashed line) in cells stimulated +/- CpG-A for 5h after o/n rest, *n*=3 in 2 experiments; (**D**) ECAR baseline levels measured by mitochondrial stress test in cells stimulated +/- CpG-A for 10h (dashed line), *n*=7 in 6 experiments; (**F**) indicated metabolites shown as fractional contribution of newly synthesized 13C-glucose carbon-incorporation after 3 h stimulation +/- CpG-A [n=5 in 2 experiments]; (**G**) OCR baseline levels using mitochondrial stress test in cells stimulated +/- CpG-A, *n*=7 in 6 experiments; Bar graphs are shown as mean +/-SD. Dots represent individual donors (n).

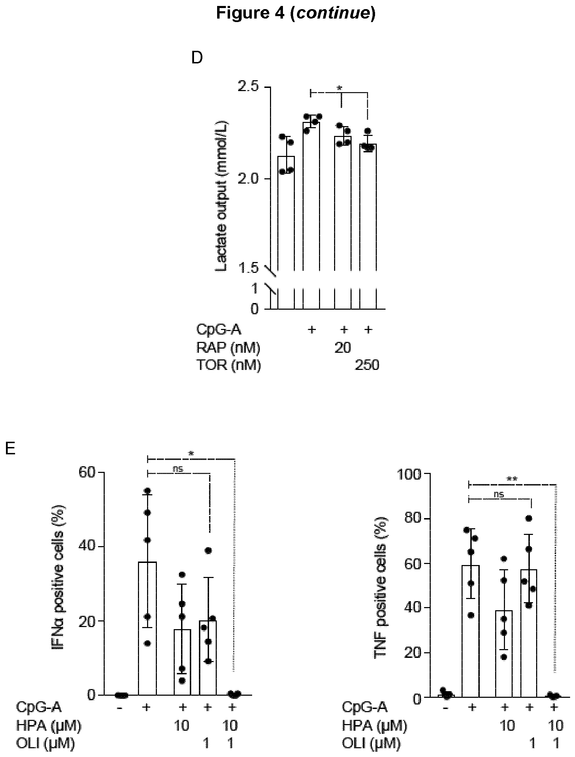

- Figure 4: shows that pDC activation is coupled to mTORC1 activity and changes in metabolism. Isolated pDC maintained in IL-3 were probed for: (A, D) lactate levels in medium from cells stimulated +/- CpG-A and +/-indicated inhibitors for 10 h, (**A**) *n*=5 in 2 experiments, (**D**) *n*=4 in 1 experiment; (**B**) protein synthesis measured by incorporation of puromycin analogue (OPP) in cells stimulated +/- CpG-A for 10 h and indicated inhibitors, *n*=5 in 2 experiments, one experiment shown; (**C**) IFNα (*n*=4 in 2 experiments) and TNFα production (*n*=7 in 4 experiments), where indicated cells were pre-treated with inhibitor o/n and then stimulated +/- CpG-A for 5h; (**E**) IFNα and TNFα production in cells stimulated +/- CpG-A and indicated inhibitors for 5h after o/n rest, *n*=5 in 2 experiments. Bar graphs are shown as mean +/- SD. Dots represent individual donors (n).

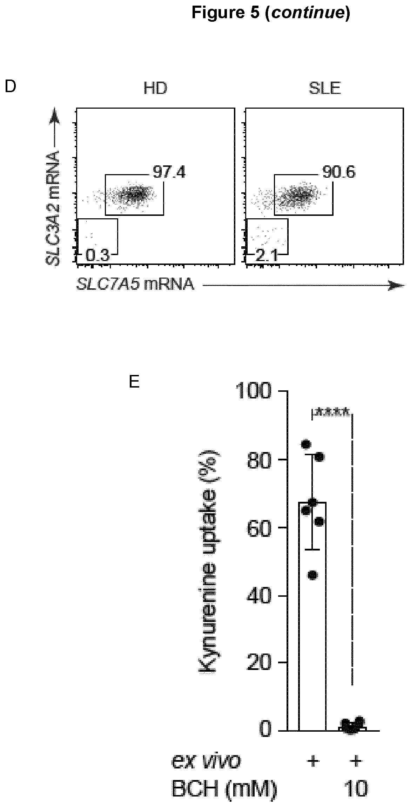

- Figure 5: shows that IL-3 induces the expression of functional System LAA transporters that license mTORC1 activation. (**A**) isolated pDC maintained in IL-3 o/n, then stimulated +/- CpG-A for 5h were subjected for scRNA-seq analysis presented as UMAP plot coloured by clusters and violin plots of *SLC7A5* and *SLC7A6* expression across clusters (*n*=1 in 1 experiment); (**B**) *SLC7A5* and *SLC3A2* mRNA expression (*n*=7 in 4 experiments); (C) kynurenine uptake (*n*=13-18 in 9 experiments); Bar graphs are shown in (B), (C) as geometric mean +/-geometric SD, (**D**) *ex vivo* isolated pDC from healthy donor (HD) and SLE patients (SLE) were probed for *SLC7A5* and *SLC7A6* expression (n=6 in 1 experiment); (**E**) *ex vivo* isolated pDC from healthy donor were probed for kynurenine uptake (n=6 in 2 experiments); in (D) and (E) representative plots from individual donors (n) are shown. Bar graphs are shown as mean +/- SD. Dots represent individual donors (n).

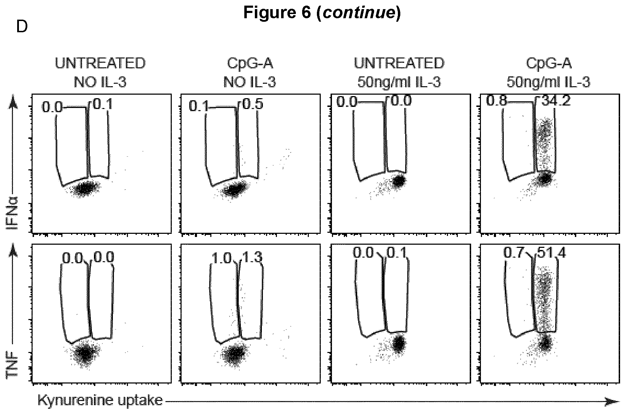

- Figure 6: shows IL-3 induces the expression of functional System LAA transporters that license mTORC1 activation. Isolated pDC maintained in IL-3 and indicated inhibitor o/n and then stimulated with CpG-A for 5h were probed for: (**A**) kynurenine uptake and pS6 (S235/236) expression (n=7-9 in 5-6 experiments); (**B**) kynurenine uptake and pS6 (Ser235/236) expression (n=3 in 3 experiments). (**C**) SLC7A5 mRNA expression and IFNα or TNFα production (IFNα: n=6 in 4 experiments and TNFα: n=3 in 2 experiments); (**D**) kynurenine uptake and IFNα or TNFα production (IFNα: n=5-7 and TNFα: n= 6-7 in 5 experiments). In (**A**) - (**D**) representative plots from individual donors (n) are shown;
- Figure 7: shows that inhibition of JAK2 prevents mTORC1 activation and cytokine production. (**A**) - (**G**): Isolated pDC maintained +/- IL-3 and indicated inhibitors o/n, then stimulated +/- CpG-A for 5 h were probed for: (**A**) pSTAT5 (Y694) expression (*n*=3-6 in 2-3 experiments); (**B**) *SLC7A5* and *SLC3A2* mRNA expression (*n*=3-6 in 2 experiments); (**C**) kynurenine uptake (*n*=5-13 donors in 3-6 experiments); (**D**) pS6 (S235/236) expression (*n*=5-6 in 4 experiments); (**E**) kynurenine uptake and IFNα (*n*=3 in 2 experiments) or TNFα positive cells (*n*=2-3 in 1-2 experiment); (**F**) kynurenine uptake and pS6 (S235/236) expression (n=5 in 3 experiments); (B - G) CpG-A and 50ng/ml IL-3 condition shared with Figures 5B, 5C, 6A, 6D; (**G**) IFNα positive cells (*n*=4-5 in 2-3 experiments). (A - C, E, F) Representative plots from individual donors (*n*) are shown. Bar graphs are shown as (A - C) geometric mean +/- geometric SD, (D, E) mean +/- SD and (F) mean. Dots represent individual donors (*n*).
- Figure 8: shows that inhibition of JAK2 prevents mTORC1 activation and cytokine production. Isolated pDC maintained +/- GM-CSF and indicated inhibitors o/n, then stimulated +/- CpG-A for 5h and probed for: (**H**) kynurenine uptake and pS6 (S235/236) expression; (**I**) IFNα production. (H, I) *n*=4 in 2 experiments. (H) Representative plots from individual donors (n) are shown. Bar graphs are shown in (I) mean +/- SD. Dots represent individual donors.

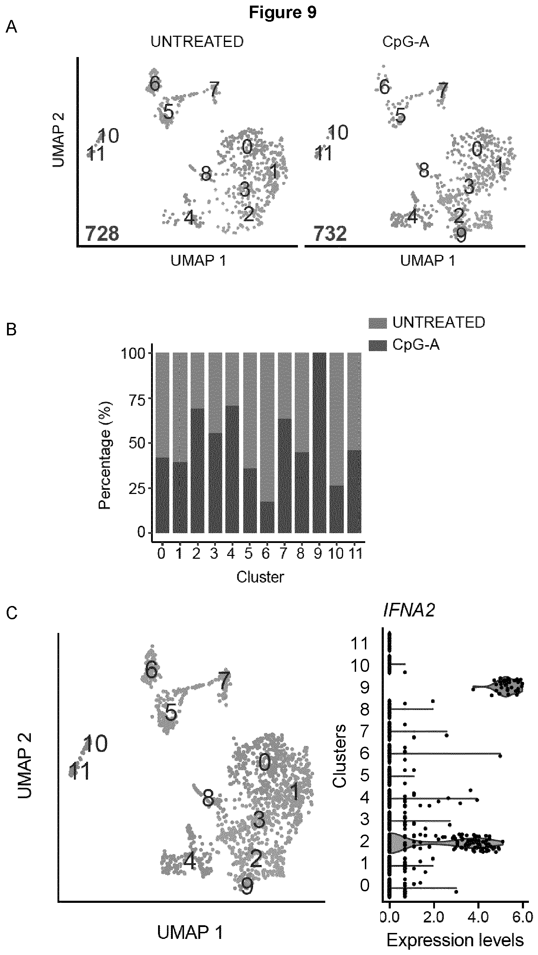

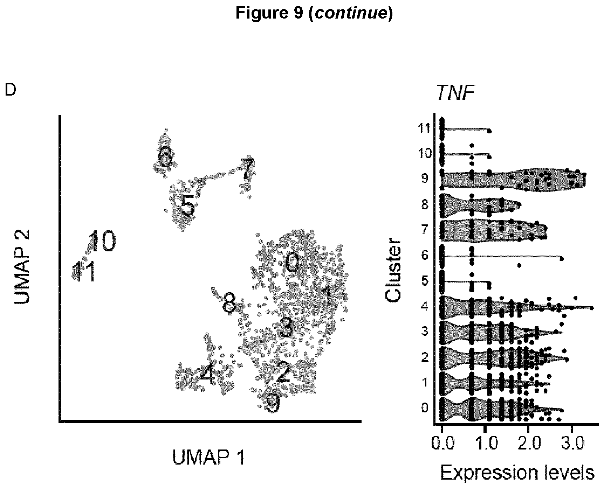

- Figure 9: shows changes in activated vs untreated pDC. (A - D) isolated pDC maintained in IL-3 o/n, then stimulated +/- CpG-A for 5h (*in vitro* pDC) were subjected to scRNA-seq analysis presented as: (**A**) UMAP plot colored by clusters and separated by condition (numbers in red indicate cells that were sequenced); (**B**) bar graph of changes in cell frequencies across clusters after CpG-A stimulation; (**C**) UMAP plot coloured by clusters and violin plot of *IFNA2;* (**D**) UMAP plot coloured by clusters and violin plot of TNFα expression across clusters.

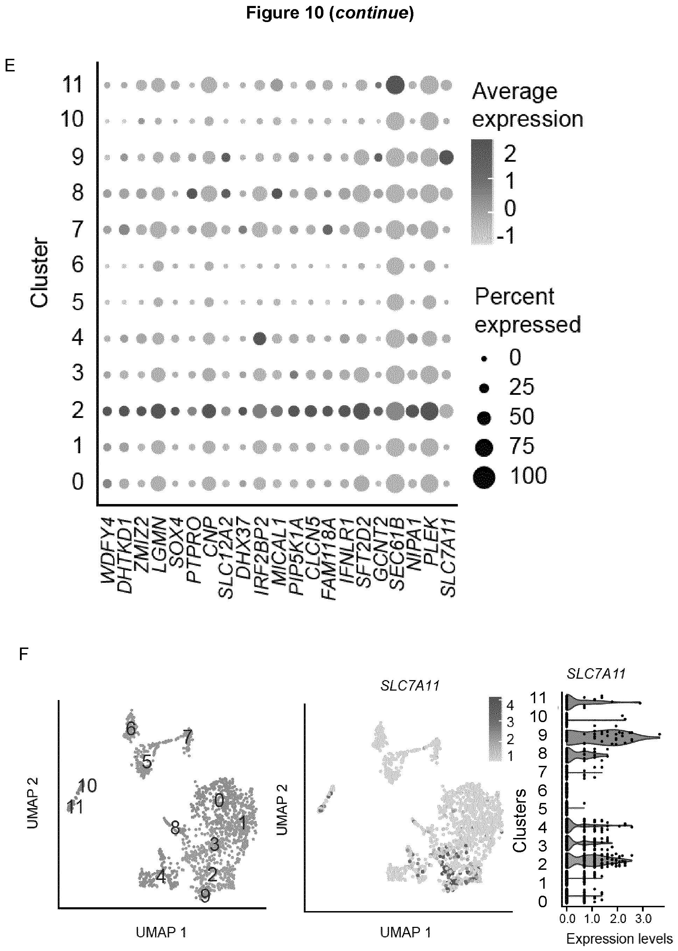

- Figure 10: shows in **(A, B, C, H, I)** re-analysed scRNA-seq data of pDC (SLE pDC) and other immune cells from kidney biopsies of lupus nephritis patients (*in vivo* SLE pDC) (Arazi *et al.,* 2019) presented as UMAP plot colored by clusters and violin plots of (**B, C**) *IL3RA, CSF2RA, CSF2RB, SLC7A5, SLC3A2* and (**H**) *SLC7A11* expression across clusters; (**D, E**) comparative analysis of differentially expressed genes from *in vitro* pDC and *in vivo* SLE pDC (C22) presented as (**D**) UpSet plot with the intersection of differentially expressed genes between pDC and SLE pDC; (**E**) dot plot with the relative expression and percent expression of genes (x axis) across clusters in pDC (y axis); (**F**) isolated pDC maintained in IL-3 o/n, then stimulated +/- CpG-A for 5h (*in vitro* pDC) were subjected for scRNA-seq analysis presented as UMAP plot colored by clusters and violin plot of *SLC7A11* expression across clusters; (**G, I**) bar graph with indicated frequencies of SLC7A11 expression within cluster of interest in (**H**) C2 and C9 *in vitro* pDC and (**I**) C22 *in vivo* SLE pDC; (C-E) n=1 in 1 experiment.
- Figure 11: shows (**A**) expression of *SLC7A11,* detected with AF488 conjugated antibodies, in skin from healthy donors (HD) and skin from cutaneous lupus patients; (**B**) expression of SLC7A11, detected as in (A), and IL-3Ra (pink) on serial sections of skin from cutaneous lupus patients; scale bar 100µm, images representative of *n=4* in 1 experiment, where n denotes individual donors. (**C**) *ex vivo* isolated pDC from healthy donor (HD) and SLE patients (SLE) were probed for *SLC7A11* and SLC3A2 expression (n=6 in 1 experiment, representative plot is shown).
- Figure 12: shows isolated pDC maintained +/- IL-3 and indicated inhibitors o/n, then stimulated +/- CpG-A for 5h and probed for: (**A**) *SLC7A11* and *SLC3A2* mRNA expression; (**B**) *SLC7A11* and *SLC7A5* mRNA expression; (**C**) *SLC7A11* mRNA expression; (**A - C**) n=5-6 in 3 experiments; (**D**) IFNα production, *n*=3-4 in 2. (A, C) Representative plots from individual donors (*n*) are shown. Bar graphs are shown as (B) geometric mean +/- geometric SD, (D) mean +/- SD. Dots represent individual donors (*n*).

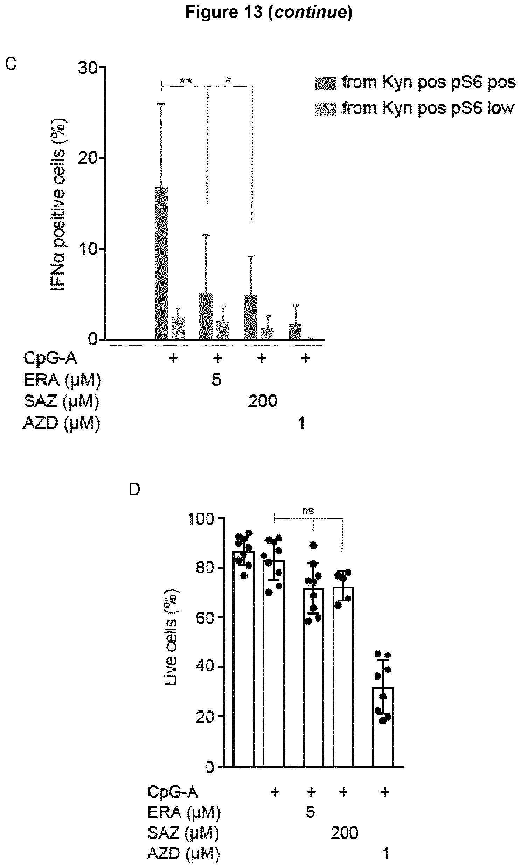

- Figure 13: shows (**A, C**) isolated pDC maintained +/- IL-3 and indicated inhibitors o/n, then stimulated +/- CpG-A for 5h and probed for: IFNα production, (**A**) *n*=9-13 in 3-5 experiments; (**C**) *n*=5-9 in 2-4 experiments; and (**A**) TNFα production, *n*=4 in 1 experiment; (**B**) kynurenine uptake and pS6 (S235/236) expression, *n*=5-9 in 2-4 experiments; (**D**) isolated pDC maintained in IL-3 and indicated inhibitors o/n, then stimulated +/- CpG-A for 5h were probed for live cells, *n*=5-9 in 2-4 experiments; Bar graphs are shown as mean +/- SD. Dots represent individual donors (*n*).
- Figure 14: shows isolated pDC were maintained +/- IL-3 and indicated inhibitors o/n, then stimulated +/- CpG-A for 5h and probed for: (**A**) IFNα production, *n*=6 in 2 experiments, (**B**) TNFα production, *n*=6 in 2 experiments; Bar graphs are shown as (**A, B**) geometric mean +/-geometric SD. (**C**) isolated pDC maintained in IL-3 and indicated inhibitors o/n, then stimulated +/- CpG-A for 5h were probed for live cells, n=6 in 2 experiments. (**C**) Bar graphs are shown as mean +/- SD. Dots represent individual donors (*n*).
- Figure 15: shows diagram illustrating the two-step model of pDC activation that leads to expression of SLC7A5:SLC3A2 and SLC7A11 and cytokine production.

### EXAMPLES

### Methods:

### Specimen preparation:

PBMC from buffy coats from healthy blood donors were isolated by density gradient centrifugation using SepMate tubes and Lymphoprep (both STEMCELL Technologies). pDC were negatively selected from PBMC using the plasmacytoid dendritic cell isolation kit II (Miltenyi Biotec). Buffy coats were kindly provided by the Institute for Transfusion Medicine and Gene Therapy, Medical Center - University of Freiburg (donor consent, anonymized). Skin samples of lupus patients were obtained through the biobank of the SFB829 (Z4 project) with informed consent from all the subjects and ethical approval obtained from the Ethics Committee at the University of Cologne (votes 12-163 and 19-1146). SLE PBMC were provided by ImmRheum biobank in Freiburg, a project that was approved by the local ethical committee (votes 507/16 and 624/14). All patients who provided blood to the biobank had provided written informed consent.

### Cell culture

Isolated pDC were maintained in RPMI (Gibco) supplemented with 10% FBS (HyClone), 4 mM L-Glutamine (Invitrogen), 100 U/ml Penicillin/Streptomycin (Invitrogen), 55 µM β-Mercaptoethanol (Sigma) and, where indicated, 50 ng/ml IL-3 (Peprotech) or 50 ng/ml GM-CSF (Peprotech). Cells were stimulated with 5 µM CpG-A ODN (ODN2216, InvivoGen) for times indicated. To detect intracellular cytokines, cells were treated with GolgiPlug (BD Bioscience) for the last 4 h of the culture. For overnight (o/n) incubations, cells were plated at 0.5 - 1x10⁶/ml. Where indicated, cells were treated with the following inhibitors for indicated times: 20 nM rapamycin, RAP (Calbiochem), 250 nM torin1, TOR (Tocris), 10µM heptelidic acid, HPA (Adipogen), 1 µM oligomycin (Sigma), 1µM AZD1480, AZD (Selleckchem), 25 nM-1µM baricitinib, BAR (LY3009104, Selleckchem), 5 µM erastin, ERA (Sigma), 100 µM or 200 µM sulfasalazine, SAZ (Tocris).

### Flow cytometry

The following fluorochrome-conjugated monoclonal antibodies were used for staining cell surface markers: IL-3Ra (CD123) (Miltenyi Biotec, clone AC145), HLA-DR (BD Bioscience, clone G46-6), CD116 (BD Bioscience, clone hGMCSFR-M1), CD4 (Biolegend, clone A161A1). Whole PBMC-staining was performed in 0.5% BSA in PBS for 30 min at 4° C, dead cells were excluded with the LIVE/DEAD Fixable Near-IR Dead Cell Stain Kit (Thermo scientific). Isolated pDC staining was performed in 0.5% BSA in PBS for 10 min at RT, dead cells were excluded with the LIVE/DEAD Fixable Near-IR or Aqua Dead Cell Stain Kits (Thermo scientific). Unspecific binding was blocked with FcR blocking reagent (Miltenyi). The following fluorochrome-conjugated Abs were used for intracellular staining: IFNα (Miltenyi, LT27:295), TNFα (BioLegend, clone Mab11), pS6(S235/236; CST, clone D57.2.2e), pSTAT5 (Y694; CST, clone C71E5). After surface staining, cells were fixed using Fixation/Permeabilization Kit (BD Bioscience) for 20 min at 4°C, and then additionally permeabilized in Perm/Wash solution for 30 min at 4°C, after which cells were incubated with antibodies against intracellular proteins, at previously determined antibody dilutions, for 30 min at RT. De novo protein synthesis was measured using Click-iT^{™} Plus OPP Alexa Fluor^{™} 647 Protein Synthesis Assay Kit (Thermo scientific) on pDC stained with LIVE/DEAD Fixable Aqua Dead Cell Stain Kit (Thermo scientific). Cycloheximide (Sigma) at 100 µg/ml was used as a positive control for protein synthesis inhibition.

Kynurenine uptake was performed using pDC stained with antibody against IL-3Ra (Miltenyi) and LIVE/DEAD Fixable Near-IR Dead Cell Stain Kit (Thermo scientific), as described previous. Briefly, stained cells were incubated in HBSS (Gibco) with 200 µM kynurenine (Sigma) for 4 min at 37°C. Cells were then fixed and additional intracellular staining was performed as described above. Where indicated, at the time of kynurenine uptake, cells were treated with 10 mM BCH (Tocris). The 405 nm laser and 450/50 BP filter were used for kynurenine fluorescence detection. Detection of intracellular mRNAs was performed using PrimeFlow RNA Assays (Thermo scientific). Isolated pDC were stained using LIVE/DEAD Fixable Aqua Dead Cell Stain Kit (Thermo scientific). Where indicated, intracellular staining with IFNα and/or TNFα was performed as described above. Fixed cells were then incubated with the following PrimeFlow target-specific probes: *IFNA2A* (type 6), *TNFα* (type 1), *SLC7A5* (type 6), *SLC3A2* (type 4), *SLC7A11* (type 1).

In all cases, cells were analyzed using LSR Fortessa flow cytometers (BD Biosciences) and data were processed using FlowJo software v9.9.6 (Tristar).

### Metabolic assays

Extracellular acidification rate (ECAR) and oxygen consumption rate (OCR) were measured using mitochondrial stress test in a 96 well Extracellular Flux Analyzer (Seahorse Bioscience). Cells were plated at 0.15x10⁶ per well on poly-D-lysine coated wells and preincubated at 37°C for a minimum of 45 min in the absence of CO₂ in un-buffered RPMI (Gibco) with 25 mM glucose (Invitrogen), 1 mM pyruvate (Invitrogen) and 2 mM L-glutamine (Invitrogen). Media was supplemented with 10% FBS (HyClone) and 50 ng/ml IL-3. (Peprotech). OCR and ECAR were measured under basal conditions following the addition of CpG-A. Results were collected with Wave software version 2.4 (Agilent). In **Figure 3C****,** ECAR was measured in real time first in the absence of glucose and then following the addition of 10 mM glucose (Sigma). In this case data is presented as baseline-corrected.

Glucose and lactate were measured in cell culture supernatants using the Cedex Bio Analyzer (Roche) and data presented as mmol/L.

### Stable-isotope tracing

Sample preparation and metabolite extraction: 10 mM uniformly-labelled ¹³C-glucose (Sigma) was used to label isolated pDC maintained in RPMI (Gibco) supplemented with 10% FBS (HyClone), 4mM L-Glutamine (Invitrogen), 100 U/ml Penicilin/Streptomycin (Invitrogen), 55µM β-Mercaptoethanol (Sigma) and 50 ng/ml IL-3 (Peprotech) for 3 h. For metabolite analysis, 0.6x10⁶ pDC were washed in ice-cold PBS (Gibco) and metabolites were extracted using 30 µl extraction buffer (50:30:20, methanol:acetonitrile:water, Sigma) cooled on dry ice for 30 min beforehand. Samples were centrifuged at 2000 g for 3 min to remove protein debris and supernatants were stored at -80°C until acquisition.

Metabolite measurement by LC-MS: LC-MS was carried out using an Agilent 1290 Infinity II UHPLC in-line with a Bruker Impact II QTOF operating in negative ion mode. Scan range was from 30 to 1050 Da. Mass calibration was performed at the beginning of each run. LC separation was on a Phenomenex Luna propylamine column (50 x 2 mm, 3 um particles) using a solvent gradient of 100% buffer B (5mM ammonium carbonate in 90% acetonitrile) to 90% buffer A (10 mM NH₄ in water). Flow rate was from 1000 to 750 µl/min. The Autosampler temperature was 5°C and injection volume was 2 µL. Metabolites were quantified using AssayR (Wills et al., Anal Chem 2017, 89, 9616 - 9619), and identified by matching accurate mass and retention time to standards.

### Single cell RNA sequencing (scRNA-seg)

Single cell RNA sequencing was performed using a 10X Genomics Chromium Controller. Single cells were processed with GemCode Single Cell Platform using GemCode Gel Beads, Chip and Library Kits (v2) following the manufacturer's protocol. An estimated 1400 cells were sequenced (∼700 from each condition), with a mean of 64338 reads per cell and a mean of 2200 genes detected per cell. Libraries were sequenced on HiSeq 3000 (Illumina). Samples were demultiplexed and aligned using Cell Ranger 2.2 (10X genomics) to genome build GRCh38 to obtain a raw read count matrix of barcodes corresponding to cells and features corresponding to detected genes. Raw read count matrices from single cell RNA sequencing data from lupus nephritis patients were retrieved from ImmPort (accession SDY997) where the ethical approval, sample acquisition and preparation, as well as library processing is described in detail (Arazi et al., Nat. Immunol. 2019, 20, 902 - 914). Read count matrices were processed, analyzed and visualized in R using Seurat v. 3 (Stuart et al., Cell 2019, 177, 1888-1902.e21.) and Uniform Manifold Approximation and Projection (UMAP) (McInnes et al., Journal of Open Source Software, 2018, 3(29), 861) as a dimensionality reduction approach. Differentially expressed genes, with greater than a 1.2 fold change and an adjusted *p* value of less than 0.05, were obtained and compared across clusters using the UpSet methodology (Conway et al., Bioinformatics 2017, 33, 2938-2940).

### ChIP sequencing analysis

STAT5 binding sites were identified by retrieving raw sequencing data from Gene Expression Omnibus (accession GSE43119), which was produced from human T cells (Schmidl et al., Blood 2014, 123, e68-78). The analysis performed was limited specifically to conventional T cells (samples SRR639410 and SRR639408). Retrieved data were processed using the default settings of the snakePipe methodology developed by the Bioinformatics core at the Max-Planck-Institute for Immunobiology and Epigenetics which is described at https://snakepipes.readthedocs.io/en/latest/index.html. Samples were mapped to GRCh38 and binding sites were identified using "Model-based Analysis of ChIP-Seq" MACS2 (Zhang et al., Genome Biol, 2008, 9, R137) with the input control used as background.

### Tissue Immunostaining

Immunostaining for SLC7A11 and IL3-Ra (CD123) was performed on paraffin sections (6 mm). First, they were deparaffinized by xylol and ethanol incubations and washed in Tris-buffered saline (TBS). Heat-induced epitope retrieval was performed using Tris-EDTA buffer (10mM Tris, 1 mM EDTA, pH 9) for 20 min in a heated (95°C) water bath. Samples underwent incubation with primary antibodies against SLC7A11 (Abcam, ab37185), and IL-3Ra (Novocastra/Leica, clone BR4MS, name NCL-L-CD123) diluted in TBS, 1% normal goat serum and 0.01% Tween in a humid chamber o/n at 4°C. For the immunofluorescence, Alexa Fluor 488 conjugated secondary antibodies were used. Cell nuclei were counterstained with DAPI. Immunohistochemistry was performed in an automated system (Leica BOND-MA, Leica Microsystem, Wetzlar, Germany). Images were captured using a BIOREVO BZ-9000 fluorescence microscope and associated software (Keyence, Neu-Isenburg, Germany).

### Quantification and statistical analysis:

With the exception of scRNASeq, statistical analysis was performed using Prism 7 software (GraphPad) and results are represented as Mean ± SD or geometric SD (indicated in legends). Comparisons for two groups were calculated using unpaired two-tailed Student's t tests (Figures 1A, 2D; Figures S1D, S1E, S2B, S2C). Comparisons of more than two groups were calculated using one-way ordinary paired (Figures 1C, 2A, 2C, 2D, 1E, 4A, 4C, 4D, 5B, 13A, 14A-B, 4E) or nonmatched (Figures 5C, 6C-6D, 7B-7E, 12B, 13A, 4B, 7D, 12A-12C) ANOVA with Tukeys multiple comparison tests or two-way nonmatched ANOVA (Figures 1E, 6A, 7G, 8A, 8B, 12D, 13C, 6B) with Tukeys multiple comparison tests. Designation of p-values were as follows: * ≤ 0.05; ** ≤ 0.01; *** ≤ 0.001; **** ≤ 0.00001.

### Example 1. IL-3 primes pDC for cytokine production and mTORC1 activation in response to CpG-A

pDC produce type I IFN in response to stimulation through endosomal/lysosomal TLR7 or TLR9. To explore if this process is dependent on mTORC1, cytokine production was measured over time by isolated pDC from healthy donors and cultured in medium containing IL-3, with or without the TLR9 agonist CpG-A (class A CpG-A oligonucleotides, ODN2216). Maximal IFNα secretion occurred between 10 h and 12 h post activation, during which time approx. 50% of the cells made this cytokine **(****Figure 1A****).** Stimulation also resulted in TNFα production, which peaked at 8 h post activation, and declined rapidly thereafter **(****Figure 1A****).** Co-production of TNFα did not affect the amount of IFNα being made per cell, but maximal TNFα production was associated with IFNα co-production **(****Figure 1A****).** Production of both cytokines ceased by approx. 24 h post activation **(****Figure 1A****).**

To examine the role of IL-3 in pDC metabolism and function, pDC were maintained overnight in media with or without IL-3, after which they were either left untreated or stimulated with CpG-A. Results showed that cell death was increased in the absence of IL-3 **(****Figure 1B****)** and that the addition of CpG-A was unable to rescue viability **(****Figure 1B****).** The absence of IL-3 had a marked inhibitory effect on CpG-A-induced IFNα and TNFα production **(****Figure 1C****).** This was not a reflection of cell death in the absence of IL-3, since cytokine measurements were made only in living cells. IL-3 was required for the CpG-A-induced transcription and translation of IFNα **(****Figure 1D****).** However, IL-3 alone was sufficient to drive TNFα transcription, but this mRNA was translated only following stimulation with CpG-A **(****Figure 1E****).**

Early work revealed that IL-3 acts as a growth factor to enhance anabolic pathways. Since anabolism is controlled centrally by mTORC1, and mTORC1 has been shown to be important for pDC activation, regulation of mTORC1 activity by IL-3 was evaluated by measuring phosphorylation of Ser235/236 of ribosomal protein S6 (pS6 (S235/236)), which provides a measure of activation of p70S6K, a direct mTORC1 target. pS6 (S235/236) was found in <20% of pDC maintained without IL-3, and this was not increased by stimulation with CpG-A **(****Figure 2A****).** Maintenance in IL-3 alone did not increase pS6 (S235/236), but the combination of IL-3 followed by stimulation with CpG-A resulted in approx. 60% of pDC becoming positive for pS6 (S235/236) **(****Figure 2A****).** Then it was examined the effect of rapamycin (RAP), a selective mTORC1 inhibitor, on activation-induced S6 phosphorylation, and found that it substantially limited the size of the pS6 (S235/236)-positive population **(****Figure 2B****).** Therefore, IL-3 plays a critical role in priming pDC to allow mTORC1 activation upon CpG-A stimulation. Since pDC were unable to make cytokines in response to CpG-A in the absence of IL-3, it was reasoned that this could reflect the role of IL-3 in mTORC1 priming. Consistent with this, RAP-mediated inhibition of S6 (S235/236) phosphorylation was sufficient to partially inhibit production of IFNα **(****Figure 2C****)** but also TNFα **(****Figure 2D****)** by CpG-A-stimulated pDC.

### Example 2. mTORC1 primes pDC for metabolic changes critical for activation

As mTORC1 controls protein synthesis through 4E-BP translation initiation factor, the inventors tested if mTORC1 drives translation in activated pDC by measuring the incorporation of a puromycin analogue into newly translated protein. Compared to untreated cells, addition of CpG-A resulted in a significant increase in translation that was prevented by treatment with RAP **(****Figure 3A****).** In this regard, RAP was almost as effective as a selective mTORC1/2 inhibitor that blocks rapamycin-resistant functions of mTORC1, torin1 (TOR) and the translation inhibitor cyclohexamide (CXH) **(****Figure 3A****).**

mTORC1 is recognized to promote anabolic metabolism linked to increased glycolysis. Consistent with this, activated pDC consumed more glucose than resting cells **(****Figure 3B****).** Moreover, the extracellular acidification rate (ECAR), a mark of lactate production, which can often accompany immune cell activation, was increased in activated vs. unstimulated pDC **(****Figures 3C, 3D****).** Increased ECAR was linked to increased release of lactate **(****Figure 3E****),** was glucose-dependent **(****Figure 3C****),** and began to occur within minutes after the addition of CpG-A **(****Figure 3D****).** Elevated ECAR persisted for at least 10 h, in line with the time course of cytokine production and mTORC1 activity. Tracing of carbon from 13C-labelled glucose supported the view that stimulation with CpG-A leads to increased incorporation of glucose carbon into lactate, but also revealed increased incorporation into TCA cycle intermediates **(****Figure 3F****).** Consistent with the latter, the mitochondrial oxygen consumption rate (OCR), a mark of mitochondrial oxidative phosphorylation, was also increased in CpG-A-stimulated pDC **(****Figure 3G****).**

The preceding findings indicated that metabolic changes occur early after pDC activation. To directly explore the role of increased glucose usage in pDC biology, we incubated CpG-A-stimulated cells with heptelidic (koningic) acid (HPA), a selective inhibitor of GAPDH, the central enzyme in the glycolysis pathway. HPA reduced secreted lactate levels to below those in cultures of unstimulated pDC **(****Figure 4A****),** and caused reductions in overall protein synthesis **(****Figure 4B****)** and production of IFNα and TNFα **(****Figure 4C****).** Inhibition of mTORC1 using RAP or TOR resulted in a decline in CpG-A-induced lactate release, supporting a link between stimulation-dependent mTORC1 activation and increased glycolytic metabolism **(****Figure 4D****).** We also found that the electron transport chain complex V ATP synthase inhibitor, oligomycin (OLI), inhibited IFNα production to approximately the same extent as HPA **(****Figure 4E****),** although it had no effect on TNFα **(****Figure 4E****).** The combination of HPA and OLI inhibited IFNα and TNFα production completely **(****Figures 4E****).**

Taken together, these data indicate that stimulation with CpG-A in pDC primed by exposure to IL-3, leads to an mTORC1-dependent increase in glucose metabolism that is critical for pDC activation.

### Example 3. IL-3 induces the expression of functional System L amino acid transporters that permit mTORC1 activation in response to CpG-A

mTORC1 activation is dependent on the presence of sufficient environmental levels of amino acids, amongst which leucine and methionine are of particular importance. In T cells both of these amino acids are acquired from the environment via System LAA transporters comprising a heterodimer of the solute carriers *SLC7A5* and *SLC3A2* (CD98). Other System LAA transporters are composed of *SLC7A6, SLC7A7* or *SLC7A8* paired with *SLC3A2.*

Single cell RNA sequencing (scRNA-seq) revealed that the majority of pDC maintained in IL-3 primarily express *SLC7A5,* regardless of CpG-A stimulation **(****Figure 5A****).** *SLC7A6* expression was limited in these cells **(****Figure 5A****)** and *SLC7A7* and *SLC7A8* transcripts were not detected.

Analysis by PrimeFlow RNA revealed that *SLC7A5* and *SLC3A2* were co-expressed in the majority of pDC cultured in IL-3 and stimulated with CpG-A **(****Figure 5B****).** This was a result of the signal delivered by IL-3, since approx. 97% of pDC cultured in IL-3 but not stimulated with CpG-A were also positive for SLC7A5 and SLC3A2 transcripts. In contrast, significantly fewer cells maintained without IL-3 expressed these solute carriers and stimulation with CpG-A did not rescue their expression **(****Figure 5B****).**

To assess whether IL-3-induced expression of SLC7A5 and SLC3A2 allowed the assembly of a functional System LAA transporter, we used a flow cytometry-based assay that measures the ability of cells to take up kynurenine, a naturally fluorescing molecule that is transported into cells via SLC7A5; this can be used as a proxy measurement of large neutral amino acid (LNAA) uptake. We found that the ability of pDC to take up kynurenine aligned with their expression of SLC7A5 and SLC3A2, and pinpointed IL-3 as being capable of conferring this capability **(****Figure 5C****).** Addition of BCH (2-Aminobicyclo[2.2.1]heptane-2-carboxylic acid), a System LAA transporter-specific inhibitor, abrogated transport of kynurenine in all of the conditions, confirming that uptake of this leucine surrogate is SLC7A5-dependent **(****Figure 5C****).** Of interest, freshly isolated pDC from PBMC of healthy donors and SLE patients also expressed System LAA transporters SLC7A5 and SLC3A2 **(****Figure 5D****)** and furthermore pDC from healthy donors had the ability to take up kynurenine immediately *ex vivo,* a process that was blocked by BCH **(****Figure 5E****).**

To confirm that functional System LAA transport is a prerequisite for mTORC1 activation in response to CpG-A, the effects of IL-3 and CpG-A were examined on combined measurements of kynurenine uptake, S6 (S235/236) phosphorylation and cytokine production. The analysis revealed that in the absence of IL-3 the majority of cells were negative for kynurenine transport and pS6 (S235/236) **(****Figure 6A****).** Stimulation with CpG-A resulted in a slight increase in cells able to take up kynurenine and positive for pS6 (S235/236) **(****Figure 6A****).** As expected, maintenance in IL-3 allowed the majority of pDC to take up kynurenine **(****Figure 6A****),** but these cells remained negative for pS6 (S235/236). However, addition of CpG-A led to the emergence of a significant population of kynurenine and pS6 (S235/236) double positive cells **(****Figure 6A****),** in which S6 (S235/236) phosphorylation, but not kynurenine uptake, could be inhibited by RAP **(****Figure 6B****).** Finally, it was evaluated if cytokine production by activated pDC was linked to System LAA transporter activity. It was found that IFNα and TNFα production occurred only in cells that expressed SLC7A5 **(****Figure 6C****)** and were therefore able to take up kynurenine **(****Figure 6D****).** Together, these data show that IL-3 is essential for induction of the System LAA transporters SLC7A5 and SLC3A2 and hence for active transport of LNAA into pDC, processes that prime subsequent activation of mTORC1 and cytokine production in response to CpG-A.

### Example 4. IL-3 stimulation of SLC7A5:SLC3A2 and cytokine production is mediated by JAK2 and STAT5

IL-3 signals through a receptor comprised of the cytokine-binding IL-3Ra dimerized with common β chain (encoded by CSF2RB), which couples the receptor to the JAK2-STAT5 pathway. Using published data examining STAT5 chromatin immunoprecipitation sequencing in human CD4⁺ T cells, STAT5 DNA binding sites were found in both SLC7A5 and SLC3A2 loci. These data suggested that the JAK2-STAT5 pathway could be responsible for the IL-3 induced expression of these genes. Moreover, IL-3, but not CpG-A, was able to induce phosphorylation of STAT5 (Y694) **(****Figure 7A****).** Therefore, we examined the effects of the selective JAK2 inhibitor AZD1480 (AZD) and the clinically used JAK2/1 inhibitor baricitinib (BAR), on CpG-A-induced activation in pDC cultured in IL-3.

As expected, AZD and BAR inhibited IL-3-induced STAT5 (Y694) phosphorylation **(****Figure 7A****).** Consistent with a role for IL-3-induced JAK2 signaling in the expression of *SLC7A5* and *SLC3A2,* both AZD and BAR significantly inhibited the expression of these genes **(****Figure 7B****),** and this was accompanied by reductions in the uptake of kynurenine **(****Figure 7C****),** and in the phosphorylation of S6 (S235/236) **(****Figure 7D****).**

JAK2 inhibition resulted in marked reductions in IFNα and TNFα production that was linked to an inhibitor-induced decrease in the ability of activated pDC to take up kynurenine **(****Figure 7E****).** Thereafter it was observed that addition of JAK2 inhibitors led to the loss of cells that were positive for both kynurenine transport and mTORC1 activity **(****Figure 7F****),** which was a similar outcome to that observed in IL-3-deprived cells **(****Figure 6A****).** Finally, only pDC with both high rates of kynurenine uptake and high mTORC1 activity (S6 phosphorylation) made IFNα **(****Figure 7G****),** and that cytokine production was lost following JAK2 inhibition **(****Figure 7G****),** in agreement with results of **Figure 7E****.**

### Example 5. GM-CSF stimulates activity of SLC7A5:SLC3A2 and cytokine production.

The common β subunit of the IL-3R is also the signaling component of the GM-CSF and IL-5 receptors. Human pDC express GM-CSFRa as well as IL-3Ra but do not express IL-5Ra, which is consistent with previous reports that pDC can respond to GM-CSF as well as to IL-3. It was found that, like IL-3, GM-CSF supported pDC survival, induced a population of cells with high rates of kynurenine uptake and mTORC1 activity **(****Figure 8A****)** and that these cells were IFNα producers in response to CpG-A **(****Figure 8B****).** Moreover, all of these parameters were inhibited by AZD **(****Figures 8A****,** **8B****).**

### Example 6. pDCstimulation induces selective expression of SLC7A11 in IFNα producing pDC

Even at peak cytokine production, only a subset of stimulated pDC was positive for pS6 (S235/236) and cytokine production **(****Figures 1A** **and** **2B****).** Therefore, the heterogeneity within the pDC population was explored through scRNA-seq. Based on transcriptional similarities, 12 clusters (C) of cells were identified in pDC cultured in IL-3, of which one, C9, was apparent only after stimulation with CpG-A **(****Figure 9A****).** In addition, the contributions of C2, C4 and C7 to the overall pDC population increased following stimulation with CpG-A **(****Figure 9B****).** Within these clusters, only C9 and C2, were enriched in type I IFN transcripts **(****Figure 9C****).** Of note, despite the expected broad expression of TNFα, C9 in particular also appeared to be enriched in TNFα transcripts **(****Figure 9D****).** C2 and C9 had similar transcriptional profiles, evident by their proximity on the Uniform Manifold Approximation and Projection (UMAP) as well as in the clustering hierarchy **(****Figure 9D****).**

To identify similarities between *in vitro*-activated pDC and *in* vivo-activated pDC, activated pDC from our analysis were compared with recently reported scRNA-seq data from immune cells isolated from lupus nephritis kidney biopsies (Arazi *et al.*)*.* The published data were reanalyzed and focusing specifically on the transcriptomes of pDC at the site of pathology (SLE pDC, C22, **Figures 10A, 10B****,** **10C****).** Consistent with the findings described above regarding the importance of IL-3 or GM-CSF-initiated signaling and System LAA transporters expression for pDC activation, it was found that SLE pDC (C22) expressed *IL3RA, CSF2RA, CSF2RB, SLC7A5* and *SLC3A2* **(****Figures 10A, 10B****,** **10C****).** Indeed, expression of these genes was highest in pDC (C22) within the immune cell populations of nephritic kidney biopsies **(****Figures 10A, 10B****,** **10C****).** Next, differentially expressed genes in C2 and C9 from *in* vitro-activated pDC and SLE pDC were identified compared to all other clusters in their respective datasets. We then performed UpSet analysis (Conway *et al.,* 2017) on these results, in order to compare the transcriptional profile of activated pDC across these datasets. Our analysis showed that of the 43 expressed genes shared between C9, C2 and SLE pDC **(****Figure 10D****),** 21 were differentially regulated in C2 and C9 but not in any other cluster from our in vitro dataset **(****Figure 10E****).** Within these, we noticed that *SLC7A11,* which was one of three genes co-expressed by all three groups of cells **(****Figure 10D****),** was specific to the type I IFN and TNFα producing C9, and to a lesser degree C2 **(****Figure 10F****),** and that these two clusters contributed most to the overall expression of *SLC7A11* **(****Figure 10G****).** Moreover, amongst lesional immune cell populations from lupus nephritis kidney biopsies, *SLC7A11* was expressed almost exclusively in SLE pDC (C22, **Figures 10H****,** **10I****).**
As expression of *SLC7A11* seemed to mark type I IFN and TNFα producing pDC, it was evaluated if *SLC7A11* is detectable in pDC in other pathological sites in which pDC infiltration is implicated in disease. To this aim, sections from diseased skin from patients with cutaneous lupus were stained with antibodies against *SLC7A11* and the IL-3Ra, a known marker of pDC in tissue sections. The microscopic analysis revealed a marked increase in *SLC7A*11-positive cells in the skin of cutaneous lupus patients compared to skin from healthy donors (HD, **Figure 11A****)** and the majority of these were IL-3Ra positive **(****Figure 11B****).** Further analysis included testing pDC isolated from PBMC collected from SLE patients with varying degrees of disease activity (Systemic Lupus Erythematosus Disease Activity Index, SLEDAI, from 0 to 8), but results showed no evidence for *SLC7A11* expression in these circulating cells **(****Figure 11C****).**
Taken together, these data suggest that *SLC7A11* expression is a mark of activated, cytokine-producing tissue-specific pDC.

### Example 7. Inhibition of JAK2 potently blocks cytokine production by activated pDC

Unlike *SLC7A5* and *SLC3A2* **(****Figure 5D****),** *SLC7A11* **(****Figure 11C****)** was not expressed by circulating pDC and therefore its expression was unlikely to be induced by signaling downstream of the common β chain of the IL-3 or GM-CSF receptors. This was confirmed by the finding that IL-3 alone was incapable of inducing expression of *SLC7A11* **(****Figures 12A, 12B****,** **12C****).** Rather, this gene was expressed in response to stimulation with CpG-A **(****Figures 12A, 12B****,** **12C****).** *SLC7A11* encodes the specific subunit of the cystine/glutamate exchanger x_{c}^{-.}, which when chaperoned by *SLC3A2* allows cells to take up cystine in exchange for glutamate. Expression of *SLC7A11* was coupled to expression of *SLC3A2* **(****Figure 12A****),** indicating that activated pDC can express functional x_{c}⁻ transporter. While IL-3 alone was incapable of inducing *SLC7A11* expression, it was critical for priming cells to express *SLC7A11* in response to CpG-A, since JAK2 inhibition prevented this from happening **(****Figures 12A****,** **C****).** This is likely the result of a requirement for IL-3 mediated System LAA transporter expression for optimal mTORC1 activation, because TOR (torin1) also inhibited *SLC7A11* expression **(****Figures 12A****,** **C****).** Consistent with this, pDC maintained in IL-3 and stimulated with CpG-A had the highest frequencies of *SLC7A11* and *SLC7A5* double positive cells **(****Figure 12B****),** and as a result produced **IFNα** at full capacity **(****Figure 12D****).** In contrast, pDC deprived of IL-3 or treated with AZD or BAR lost expression of both transporters and hence **IFNα** production **(****Figures 12B****,** **12D****).** Again, treatment with TOR had similar, although less pronounced effect to AZD and BAR **(****Figures 12B****,** **D****).**

### Example 8. Inhibition of x_{c} ^{-.}potently blocks cytokine production by activated pDC

The finding that *SLC7A11* expression distinguishes activated tissue-specific pDC from circulating pDC makes it an attractive therapeutic target. The antiporter x_{c}⁻, is a target of the small molecule inhibitors erastin (ERA) and sulfasalazine (SAZ). It was reasoned that inhibition of x_{c}⁻, which comprises *SLC7A11,* might prevent the production of cytokines by pDC.

As predicted, both ERA and SAZ inhibited CpG-A- induced IFNα and TNFα (**Figure 13A**) production. This was associated with reductions of cells that had both high rates of kynurenine uptake and S6 (S235/236) phosphorylation (**Figure 13B**), which is consistent with the fact that only pDC with both of these attributes make cytokines (**Figure 13C**). Inhibition occurred in the absence of appreciable cell death (**Figure 13D**).

### Example 9. Coordinated inhibition of JAK2 and antiporter x_{c}⁻ potently blocks cytokine production by activated pDCs.

Given the effectiveness of targeting JAK2 and x_{c}⁻ in limiting cytokine production, it was analysed whether a combinatorial approach of inhibiting both JAK2 and x_{c}⁻ might result in further loss of pDC function. An *in vitro* dose escalation was performed to define the effects of BAR on viability and cytokine production by activated pDC. Results showed that production of IFNα (**Figure 14A**) and TNFα (**Figure 14B**) declined as BAR concentration rose from 25 to 100 nM, and that this was accompanied by increased cell death (**Figure 14C**). Moreover, the doses of BAR and SAZ that alone were suboptimal, synergized to increase the inhibition of both IFNα and TNFα production (**Figures 14A, 14B**), without diminishing cell survival beyond that seen with BAR alone (**Figure 14C**).

Taken together, these data indicate that pDC activation is associated with the expression of *SLC7A11* and that inhibition of x_{c}⁻ is able to suppress the ability of these cells to produce IFNα and TNFα. Furthermore, a combinatorial approach for suppressing pDC activation by targeting both JAK2 and x_{c}⁻, resulted in an additive inhibitory effect that essentially prevented cytokine production (Figure 15).

## Claims

1. A combination for use in the treatment of an autoimmune disease, wherein the combination consists of:
at least one inhibitor of antiporter system x_{c}⁻ and
at least one inhibitor selected from the group consisting of:
a JAK2 inhibitor,
a SLC7A5:SLC3A2 inhibitor,
an inhibitor targeting IL-3 receptor alpha,
an inhibitor targeting IL-3 receptor beta,
an inhibitor targeting GM-CSF,
an inhibitor targeting GM-CSF receptor alpha, and
an inhibitor targeting STAT5,
wherein the autoimmune disease is mediated by plasmacytoid dendritic cells.

2. A combination for use according to claim 1, wherein the combination consists of:
at least one inhibitor of antiporter system x_{c}⁻ and
at least one inhibitor selected from the group consisting of:
a JAK2 inhibitor, and
a SLC7A5:SLC3A2 inhibitor.

3. The combination for use according to claim 1 or 2, wherein the at least one inhibitor of the antiporter system x_{c}⁻ is selected from the group comprising sulfasalazine, erastin, erastin analogs, azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-I-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthyl-ethyl-ACPA, bis-trifluoromethylphenyl-isoxazole-4-hydrazone, 5-naphthylethyl isoxazole-4-(2,4-dinitrophenol)hydrazone-dinitrophenol, sorafenib.

4. The combination for use according to any one of claims 1 - 2, wherein the at least one inhibitor of the antiporter system x_{c}⁻ is sulfasalazine.

5. The combination for use according to any one of the claims 1 - 4, wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acidsphenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine; KHK2898 antibody, IGN523 antibody; or
wherein the inhibitor targeting IL-3 receptor alpha is selected from the group comprising CSL362 antibody, flotetuzumab, IL3LDM, DT388IL3, and SGN-CD123A,; or
wherein the inhibitor targeting IL-3 receptor beta is CSL311 antibody, or wherein the inhibitor targeting GM-CSF is otilimab, namilumab, lenzilumab, plonmarlimab, or gimsilumab; or
wherein the inhibitor targeting GM-CSF receptor alpha is mavrilimumab; or wherein the inhibitor targeting STAT5 is JPX1188, JPX0802, JPX1185, compound 17f, compound AC-4-130, CAS 285986-31-4, BP-1-075, BP-1-107, BP-1-108, SF-1-087, SF-1-088, IQDMA, or CAS 2062-78-4.

6. The combination for use according to any one of the claims 1 - 5, wherein the at least one JAK2 inhibitor is barcitinib or delgocitinib.

7. The combination for use according to any one of the claims 1 - 6, wherein the autoimmune disease is selected from the group comprising lupus; cutaneous lupus; systemic lupus erythematosus (SLE); neonatal lupus; drug-induced lupus; SLE-induced conditions; systemic sclerosis; cutaneous scleroderma; Sjögren's syndrome; juvenile and adult dermatomyositis; psoriasis; alopecia areata; lichen planus; lichen sclerosus; vitiligo; polymyositis; rheumatoid arthritis; ulcerative colitis; Crohn's disease; rejection of transplanted organs and tissues; rhinitis; chronic obstructive pulmonary diseases; sinusitis; atopic dermatitis; anaphylaxis; DiGeorge syndrome; Hyper-IgE syndrome; Wiskott-Aldrich syndrome; ataxia-telangiectasia; immune mediated cancers; multiple sclerosis (MS); immune-mediated or Type 1 Diabetes Mellitus; immune mediated glomerulonephritis; pemphigus; pemphigus vulgaris; myasthenia gravis; inflammatory bowel diseases (IBD); autoimmune thyroid diseases including Hashimoto's disease and Grave's disease; Goodpasture syndrome (GPS); myasthenia gravis pseudoparalytica; phacoantigenic uveitis; chronical aggressive hepatitis; primary biliary cholangitis; autoimmune hemolytic anemia; immune thrombocytopenia; osteoporosis; pernicious anemia; ophtalmia sympatica; Werlhof's disease.

8. The combination for use according to any one of the claims 1 - 7, wherein the autoimmune disease is systemic lupus erythematosus.

9. A pharmaceutical composition consisting of at least one inhibitor of antiporter system x_{c}⁻ and at least one inhibitor selected from the group consisting of a JAK2 inhibitor, a SLC7A5:SLC3A2 inhibitor, an inhibitor targeting IL-3 receptor alpha, an inhibitor targeting IL-3 receptor beta, an inhibitor targeting GM-CSF, an inhibitor targeting GM-CSF receptor alpha, and an inhibitor targeting STAT5, together with at least one pharmaceutically acceptable vehicle, excipient and/or diluent.

10. The pharmaceutical composition according to claim 9, wherein the at least one inhibitor of the antiporter system x_{c}⁻ is selected from the group comprising sulfasalazine, erastin, erastin analogs, , azo-linked amino-naphthyl-sulfonate analogs of sulfasalazine, ethyl 2-hydroxy-5-[(E)-2-4{4-[(pyridine-2-yl)sulfamoyl]phenyl}diazen-1-yl]-benzoate, 2-Hydroxy-5-[(E)-2-{4-[(pyridine-2-yl)sulfamoyl]phenyl}diazen-1-yl]benza-mide, 4-[(E)-2-(2-Hydroxynaphthalen-1-yl)diazen-1-yl]benzene-1-sulfonic acid, 4-nitro-N-phenylbenzene-1-sulfonamide, 2-Hydroxy-5-(3-phenylpropanamido)benzoic acid, HG106, glutamate α-aminoadipic acid, α-aminosuberic acid, cystathionine, ibotenate, quisqualate, L-β-N-oxalyl-L-α,β-diaminopropionate, L-alanosine, β-N-methylamino-L-alanine, L-serine-O-sulphate, L-α-aminopimelate, L-homocysteate, S-sulpho-l-cysteine, S-4-carboxyphenylglycine, S-4-carboxy,3-hydroxy-phenylglycine, S-3-carboxy,3-hydroxy-phenylglycine, R,S-sulphothienylglycine, [(R,S)-4-[4'-carboxyphenyl]-phenylglycine, 5-benzyl-4-bis-TFM-HMICA, 5-naphthyl-4-bis-TFM-HMICA, 5-4-TFM-benzyl-4-bis-TFM-HMICA, R,S-4-Br-homoibotenate, S-2-naphthyl-ethyl-ACPA, bis-trifluoromethylphenyl-isoxazole-4-hydrazone, 5-naphthylethyl isoxazole-4-(2,4-dinitrophenol)hydrazone-dinitrophenol, sorafenib.

11. The pharmaceutical composition according to claim 9 or 10, wherein the at least one inhibitor of the antiporter system x_{c}⁻ is sulfasalazine.

12. The pharmaceutical composition according to any one of the claims 9 - 11, wherein the at least one JAK2 inhibitor is selected from the group comprising baricitinib, delgocitinib, fedratinib, gandotinib, momelotinib, oclacitinib, pacritinib, peficitinib, ruxolitinib, tofacitinib, AZD1480; or
wherein the at least one SLC7A5:SLC3A2 inhibitor is selected from the group comprising JPH203 (KYT-0353), triiodothyronine analogs; tetraiodothyronine; 1,2,3-dithiazole derivatives, 1,2,4-dithiazine derivatives; phenylalanine; phenylalanine derivates; tryptophan analogs; 2-aminobicyclo-(2,2,1)-heptane-2-carboxylic acid; (S)- 2-Amino-3-(3-((2,4-dicyano-3-(4-(2-(methylamino)-2-oxoethoxy)phenyl) benzo[4,5]imidazo[1,2-a]pyridin-1-yl)-carbamoyl)phenyl)propanoic acid 1; 2-amino-3-(3-benzylphenyl)propanoic acid; 2-amino-3-(3-benzyl-4-hydroxyphenyl)propanoic acid; hydroxamic acidsphenylalanine, hydroxamic acids-leucine, hydroxamic acids-isoleucine, hydroxamic acids-methionine; β-(4-chlorophenyl)-γ-aminobutyric acid (β-(4-chlorophenyl)-GABA); gabapentin; (2S)-Amino[(5S)-3-chloro-4,5-dihydro-1,2-oxazol-5-yl]ethanoic acid; para-chlorophenylalanine; L-3,4-dihydroxyphenylalanine, KHK2898 antibody, IGN523 antibody; or
wherein the inhibitor targeting IL-3 receptor alpha is selected from the group comprising CSL362 antibody, flotetuzumab, IL3LDM, DT388IL3, and SGN-CD123A; or
wherein the inhibitor targeting IL-3 receptor beta is CSL311 antibody, or wherein the inhibitor targeting GM-CSF is otilimab, namilumab, lenzilumab, plonmarlimab, or gimsilumab; or
wherein the inhibitor targeting GM-CSF receptor alpha is mavrilimumab; or
wherein the inhibitor targeting STAT5 is JPX1188, JPX0802, JPX1185, compound 17f, compound AC-4-130, CAS 285986-31-4, BP-1-075, BP-1-107, BP-1-108, SF-1-087, SF-1-088, IQDMA, or CAS 2062-78-4.

13. The pharmaceutical composition according to any one of the claims 9 - 12, wherein the at least one JAK2 inhibitor is barcitinib.

14. The pharmaceutical composition according to any one of the claims 9 - 13, wherein the at least one JAK2 inhibitor is delgocitinib.

15. The pharmaceutical composition according to any one of the claims 9 - 14 for use in the treatment of an autoimmune disease, wherein the autoimmune disease is mediated by plasmacytoid dendritic cells.
